(19) Europäisches Patentamt / European Patent Office / Office européen des brevets

(11) EP 1 976 883 B1

(12) **EUROPEAN PATENT SPECIFICATION**

(45) Date of publication and mention of the grant of the patent:
**03.10.2012 Bulletin 2012/40**

(21) Application number: **07718000.8**

(22) Date of filing: **17.01.2007**

(51) Int Cl.:
***C07K 16/28*** *(2006.01)* ***A61P 35/00*** *(2006.01)*
***A61P 37/00*** *(2006.01)*

(86) International application number:
**PCT/US2007/001451**

(87) International publication number:
**WO 2007/084672 (26.07.2007 Gazette 2007/30)**

(54) **MONOCLONAL ANTIBODIES AGAINST CD30 LACKING IN FUCOSYL AND XYLOSYL RESIDUES**

MONOKLONALE ANTIKÖRPER GEGEN CD30 OHNE FUCOSYL- UND XYLOSYLRESTE

ANTICORPS MONOCLONAUX ANTI-CD30 DEPOURVUS DE RESIDUS FUCOSYL ET XYLOSYL

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**RS**

(30) Priority: **17.01.2006 US 759298 P**
**07.04.2006 US 790373 P**
**11.04.2006 US 791178 P**
**09.06.2006 US 812702 P**
**11.08.2006 US 837202 P**
**11.08.2006 US 836998 P**

(43) Date of publication of application:
**08.10.2008 Bulletin 2008/41**

(73) Proprietors:
- **Medarex, Inc.**
  **Princeton, NJ 08540 (US)**
- **Synthon B.V.**
  **6545 CM Nijmegen (NL)**

(72) Inventors:
- **BLACK, Amelia, Nancy**
  **Los Gatos, CA 95032 (US)**
- **PASSMORE, David, B.**
  **San Carlos, CA 94070 (US)**
- **SRINIVASAN, Mohan**
  **San Jose, CA 95129 (US)**
- **DICKEY, Lynn, F.**
  **Cary, NC 27511 (US)**
- **COX, Kevin, M.**
  **Raleigh, NC 27608 (US)**
- **PEELE, Charles, G.**
  **Apex, NC 27502 (US)**
- **WANG, Ming-Bo**
  **Canberra Australian Capital Territory 2617 (AU)**

(74) Representative: **Tuxworth, Pamela M.**
**J A Kemp**
**14 South Square**
**Gray's Inn**
**London WC1R 5JJ (GB)**

(56) References cited:
**WO-A-03/059282 US-A1- 2004 261 148**

- **P. BORCHMANN ET AL.: "The human anti-CD30 antibody 5F11 shows in vitro and in vivo activity against malignant lymphoma." BLOOD, vol. 102, no. 10, 15 November 2003 (2003-11-15), pages 3737-3742, XP002372400**
- **N. YAMANE-OHNUKI ET AL.: "Establishment of FUT8 knockout Chinese hamster ovary cells: An ideal host cell line for producing completely defucosylated antibodies with enhanced antibody-dependent cellular cytotoxicity." BIOTECHNOLOGY AND BIOENGINEERING, vol. 87, no. 5, 5 September 2004 (2004-09-05), pages 614-622, XP002983153**
- **T. SHINKAWA ET AL.: "The absence of fucose but not the presence of galactose or bisecting N-acetylglucosamine of human IgG1 complex-type oligosaccharides shows the critical role of enhancing antibody-dependent cellular cytotoxicity." THE JOURNAL OF BIOLOGICAL CHEMISTRY, vol. 278, no. 5, 31 January 2003 (2003-01-31), pages 3466-3473, XP002355427**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- A. ENGERT ET AL.: "Evaluation of ricin A chain-containing immunotoxins directed against the CD30 antigen as potential reagents for the treatment of Hodgkin's disease." CANCER RESEARCH, vol. 50, no. 1, 1 January 1990 (1990-01-01), pages 84-88, XP000406156
- R. SCHNELL ET AL.: "Development of new ricin A-chain immunotoxins with potent anti-tumor effects against human Hodgkin cells in vitro and disseminated Hodgkin tumors in SCID mice using high-affinity monoclonal antibodies directed against the CD30 antigen." INTERNATIONAL JOURNAL OF CANCER, vol. 63, no. 2, 9 October 1995 (1995-10-09), pages 238-244, XP000567764
- J. GASDASKA ET AL.: "Advantages of therapeutic protein production in the aquatic plant Lemna." BIOPROCESSING, vol. 2, March 2003 (2003-03), pages 49-56, XP002308458
- K. COX ET AL.: "Glycan optimization of a human monoclonal antibody in the aquatic plant Lemna minor." NATURE BIOTECHNOLOGY, vol. 24, no. 12, December 2006 (2006-12), pages 1591-1597, XP009084966

## Description

## Background of the Invention

The CD30 cell surface molecule is a member of the tumor necrosis factor receptor (TNF-R) superfamily. This family of molecules has variable homology among its members and includes nerve growth factor receptor (NGFR), CD120(a), CD120(b), CD27, CD40 and CD95. These molecules are typically characterized by the presence of multiple cysteine-rich repeats in the extracytoplasmic region (de Bruin, P.C., et al. Leukemia 9:1620-1627 (1995)). Members of this family are considered crucial for regulating proliferation and differentiation of lymphocytes.

CD30 is a type I transmembrane glycoprotein with six (human) or three (murine and rat) cysteine-rich repeats with a central hinge sequence. CD30 exists as a 120 kDa membrane molecule which develops from an intercellular precursor protein of 90 kDa. It is shed from the cell surface as a soluble protein (sCD30) of approximately 90 kDa. Shedding of sCD30 occurs as an active process of viable CD30 cells and is not merely caused by the release from dying or dead cells. cDNAs encoding the CD30 protein have been cloned from expression libraries of the HLTV-1 human T-cell line HUT-102 by immunoscreening with monoclonal antibodies Ki-1 and Ber-H2 (Schwab, U., et al. Nature 299:65 (1982)). The mouse and rat CD30 cDNA has been found to encode 498 and 493 amino acids, respectively. Human CD30 cDNA encodes an additional 90 amino acids, partially duplicated from one of the cysteine rich domains. The CD30 gene has been mapped to 1p36 in humans and 5q36.2 in rats.

CD30 is preferentially expressed by activated lymphoid cells. Specifically, stimulation of CD30 in lymphoid cells has been shown to induce pleiotropic biological effects, including proliferation, activation, differentiation and cell death, depending on cell type, stage of differentiation and presence of other stimuli (Gruss, H.J. et al., Blood 83:2045-2056 (1994)). CD30 was originally identified by the monoclonal antibody Ki-1, which is reactive with antigens expressed on Hodgkin and Reed-Sternberg cells of Hodgkin's disease (Schwab et al., Nature 299:65 (1982)). Accordingly, CD30 is widely used as a clinical marker for Hodgkin's lymphoma and related hematological malignancies (Froese et al., J. Immunol. 139:2081 (1987); Carde et al., Eur. J. Cancer 26:474 (1990)).

CD30 was subsequently shown to be expressed on a subset of non-Hodgkin's lymphomas (NHL), including Burkitt's lymphoma, anaplastic large-cell lymphomas (ALCL), cutaneous T-cell lymphoma, nodular small cleaved-cell lymphomas, lymphocytic lymphomas, peripheral T-cell lymphomas, Lennert's lymphomas, immunoblastic lymphomas, T-cell leukemia/lymphomas (ATLL), adult T-cell leukemia (T-ALL), and entroblastic/centrocytic (cb/cc) follicular lymphomas (Stein et al., Blood 66:848 (1985); Miettinen, Arch. Pathol. Lab. Med. 116:1197 (1992); Piris et al., Histopathology 17:211 (1990); Burns et al., Am. J. Clin. Pathol. 93:327(1990); and Eckert et al., Am. J. Dermatopathol. 11:345 (1989)), as well as several virally-transformed lines such as human T-Cell Lymphotrophic Virus I or II transformed T-cells, and Epstein-Barr Virus transformed B-cells (Stein et al., Blood 66:848 (1985); Andreesen et al., Blood 63:1299 (1984)). In addition, CD30 expression has been documented in embryonal carcinomas, nonembryonal carcinomas, malignant melanomas, mesenchymal tumors, and myeloid cell lines and macrophage at late stages of differentiation (Schwarting et al., Blood 74:1678 (1989); Pallesen et al., Am J. Pathol. 133:446 (1988); Mechtersheimer et al., Cancer 66:1732 (1990); Andreesen et al., Am. J. Pathol. 134:187 (1989)).

Since the percentage of CD30-positive cells in normal individuals is quite small, the expression of CD30 in tumor cells renders it an important target for antibody mediated therapy to specifically target therapeutic agents against CD30-positive neoplastic cells (Chaiarle, R., et al. Clin. Immunol. 90(2):157-164 (1999)). Antibody mediated therapy has been shown to increase cytotoxicity of CD30-positive cells by both complement activation and antibody dependent cellular cytotoxicity (ADCE) (Pohl C., et al. Int J Cancer 54:418 (1993)). However, while the results obtained to date clearly establish CD30 as a useful target for immunotherapy, they also show that currently available murine antibodies do not constitute ideal therapeutic agents. Passive antibody therapy has not been effective *in vitro* or *in vivo* against patients with refractory Hodgkin's disease. A clinical trial of the anti-CD30 antibody Ber-H2 showed localization of the antibody, but no responses (Falini B. et al. (1992) Brit J Haematol. 82:38-45; Koon, H.B. et al. (2000) Curr Opin in Oncol. 12:588-593). Through coupling of an anti-CD30 antibody to a deglycosylated Ricin toxin-A chain toxin, cytotoxicity was shown in the treatment of human Hodgkin's Disease in a SCID mouse model, although grade 3 toxicities were also seen in the subjects (Schell, R. et al. (2002) Annals of Oncology 13:57-66). WO03/059282 discloses the characterization of the human anti-CD30 monoclonal antibodies 5F11, 17G1 and 2H9. Borchmann et al (Blood; 2003; 102(10) p3737-3742) also discloses the characterization of the antibody 5F11. Shinkawa et al (Journal of Biological Chemistry; 2003; 278(5) p3466-3473) and Yamane-Ohnuki et al (Biotechnology and Bioengineering; 2004; 87(5) p614-622) disclose methods of producing antibodies lacking fucose which have enhanced ADCC.

A number of plant species have been targeted for use in "molecular farming" of mammalian proteins of pharmaceutical interest. These plant expression systems provide for low cost production of biologically active mammalian proteins and are readily amenable to rapid and economical scale-up (Ma et al. (2003) Nat. Rev. Genet. 4:794-805; Raskin et al. (2002) Trends Biotechnol. 20:522-531). Gasdaska et al (Bioprocessing; 2003; 2 p49-56) and US 2004/261148 disclose the production of therapeutic proteins including antibodies, using the waterplant Lemna (duckweed). The differences in glycosylation patterns between plants and mammals offer a challenge to the feasibility of plant expression systems to produce high quality recombinant mammalian proteins for pharaceutical use. Methods are needed to alter the glycosylation pattern in plant expressed proteins, specifically to inhibit plant-specific glycosylation of the eukaryotic core structure, to advantageously produce recombinant mammalian proteins with a humanized glycosylation pattern.

Accordingly, the need exists for improved therapeutic antibodies against CD30 which are more effective for treating and/or preventing diseases mediated by CD30.

## Summary of the Invention

The present invention provides an isolated glycosylated anti-CD30 antibody, where at least 90% is a single glycoform, which lacks fucosyl and xylosyl residues, and which, optionally, does not contain galactosyl residues.

In another aspect, the invention pertains to:

- a glycoprotein composition comprising an anti-CD30 antibody composition comprising a an *N*-glycosylation profile wherein at least 90% of the *N*-glycans species present in said profile are GlcNAc2Man3GlcNAc2 (G0), said profile comprising a trace amount of precursor *N*-glycan species, wherein said precursor *N*-glycan species is selected from Man3GlcNAc2, GlcNaclMan3GlcNAc2 wherein GlcNac is attached to the 1,3 mannose arm (MGn), GlcNaclMan3GlcNAc2 wherein GlcNac is attached to the 1,6 mannose arm (GnM), and any combination thereof;
- a pharmaceutical composition comprising an antibody or glycoprotein composition of the invention;
- a host cell comprising a glycoprotein composition of the invention;
- a host cell comprising immunoglobulin heavy and light chain genes encoding an anti-CD30 antibody, wherein said host cell lacks a fucosyltransferase and a xylosyltransferase such that the anti-CD30 antibody expressed by said host cell lacks fucosyl and xylosyl residues, wherein the host cell is optionally a plant cell, wherein the plant cell is optionally a member of the *Lemnaceae* family, such as *Lemna minor;*
- an *in vitro* method of inhibiting growth of $CD30^+$ cells such as tumor cells comprising contacting said cells with an anti-CD30 antibody, where at least 90% is a single glycoform, which lacks fucosyl and xylosyl residues, under conditions sufficient to induce antibody-dependent cellular cytoxicity (ADCC) of said cells; and
- an anti-CD30 antibody, where at least 90% is a single glycoform, which lacks fucosyl and xylosyl residues, for use in a method of inhibiting growth of tumor cells expressing CD30 or in a method of treating an autoimmune disorder.

The antibody of the present invention binds to CD30 and inhibits the growth of cells expressing CD30 by enhancing antibody dependent cellular cytotoxicity (ADCC) in the presence of human effector cells (*e.g.*, monocytes or mononuclear cells), as compared to the fucosylated form of the antibody. In one embodiment, the antibody mediates increased ADCC of cells expressing CD30 in the presence of human effector cells but not in the presence of mouse effector cells.

Preferably, the antibody of the invention is a monoclonal antibody. In one aspect, the invention pertains to a humanized or chimeric monoclonal antibody. Preferably, the humanized or chimeric antibody is prepared from a mouse anti-CD30 antibody selected from the group consisting of: AC10, HeFi-1, Ber-H2, Ki-1, Ki-4, HRS-3, Irac, HRS-4, M44, M67, Ber-H8. In another aspect, the invention pertains to a human monoclonal antibody.

In another aspect, the invention provides a glycoprotein composition comprising an antibody composition, wherein the antibody composition comprises:

(a) a human heavy chain variable region CDR1 comprising an amino acid sequence selected from SEQ ID NOs: 7, 8, and 9;
(b) a human heavy chain variable region CDR2 comprising an amino acid sequence selected from SEQ ID NOs: 10, 11, and 12;
(c) a human heavy chain variable region CDR3 comprising an amino acid sequence selected from SEQ ID NOs: 13, 14, and 15;
(d) a human light chain variable region CDR1 comprising an amino acid sequence selected from SEQ ID NOs: 16, 17, and 18;
(e) a human light chain variable region CDR2 comprising an amino acid sequence selected from SEQ ID NOs: 19, 20, and 21; and
(f) a human light chain variable region CDR3 comprising an amino acid sequence selected from SEQ ID NOs: 22, 23, and 24.

In another aspect, the invention provides a glycoprotein composition comprising an antibody composition, wherein the antibody composition comprises:

(a) a human heavy chain variable region CDR1 comprising SEQ ID NO:7, a human heavy chain variable region CDR2 comprising SEQ ID NO: 10, a human heavy chain variable region CDR3 comprising SEQ ID NO: 13, a human light chain variable region CDR1 comprising SEQ ID NO: 16, a human light chain variable region CDR2 comprising SEQ ID NO:19 and a human light chain variable region CDR3 comprising SEQ ID NO:22;
(b) a human heavy chain variable region CDR1 comprising SEQ ID NO:8, a human heavy chain variable region CDR2 comprising SEQ ID NO: 11, a human heavy chain variable region CDR3 comprising SEQ ID NO:14, a human light chain variable region CDR1 comprising SEQ ID NO:17, a human light chain variable region CDR2 comprising SEQ ID NO:20 and a human light chain variable region CDR3 comprising SEQ ID NO:23;
(c) a human heavy chain variable region CDR1 comprising SEQ ID NO:9, a human heavy chain variable region CDR2 comprising SEQ ID NO:12, a human heavy chain variable region CDR3 comprising SEQ ID NO: 15, a human light chain variable region CDR1 comprising SEQ ID NO:18, a human light chain variable region CDR2 comprising SEQ ID NO:21 and a human light chain variable region CDR3 comprising SEQ ID NO:24; or a human heavy chain variable region comprising an amino acid sequence selected from SEQ ID NOs: 1, 2 and 3 and a human light chain variable region comprising an amino acid sequence selected from SEQ ID NOs: 4, 5 and 6.

In another aspect, the invention provides a glycoprotein composition comprising an antibody composition, wherein the antibody composition comprises:

(a) the antibody composition heavy chain variable region comprises the amino acid sequence of SEQ ID NO: 1 and the light chain variable region comprises the amino acid sequence of SEQ ID NO: 4;
(b) the antibody composition heavy chain variable region comprises the amino acid sequence of SEQ ID NO: 2 and the light chain variable region comprises the amino acid sequence of SEQ ID NO: 5; or
(c) the antibody composition heavy chain variable region comprises the amino acid sequence of SEQ ID NO: 3 and the light chain variable region comprises the amino acid sequence of SEQ ID NO: 6.

In another aspect, the invention provides a glycoprotein composition comprising an antibody composition, wherein the antibody composition comprises: a heavy chain variable region that is a product of or derived from a human $V_H$ 4-34 or $V_H$ 3-07 gene and/or a light chain variable region that is a product of or derived from a human $V_k$ L15, A27 or L6 gene.

**Brief Description of the Drawings**

Figure 1A shows the nucleotide sequence (SEQ ID NO: 30) and amino acid sequence (SEQ ID NO: 1) of the heavy chain variable region of the 5F11 human monoclonal antibody. The CDR1 (SEQ ID NO: 7), CDR2 (SEQ ID NO: 10) and CDR3 (SEQ ID NO: 13) regions are delineated and the V, D and J germline derivations are indicated.
Figure 1B shows the nucleotide sequence (SEQ ID NO: 33) and amino acid sequence (SEQ ID NO: 4) of the light chain variable region of the 5F11 human monoclonal antibody. The CDR1 (SEQ ID NO: 16), CDR2 (SEQ ID NO: 19) and CDR3 (SEQ ID NO:22) regions are delineated and the V and J germline derivations are indicated.
Figure 2A shows the nucleotide sequence (SEQ ID NO: 31) and amino acid sequence (SEQ ID NO: 2) of the heavy chain variable region of the 17G1 human monoclonal antibody. The CDR1 (SEQ ID NO: 8), CDR2 (SEQ ID NO: 11) and CDR3 (SEQ ID NO: 14) regions are delineated and the V and J germline derivations are indicated.
Figure 2B shows the nucleotide sequence (SEQ ID NO: 34) and amino acid sequence (SEQ ID NO: 5) of the light chain variable region of the 17G1 human monoclonal antibody. The CDR1 (SEQ ID NO: 17), CDR2 (SEQ ID NO: 20) and CDR3 (SEQ ID NO: 23) regions are delineated and the V and J germline derivations are indicated.
Figure 3A shows the nucleotide sequence (SEQ ID NO: 32) and amino acid sequence (SEQ ID NO: 3) of the heavy chain variable region of the 2H9 human monoclonal antibody. The CDR1 (SEQ ID NO: 9), CDR2 (SEQ ID NO: 12) and CDR3 (SEQ ID NO: 15) regions are delineated and the V, D, and J germline derivations are indicated.
Figure 3B shows the nucleotide sequence (SEQ ID NO: 35) and amino acid sequence (SEQ ID NO: 6) of the light chain variable region of the 2H9 human monoclonal antibody. The CDR1 (SEQ ID NO: 18), CDR2 (SEQ ID NO: 21) and CDR3 (SEQ ID NO: 24) regions are delineated and the V and J germline derivations are indicated.

Figure 4 is a graph showing the cytotoxic cell killing activity of the fucosylated and defucosylated forms of 5F11 on the L540 human Hodgkin's lymphoma cell line, as compared to an isotype-matched control antibody (1D4).
Figure 5 is a graph showing the cytotoxic cell killing activity of the fucosylated and defucosylated forms of 5F11 on the L428 human Hodgkin's lymphoma cell line, as compared to an isotype-matched control antibody (1D4).
Figure 6 is a graph showing the cytotoxic cell killing activity of the fucosylated and defucosylated forms of 5F11 on the L1236 human Hodgkin's lymphoma cell line, as compared to an isotype-matched control antibody (1D4).
Figure 7 is a graph showing the cytotoxic cell killing activity of the fucosylated and defucosylated forms of 5F11 on the Karpas human T cell lymphoma cell line, as compared to an isotype-matched control antibody (1D4).
Figures 8A-8B show the amino acid sequences of the human germlines $V_H$ 4-34, $V_H$ 3-07, $V_K$ L15, $V_K$ A27, and $V_K$ L6 (SEQ ID NOs:25-29, respectively), the CDRs are delineated.
Figure 9 is a graph showing blockade of ADCC activity with an anti-CD16 antibody.
Figure 10 is a graph showing the cytotoxic cell killing activity of the fucosylated and defucosylated forms of 5F11 in the presence of mouse (left panel) or human (right panel) effector cells.
Figure 11 is a graph showing an ADCC assay using cynomolgus blood.
Figure 12 shows glycosyltransferase activity in *Lemna* wild-type and 5F11 $LEX^{Opt}$ RNAi lines. Microsomal membranes from wild-type (WT) and 5F11 $LEX^{Opt}$ RNAi (line numbers are indicated) plants were incubated in the presence of a reaction buffer containing GDP-Fuc, UDP-Xyl and GnGn-dabsyl-peptide acceptor. Mass peaks corresponding to fucosylated (white bars) or xylosylated (black bars) products synthesized by microsomes from each line were measured by positive reflectron mode MALDI-TOF MS and normalized, in percent, to the WT positive control. Boiled wildtype membranes (BWT) indicate background ion counts.
Figure 13 shows SDS-PAGE of plant extracts and protein A or hydroxyapatite purified samples from 5F11 $LEX^{Opt}$ under non-reducing (Figure 13A) and reducing (Figure 13B) conditions, respectively. MAb purified from a CHO cell line (5F11 CHO) was used as a positive control. Mark12 molecular weight markers were included on the gels. Gels were stained with Colloidal Blue.
Figure 14 shows the spectra obtained from negative, reflectron mode MALDI-TOF mass spectrometric analysis of 2-AA labeled *N*-glycans released from 5F11 mAbs expressed in CHO (5F11 CHO), wild-type *Lemna* (5F11 LEX), or *Lemna* transformed with the XylT/FucT RNAi construct (5F11 $LEX^{Opt}$). Significant peaks are identified by the corresponding mass ($[M-H]^-$). The * indicates the location of matrix artefacts.
Figure 15 shows the spectra obtained from NP-HPLC-QTOF MS analysis of 2-AA labeled *N*-glycans released from 5F11 mAbs expressed in CHO (5F11 CHO), wild-type *Lemna* (5F11 LEX), *or Lemna* transformed with the XylT/FucT RNAi construct (5F11 $LEX^{Opt}$). 2-AA labeled *N*-glycans were separated by normal phase chromatography and detected by fluorescence. The most abundant peaks from each sample (labeled a-i) were characterized by online negative mode QTOF MS and their corresponding QTOF mass spectra ($[M-2H]^{2-}$) are shown.
Figure 16 shows *in vitro* activity of 5F11 mAbs as measured by flow cytometric analysis of 5F11 CHO, LEX, or glyco-optimized $LEX^{Opt}$ mAb binding to CD30 expressed on L540 cells. L540 cells were incubated with increasing concentrations of the indicated antibody as outlined in Example 6 herein below. Geo Mean Fluorescence Intensity (GMFI) is plotted against the various concentrations of mAb used. ■: 5F11 CHO; ▲: 5F11 LEX;▼: 5F11 $LEX^{opt}$.
Figure 17 shows equilibrium binding of glyco-optimized and wild-type mAb to two different human FcRγIIIa allotypes ($Val^{158}$ or $Phe^{158}$). The binding signal as a function of FcRγIIIa was fit to a one-site binding model. ■: 5F11 CHO; ▲: 5F11 LEX;▼: 5F11 $LEX^{opt}$.
Figure 18 shows ADCC activity of 5F11 mAb derived from CHO, LEX (wild-type *Lemna* glycosylation), or $LEX^{Opt}$ (RNAi transgenic *Lemna*). Human effector cells from a $Fc\gamma RIIIaPhe^{158}$ homozygote donor and a $Fc\gamma RIIIaPhe/Val^{158}$ heterozygote donor were incubated with BATDA-labeled L540 cells at an effector:target ratio of 50:1 in the presence of increasing concentrations of the indicated antibodies. Specific percent lysis at each mAb concentration is plotted. Human mAb1 not recognizing antigen on L540 cells was used as an isotype control in all experiments. $EC_{50}$ values (μg/mL), binding constants and maximal percent lysis were calculated using GraphPad Prism 3.0 software. ■: 5F11 CHO; ▲: 5F11 LEX; ▼: 5F11 $LEX^{opt}$.
Figure 19 shows intact mass analysis of the 5F11 LEX mAb compositions produced in wild-type *L. minor* comprising the MDXA01 construct. When XylT and FucT expression are not suppressed in *L. minor,* the recombinantly produced 5F11 LEX mAb composition comprises at least 7 different glycoforms, with the $G0XF^3$ glycoform being the predominate species present. Note the absence of a peak representing the G0 glycoform.
Figure 20 shows glycan mass analysis of the heavy chain of the 5F11 LEX mAb produced in wild-type *L. minor* comprising the MDXA01 construct. When XylT and FucT expression are not suppressed in *L. minor,* the predominate *N*-glycan species present is $GOXF^3$, with additional major peaks reflecting the G0X species. Note the minor presence of the G0 glycan species.
Figure 21 shows intact mass analysis of the 5F11 $LEX^{Opt}$ mAb compositions produced in transgenic *L. minor* comprising the MDXA04 construct. When Xy1T and FucT expression are suppressed in *L. minor,* the intact mAb composition contains only G0 *N*-glycans. In addition, the composition is substantially homogeneous for the G0

glycoform (peak 2), wherein both glycosylation sites are occupied by the G0 *N*-glycan species, with two minor peaks reflecting trace amounts of precursor glycoforms (peak 1, showing mAb having an Fc region wherein the $C_H2$ domain of one heavy chain has a G0 glycan species attached to Asn 297, and the $C_H2$ domain of the other heavy chain is unglycosylated; and peak 3, showing mAb having an Fc region wherein the Asn 297 glycosylation site on each of the $C_H2$ domains has a G0 glycan species attached, with a third G0 glycan species attached to an additional glycosylation site within the mAb structure).

Figure 22 shows glycan mass analysis of the heavy chain of the 5F11 $LEX^{Opt}$ mAb produced in transgenic *L. minor* comprising the MDXA04 construct. When XylT and FucT expression are suppressed in *L. minor,* the only readily detectable *N*-glycan species attached to the Asn 297 glycosylation sites of the $C_H2$ domains of the heavy chains is G0.

**Detailed Description of the Invention**

The present invention provides an isolated glycosylated anti-CD30 antibody, where at least 90% is a single glycoform, which lacks fucosyl and xylosyl residues, and which, optionally, does not contain galactosyl residues.

Furthermore, the antibodies exhibit enhanced antibody directed cellular cytotoxic (ADCC) killing of CD30+ cells. In one embodiment, the antibodies of the present invention are fully human antibodies and are particularly useful for the therapeutic treatment in humans of disorders associated with CD30 expressing cells. Methods of using anti-CD30 antibodies for therapeutic treatment (*e.g.*, to treat and/or prevent diseases associated with expression of CD30) are also encompassed by the invention.

In order that the present invention may be more readily understood, certain terms will be defined as follows. Additional definitions are set forth throughout the detailed description.

The terms "CD30" and "CD30 antigen" are used interchangeably herein, and include any variants, isoforms and species homologs of human CD30 which are naturally expressed by cells. The complete amino acid sequence of human CD30 protein has the Genbank accession number NP_001234. The complete cDNA sequence encoding the human CD30 protein has the Genbank accession number NM_001243.

As used herein, the terms "antibody that lacks fucosyl residues", "defucosylated antibody," and "nonfucosylated antibody" are used interchangeably and are intended to refer to an antibody in which the carbohydrate portion of the antibody does not contain a fucosyl residue or from which the fucosyl residue has been removed. An antibody that lacks fucosyl residues can be generated, for example, by expression of the antibody in a cell or expression system that minimizes or does not attach fucosyl residues to the antibody carbohydrate chain, or by chemical modification of the antibody to remove fucosyl residues from the carbohydrate chain (e.g., treatment of the antibody with a fucosidase). As such, the terms "lacks fucosyl residues" and "defucosylated" are not intended to be limited by the mechanism by which the antibody with altered carbohydrate structure is prepared.

As used herein, the terms "antibody that lacks xylosyl residues," "dexylosylated antibody," and "nonxylosylated antibody" are used interchangeably and are intended to refer to an antibody in which the carbohydrate portion of the antibody does not contain a xylosyl residue or from which the xylosyl residue has been removed. An antibody that lacks xylosyl residues can be generated, for example, by expression of the antibody in a cell or expression system that minimizes or does not attach xylosyl residues to the antibody carbohydrate chain, or by chemical modification of the antibody to remove xylosyl residues from the carbohydrate chain (e.g., treatment of the antibody with a xylosidase). As such, the terms "lacks xylosyl residues" and "dexylosylated" are not intended to be limited by the mechanism by which the antibody with altered carbohydrate structure is prepared.

As used herein, the term "antibody expressing fucosyl residues" and "fucosylated antibody" are used interchangeably and are intended to refer to an antibody in which the carbohydrate portion of the antibody contains fucosyl.

As used herein, the term "antibody expressing xylosyl residues" and "xylosylated antibody" are used interchangeably and are intended to refer to an antibody in which the carbohydrate portion of the antibody contains xylosyl.

For purposes of the present invention, the terms "N-glycan," "N-linked glycan," and "glycan" are used interchangeably and refer to an N-linked oligosaccharide, e.g., one that is or was attached by an N-acetylglucosamine (GlcNAc) residue linked to the amide nitrogen of an asparagine residue in a protein. The predominant sugars found on glycoproteins are glucose, galactose, mannose, fucosyl, N-acetylgalactosamine (GalNAc), N-acetylglucosamine (GlcNAc), and sialic acid (e.g., N-acetyl-neuraminic acid (NeuAc)). The processing of the sugar groups occurs cotranslationally in the lumen of the ER and continues in the Golgi apparatus for N-linked glycoproteins.

The N-glycans attached to glycoproteins differ with respect to the number of branches (antennae) comprising peripheral sugars (e.g., GlcNAc, galactose, fucosyl, and sialic acid) that are added to the trimannose core structure. N-glycans are commonly classified according to their branched constituents (e.g., complex, high mannose, or hybrid). A "complex" type N-glycan typically has at least one GlcNAc attached to the 1,3 mannose arm and at least one GlcNAc attached to the 1,6 mannose arm of a "trimannose" core. Where one GlcNAc is attached to each mannose arm, the species of N-linked glycan is denoted herein as "GlcNAc2Man3GlcNAc2" or "GnGn." Where only one GlcNac is attached, the N-glycan species is denoted herein as "GlcNAclMan3GlcNAc2", wherein the GlcNac is attached to either the 1,3

mannose arm (denoted "MGn" herein) or the 1,6 mannose arm (denoted "GnM" herein) (see Figure 30). Complex N-glycans may also have galactose ("Gal") or N-acetylgalactosamine ("GalNAc") sugar residues that are optionally modified with sialic acid or derivatives (e.g., "NeuAc," where "Neu" refers to neuraminic acid and "Ac" refers to acetyl). Where a galactose sugar residue is attached to each GlcNAc on each mannose arm, the species of N-linked glycan is denoted herein as "Gal2GlcNAc2Man3GlcNAc2." Complex N-glycans may also have intrachain substitutions comprising "bisecting" GlcNAc and core fucosyl ("Fuc"). Complex N-glycans may also have multiple antennae on the "trimannose core," often referred to as "multiple antennary glycans." A "high mannose" type N-glycan has five or more mannose residues. A "hybrid" N-glycan has at least one GlcNAc on the terminal of the 1,3 mannose arm of the trimannose core and zero or more mannoses on the 1,6 mannose arm of the trimannose core.

The terms "G0 glycan" and "G0 glycan structure" and "G0 glycan species" are used interchangeably and are intended to mean the complex N-linked glycan having the GlcNAc2Man3GlcNAc2 structure, wherein no terminal sialic acids (NeuAcs) or terminal galactose (Gal) sugar residues are present. If a G0 glycan comprises a fucosyl ("Fuc") residue attached to the trimannose core structure, it is referred to herein as a "G0F3 glycan" (having the plant-specific $\alpha$1,3-linked fucosyl residue) or "G0F6 glycan" (having the mammalian $\alpha$1,6-linked fucosyl residue). In plants, a G0 glycan comprising the plant-specific $\beta$ 1,2-linked xylosyl residue attached to the trimannose core structure is referred to herein as a "G0X glycan," and a G0 glycan comprising both the plant-specific $\beta$ 1,2-linked xylosyl residue and plant-specific $\alpha$1,3-linked fucosyl residue attached to the trimannose core structure is referred to herein as a "G0XF3 glycan."

The terms "substantially homogeneous," "substantially uniform," "substantially a single glycoform," and "substantial homogeneity" in the context of a glycosylation profile for a glycoprotein composition or glycoprotein product are used interchangeably and are intended to mean a glycosylation profile wherein at least 90%, 91%, 92%, 93%, 94%, 95%, 96%, 97%, 98%, or at least 99% of the total N-glycan species within the profile are represented by one desired N-glycan species, with a trace amount of precursor N-glycan species appearing in the profile. By "trace amount" is intended that any given precursor N-glycan species that is present in the glycosylation profile is present at less than 5%, preferably less than 4%, less than 3%, less than 2%, less than 1%, and even less than 0.5% or even less than 0.1% of the total amount ofN-glycan species appearing in the profile. By "precursor" N-glycan species is intended an N-glycan species that is incompletely processed. Examples of precursor N-glycan species present in trace amounts in the glycoprotein compositions or glycoprotein products of the invention, and thus appearing in the glycosylation profiles thereof, are the Man3GlcNAc2, MGn (GlcNac1Man3GlcNAc2 wherein GlcNac1 is attached to the 1,3 mannose arm), and GnM (GlcNac1Man3GlcNAc2 wherein GlcNac1 is attached to the 1,6 mannose arm) precursor N-glycan species described above.

Thus, for example, where the desired N-glycan species within a glycoprotein product or composition is G0, a substantially homogeneous glycosylation profile for that product or composition would be one wherein at least 90%, 91%, 92%, 93%, 94%, 95%, 96%, 97%, 98%, or at least 99% of the total amount of N-glycan species appearing in the glycosylation profile for the product or composition is represented by the G0 glycan species, with a trace amount of precursor N-glycan species appearing in the glycosylation profile. For such a composition, a representative precursor N-glycan species appearing in its glycosylation profile would be the Man3GlcNAc2, MGn (GlcNac1Man3GlcNAc2 wherein GlcNac1 is attached to the 1,3 mannose arm), and GnM (GlcNac1Man3GlcNAc2 wherein GlcNac1 is attached to the 1,6 mannose arm) precursor N-glycan species described above.

The term "glyco-optimized" refers to an antibody having a particular N-glycan structure that produces certain desireable properties, including but not limited to, enhanced antibody-dependent cell-mediated cytotoxicity (ADCC) and effector cell receptor binding activity when compared to CHO-expressed antibodies.

The term "duckweed" refers to members of the family *Lemnaceae.* This family currently is divided into five genera and 38 species of duckweed as follows: genus *Lemna (L. aequinoctialis, L. disperma, L. ecuadoriensis, L. gibba, L japonica, L. minor, L. miniscula, L. obscura, L. perpusilla, L. tenera, L. trisulea, L. turionifera, L. valdiviana);* genus *Spiroddla (S. intermedia, S. polyrrhiza, S. punctata*)*;* genus *Wolffia (Wa. angusta, Wa. arrhiza, Wa. australina, Wa. borealis, Wa. brasiliensis, Wa. columbiana, Wa. elongata, Wa. globosa, Wa. microscopica, Wa. neglecta*)*;* genus *Wolfiella* (*Wl. caudata, Wl. denticulata, Wl. gladiata, Wl. hyalina, Wl. lingulata, Wl. repunda, Wl. rotunda, and Wl. neotropica*) and genus *Landoltia (L. punctata).* Any other genera or species *of Lemnaceae,* if they exist, are also aspects of the present invention. *Lemna* species can be classified using the taxonomic scheme described by Landolt (1986) *Biosystematic Investigation on the Family of Duckweeds: The family of Lemnaceae-A Monograph Study* (Geobatanischen Institut ETH, Stiftung Rubel, Zurich).

The term "immune response" refers to the action of, for example, lymphocytes, antigen presenting cells, phagocytic cells, granulocytes, and soluble macromolecules produced by the above cells or the liver (including antibodies, cytokines, and complement) that results in selective damage to, destruction of, or elimination from the human body of invading pathogens, cells or tissues infected with pathogens, cancerous cells, or, in cases of autoimmunity or pathological inflammation, normal human cells or tissues.

A "signal transduction pathway" refers to the biochemical relationship between various of signal transduction molecules that play a role in the transmission of a signal from one portion of a cell to another portion of a cell. As used

herein, the phrase "cell surface receptor" includes, for example, molecules and complexes of molecules capable of receiving a signal and the transmission of such a signal across the plasma membrane of a cell. An example of a "cell surface receptor" of the present invention is the CD30 receptor.

As used herein, the term "effector cell" refers to an immune cell which is involved in the effector phase of an immune response, as opposed to the cognitive and activation phases of an immune response. Exemplary immune cells include a cell of a myeloid or lymphoid origin, *e.g.*, lymphocytes (*e.g.*, B cells and T cells including cytolytic T cells (CTLs)), killer cells, natural killer cells, macrophages, monocytes, eosinophils, neutrophils, polymorphonuclear cells, granulocytes, mast cells, and basophils. Some effector cells express specific Fc receptors and carry out specific immune functions. In preferred embodiments, an effector cell is capable of inducing antibody-dependent cell-mediated cytotoxicity (ADCC), e.g., a neutrophil capable of inducing ADCC. For example, monocytes and macrophages, which express FcR are involved in specific killing of target cells and presenting antigens to other components of the immune system, or binding to cells that present antigens. In other embodiments, an effector cell can phagocytose a target antigen or target cell. The expression of a particular FcR on an effector cell can be regulated by humoral factors such as cytokines. For example, expression of Fc$\alpha$RI has been found to be up-regulated by G-CSF or GM-CSF. This enhanced expression increases the effector function of Fc$\alpha$RI-bearing cells against targets. An effector cell can phagocytose or lyse a target antigen or a target cell.

"Target cell" refers to any cell or pathogen whose elimination would be beneficial in a subject (e.g., a human or animal) and that can be targeted by a composition (e.g., antibody) of the invention. For example, the target cell can be a cell expressing or overexpressing CD30.

The term "antibody-dependent cellular cytotoxicity" or "ADCC" refers to a cell-mediated cytotoxic reaction in which a CD30+ target cell with bound anti-CD30 antibody is recognized by an effector cell bearing Fc receptors and is subsequently lysed without requiring the involvement of complement.

As used herein, the term "enhances ADCC" (*e.g.,* referring to cells) is intended to include any measurable increase in cell lysis when contacted with an anti-CD30 antibody lacking fucosyl and xylosyl residues as compared to the cell killing of the same cell in contact with a fucosylated and xylosylated anti-CD30 antibody in the presence of effector cells (for example, at a ratio of target cells:effector cells of 1:50), *e.g.*, an increase in cell lysis by at least about 10%, 20%, 30%, 40%, 50%, 60%, 70%, 80%, 90%, 100%, 150%, 200%, 250%, 300%, or 325%.

The term "antibody" as referred to herein includes whole antibodies and any antigen binding fragment (*i.e.*, "antigen-binding portion") or single chains thereof. An "antibody" refers to a glycoprotein comprising at least two heavy (H) chains and two light (L) chains inter-connected by disulfide bonds, or an antigen binding portion thereof. Each heavy chain is comprised of a heavy chain variable region (abbreviated herein as $V_H$) and a heavy chain constant region. The heavy chain constant region is comprised of three domains, $C_{H1}$, $C_{H2}$ and $C_{H3}$. Each light chain is comprised of a light chain variable region (abbreviated herein as $V_L$) and a light chain constant region. The light chain constant region is comprised of one domain, $C_L$. The $V_H$ and $V_L$ regions can be further subdivided into regions of hypervariability, termed complementarity determining regions (CDR), interspersed with regions that are more conserved, termed framework regions (FR). Each $V_H$ and $V_L$ is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The term "antigen-binding portion" of an antibody (or simply "antibody portion"), as used herein, refers to one or more fragments of an antibody that retain the ability to specifically bind to an antigen (e.g., CD30). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the $V_L$, $V_H$, $C_L$ and $C_H1$ domains; (ii) a $F(ab')_2$ fragment, a bivalent fragment comprising two Fab fragments linked by a disulfide bridge at the hinge region; (iii) a Fab' fragment, which is essentially an Fab with part of the hinge region (see, FUNDAMENTAL IMMUNOLOGY (Paul ed., 3.sup.rd ed. 1993); (iv) a Fd fragment consisting of the $V_H$ and $C_H1$ domains; (v) a Fv fragment consisting of the $V_L$ and $V_H$ domains of a single arm of an antibody, (vi) a dAb fragment (Ward et al., (1989) Nature 341:544-546), which consists of a $V_H$ domain; (vii) an isolated complementarity determining region (CDR); and (viii) a nanobody, a heavy chain variable region containing a single variable domain and two constant domains. Furthermore, although the two domains of the Fv fragment, $V_L$ and $V_H$, are coded for by separate genes, they can be joined, using recombinant methods, by a synthetic linker that enables them to be made as a single protein chain in which the $V_L$ and $V_H$ regions pair to form monovalent molecules (known as single chain Fv (scFv); *see e.g.,* Bird et al. (1988) Science 242:423-426; and Huston et al. (1988) Proc. Natl. Acad Sci. USA 85:5879-5883). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies.

The term "recombinant human antibody", as used herein, includes all human antibodies that are prepared,

expressed, created or isolated by recombinant means, such as (a) antibodies isolated from an animal (*e.g.*, a mouse) that is transgenic or transchromosomal for human immunoglobulin genes or a hybridoma prepared therefrom (described further below), (b) antibodies isolated from a host cell transformed to express the human antibody, *e.g.,* from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable regions in which the framework and CDR regions are derived from human germline immunoglobulin sequences. In certain embodiments, however, such recombinant human antibodies can be subjected to *in vitro* mutagenesis (or, when an animal transgenic for human Ig sequences is used, *in vivo* somatic mutagenesis) and thus the amino acid sequences of the $V_H$ and $V_L$ regions of the recombinant antibodies are sequences that, while derived from and related to human germline $V_H$ and $V_L$ sequences, may not naturally exist within the human antibody germline repertoire *in vivo.*

The terms "monoclonal antibody" or "monoclonal antibody composition" as used herein refer to a preparation of antibody molecules of single molecular composition. A monoclonal antibody composition displays a single binding specificity and affinity for a particular epitope.

The phrases "an antibody recognizing an antigen" and "an antibody specific for an antigen" are used interchangeably herein with the term "an antibody which binds specifically to an antigen."

The term "human antibody derivatives" refers to any modified form of the human antibody, e.g., a conjugate of the antibody and another agent or antibody.

The term "human antibody", as used herein, is intended to refer to antibodies having variable regions in which both the framework and CDR regions are derived from human germline immunoglobulin sequences. Furthermore, if the antibody contains a constant region, the constant region also is derived from human germline immunoglobulin sequences. The human antibodies of the invention may include amino acid residues not encoded by human germline immunoglobulin sequences (*e.g.*, mutations introduced by random or site-specific mutagenesis *in vitro* or by somatic mutation *in vivo*).

The term "human monoclonal antibody" refers to antibodies displaying a single binding specificity which have variable regions in which both the framework and CDR regions are derived from human germline immunoglobulin sequences. In one embodiment, the human monoclonal antibodies are produced by a hybridoma which includes a B cell obtained from a transgenic nonhuman animal, *e.g.*, a transgenic mouse, having a genome comprising a human heavy chain transgene and a light chain transgene fused to an immortalized cell. The term "human monoclonal antibody", as used herein, also includes all human antibodies that are prepared, expressed, created or isolated by recombinant means, such as (a) antibodies isolated from an animal (*e.g.*, a mouse) that is transgenic or transchromosomal for human immunoglobulin genes or a hybridoma prepared therefrom (described further below), (b) antibodies isolated from a host cell transformed to express the human antibody, *e.g.*, from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable regions in which the framework and CDR regions are derived from human germline immunoglobulin sequences. In certain embodiments, however, such recombinant human antibodies can be subjected to *in vitro* mutagenesis (or, when an animal transgenic for human Ig sequences is used, *in vivo* somatic mutagenesis) and thus the amino acid sequences of the $V_H$ and $V_L$ regions of the recombinant antibodies are sequences that, while derived from and related to human germline $V_H$ and $V_L$ sequences, may not naturally exist within the human antibody, germline repertoire *in vivo*.

An "isolated antibody," as used herein, is intended to refer to an antibody which is substantially free of other antibodies having different antigenic specificities (*e.g.*, an isolated antibody that specifically binds to CD30 is substantially free of antibodies that specifically bind antigens other than CD30). An isolated antibody that specifically binds to an epitope, isoform or variant of human CD30 may, however, have cross-reactivity to other related antigens, *e.g.*, from other species (*e.g.*, CD30 species homologs). Moreover, an isolated antibody may be substantially free of other cellular material and/or chemicals. In one embodiment of the invention, a combination of "isolated" monoclonal antibodies having different specificities are combined in a well defined composition.

The term "humanized antibody" is intended to refer to antibodies in which CDR sequences derived from the germline of another mammalian species, such as a mouse, have been grafted onto human framework sequences. Additional framework region modifications may be made within the human framework sequences.

The term "chimeric antibody" is intended to refer to antibodies in which the variable region sequences are derived from one species and the constant region sequences are derived from another species, such as an antibody in which the variable region sequences are derived from a mouse antibody and the constant region sequences are derived from a human antibody.

As used herein, an antibody that "specifically binds to human CD30" is intended to refer to an antibody that binds to human CD30 with a $K_D$ of 1 x $10^{-7}$ M or less, more preferably 5 x $10^{-8}$ M or less, more preferably 3 x $10^{-8}$ M or less, more preferably 1 x $10^{-8}$ M or less, even more preferably 5 x $10^{-9}$ M or less.

The term "does not substantially bind" to a protein or cells, as used herein, means does not bind or does not

bind with a high affinity to the protein or cells, i.e. binds to the protein or cells with a $K_D$ of 1 x $10^{-6}$ M or more, more preferably 1 x $10^{-5}$ M or more, more preferably 1 x $10^{-4}$ M or more, more preferably 1 x $10^{-3}$ M or more, even more preferably 1 x $10^{-2}$ M or more.

The term "$K_{assoc}$" or "$K_a$," as used herein, is intended to refer to the association rate of a particular antibody-antigen interaction, whereas the term "$K_{dis}$" or "$K_d$," as used herein, is intended to refer to the dissociation rate of a particular antibody-antigen interaction. The term "$K_D$," as used herein, is intended to refer to the dissociation constant, which is obtained from the ratio of $K_d$ to $K_a$ (i.e,. $K_d/K_a$) and is expressed as a molar concentration (M). $K_D$ values for antibodies can be determined using methods well established in the art. A preferred method for determining the $K_D$ of an antibody is by using surface plasmon resonance, preferably using a biosensor system such as a Biacore® system.

As used herein, the term "high affinity" for an IgG antibody refers to an antibody having a $K_D$ of 1 x $10^{-7}$ M or less, more preferably 5 x $10^{-8}$ M or less, even more preferably 1x$10^{-8}$ M or less, even more preferably 5 x $10^{-9}$ M or less and even more preferably 1 x $10^{-9}$ M or less for a target antigen. However, "high affinity" binding can vary for other antibody isotypes. For example, "high affinity" binding for an IgM isotype refers to an antibody having a $K_D$ of $10^{-6}$ M or less, more preferably $10^{-7}$ M or less, even more preferably $10^{-8}$ M or less.

The term "epitope" means a protein determinant capable of specific binding to, or specific binding by, an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

As used herein, "specific binding" refers to antibody binding to a predetermined antigen. Typically, the antibody binds with a dissociation constant ($K_D$) of $10^{-7}$ M or less, and binds to the predetermined antigen with a $K_D$ that is at least two-fold less than its $K_D$ for binding to a non-specific antigen (*e.g.*, BSA, casein) other than the predetermined antigen or a closely-related antigen. The phrases "an antibody recognizing an antigen" and "an antibody specific for an antigen" are used interchangeably herein with the term "an antibody which binds specifically to an antigen".

As used herein, "isotype" refers to the antibody class (*e.g.*, IgM or IgGl) that is encoded by heavy chain constant region genes.

The term "vector," as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e.g.*, bacterial vectors having a bacterial origin of replication and episomal mammalian vectors). Other vectors (*e.g.*, non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (*e.g.*, replication defective retroviruses, adenoviruses and adeno-associated viruses), which serve equivalent functions.

The term "recombinant, host cell" (or simply "host cell"), as used herein, is intended to refer to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein. Recombinant host cells include, for example, *Lemna* cells, CHO cells, transfectomas, and lymphocytic cells.

As used herein, the term "subject" includes any human or nonhuman animal. The term "nonhuman animal" includes all vertebrates, *e.g.*, mammals and non-mammals, such as nonhuman primates, sheep, dogs, cats, horses, cows, chickens, amphibians, reptiles, etc.

The terms "transgenic, nonhuman animal" refers to a nonhuman animal having a genome comprising one or more human heavy and/or light chain transgenes or transchromosomes (either integrated or non-integrated into the animal's natural genomic DNA) and which is capable of expressing fully human antibodies. For example, a transgenic mouse can have a human light chain transgene and either a human heavy chain transgene or human heavy chain transchromosome, such that the mouse produces human anti-CD30 antibodies when immunized with CD30 antigen and/or cells expressing CD30. The human heavy chain transgene can be integrated into the chromosomal DNA of the mouse, as is the case for transgenic, *e.g.*, HuMAb mice, or the human heavy chain transgene can be maintained extrachromosomally, as is the case for transchromosomal (*e.g.*, KM) mice as described in WO 02/43478. Such transgenic and transchromosomal mice are capable of producing multiple isotypes of human monoclonal antibodies to CD30 (*e.g.*, IgG, IgA and/or IgE) by undergoing V-D-J recombination and isotype switching.

Various aspects of the invention are described in further detail in the following subsections.

Human Monoclonal Anti-CD30 Antibodies

Preferred antibodies of the invention include human anti-CD30 monoclonal antibodies. Examples of human anti-CD30 monoclonal antibodies include the 5F11, 17G1, and 2H9 antibodies, isolated and structurally characterized as described in PCT Publication WO 03/059282, U.S. Pat. Publ. No. 2004/0006215 and Lahive and Cockfield LLP attorney docket No. MXI-333-1. The $V_H$ amino acid sequences of 5F11, 17G1, and 2H9 are shown in SEQ ID NOs: 1, 2, and 3, respectively. The $V_L$ amino acid sequences of 5F11, 17G1, and 2H9 are shown in SEQ ID NOs: 4, 5, and 6, respectively.

Given that each of these antibodies can bind to CD30, the $V_H$ and $V_L$ sequences can be "mixed and matched" to create other anti-CD30 binding molecules of the invention. CD30 binding of such "mixed and matched" antibodies can be tested using the binding assays well known in the art, such as FACS analysis and ELISA assays. Preferably, when $V_H$ and $V_L$ chains are mixed and matched, a $V_H$ sequence from a particular $V_H$/$V_L$ pairing is replaced with a structurally similar $V_H$ sequence. Likewise, preferably a $V_L$ sequence from a particular $V_H$/$V_L$ pairing is replaced with a structurally similar $V_L$ sequence. For example, the $V_H$ sequences of SF11 and 2H9 are particularly amenable for mixing and matching, since these antibodies use $V_H$ sequences derived from the same germline sequence ($V_H$ 4-34) and thus they exhibit structural similarity.

In particular embodiments, the invention provides a defucosylated and dexylosylated monoclonal antibody, or antigen binding portion thereof, comprising:

(a) a heavy chain variable region comprising an amino acid sequence selected from the group consisting of SEQ ID NOs: 1, 2, and 3; and
(b) a light chain variable region comprising an amino acid sequence selected from the group consisting of SEQ ID NO: 4, 5, and 6;
wherein the antibody specifically binds human CD30.

Preferred heavy and light chain combinations include:

(a) a heavy chain variable region comprising the amino acid sequence of
SEQ ID NO: 1; and (b) a light chain variable region comprising the amino acid sequence of SEQ ID NO: 4; or
(a) a heavy chain variable region comprising the amino acid sequence of
SEQ ID NO: 2; and (b) a light chain variable region comprising the amino acid sequence of SEQ ID NO: 5; or
(a) a heavy chain variable region comprising the amino acid sequence of
SEQ ID NO: 3; and (b) a light chain variable region comprising the amino acid sequence of SEQ ID NO: 6.

In another aspect, the invention provides antibodies that comprise the heavy chain and light chain CDR1s, CDR2s and CDR3s of 5F11, 17G1, and 2H9, or combinations thereof. The amino acid sequences of the $V_H$ CDR1s of 5F11, 17G1, and 2H9 are shown in SEQ ID NOs: 7, 8, and 9, respectively. The amino acid sequences of the $V_H$ CDR2s of 5F11, 17G1, and 2H9 are shown in SEQ ID NOs: 10, 11, and 12, respectively. The amino acid sequences of the $V_H$ CDR3s of 5F11, 17G1, and 2H9 are shown in SEQ ID NOs: 13, 14, and 15, respectively. The amino acid sequences of the $V_K$ CDR1s of 5Fl1, 17G1, and 2H9 are shown in SEQ ID NOs: 16, 17, and 18, respectively. The amino acid sequences of the $V_K$ CDR2s of 5F11, 17G1, and 2H9 are shown in SEQ ID NOs: 19, 20, and 21, respectively. The amino acid sequences of the $V_K$ CDR3s of 5F11, 17G1, and 2H9 are shown in SEQ ID NOs: 22, 23, and 24, respectively. The CDR regions are delineated using the Kabat system (Kabat, E. A., et al. (1991) Sequences of Proteins of Immunological Interest, Fifth Edition, U.S. Department of Health and Human Services, NIH Publication No. 91-3242).

Given that each of these antibodies can bind to CD30 and that antigen-binding specificity is provided primarily by the CDR1, 2 and 3 regions, the $V_H$ CDR1, 2 and 3 sequences and $V_k$ CDR1, 2 and 3 sequences can be "mixed and matched" (*i.e.*, CDRs from different antibodies can be mixed and match, although each antibody must contain a $V_H$ CDR1, 2 and 3 and a $V_k$ CDR1, 2 and 3) to create other anti-CD30 binding molecules of the invention. CD30 binding of such "mixed and matched" antibodies can be tested using binding assays know in the art, for example, FACS analysis and ELISA assays. Preferably, when $V_H$ CDR sequences are mixed and matched, the CDR1, CDR2 and/or CDR3 sequence from a particular $V_H$ sequence is replaced with a structurally similar CDR sequence(s). Likewise, when $V_k$ CDR sequences are mixed and matched, the CDR1, CDR2 and/or CDR3 sequence from a particular $V_k$ sequence preferably is replaced with a structurally similar CDR sequence(s). It will be readily apparent to the ordinarily skilled artisan that novel $V_H$ and $V_L$ sequences can be created by substituting one or more $V_H$ and/or $V_L$ CDR region sequences with structurally similar sequences from the CDR sequences disclosed herein for monoclonal antibodies antibodies 5F11, 17G1, and 2H9.

Accordingly, in another aspect, the invention provides an antibody comprising:

(a) a heavy chain variable region CDR1 comprising an amino acid sequence selected from the group consisting of SEQ ID NOs: 7, 8, and 9;
(b) a heavy chain variable region CDR2 comprising an amino acid sequence selected from the group consisting of SEQ ID NOs: 10, 11, and 12;
(c) a heavy chain variable region CDR3 comprising an amino acid sequence selected from the group consisting of SEQ ID NOs: 13, 14, and 15;
(d) a light chain variable region CDR1 comprising an amino acid sequence selected from the group consisting of SEQ ID NOs: 16, 17, and 18;
(e) a light chain variable region CDR2 comprising an amino acid sequence selected from the group consisting of SEQ ID NOs: 19, 20, and 21; and
(f) a light chain variable region CDR3 comprising an amino acid sequence selected from the group consisting of SEQ ID NOs: 22, 23, and 24;

wherein the antibody specifically binds CD30.

In a preferred embodiment, the antibody comprises:

(a) a heavy chain variable region CDR1 comprising SEQ ID NO: 7;
(b) a heavy chain variable region CDR2 comprising SEQ ID NO: 10;
(c) a heavy chain variable region CDR3 comprising SEQ ID NO: 13;
(d) a light chain variable region CDR1 comprising SEQ ID NO: 16;
(e) a light chain variable region CDR2 comprising SEQ II7 NO: 19; and
(f) a light chain variable region CDR3 comprising SEQ ID NO: 22.

In another preferred embodiment, the antibody comprises:

(a) a heavy chain variable region CDR1 comprising SEQ ID NO: 8;
(b) a heavy chain variable region CDR2 comprising SEQ ID NO: 11;
(c) a heavy chain variable region CDR3 comprising SEQ ID NO: 14;
(d) a light chain variable region CDR1 comprising SEQ ID NO: 17;
(e) a light chain variable region CDR2 comprising SEQ ID NO: 20; and
(f) a light chain variable region CDR3 comprising SEQ ID NO: 24.

In another preferred embodiment, the antibody comprises:

(a) a heavy chain variable region CDR1 comprising SEQ ID NO: 9;
(b) a heavy chain variable region CDR2 comprising SEQ ID NO: 12;
(c) a heavy chain variable region CDR3 comprising SEQ ID NO: 15;
(d) a light chain variable region CDR1 comprising SEQ ID NO: 18;
(e) a light chain variable region CDR2 comprising SEQ ID NO: 21; and
(f) a light chain variable region CDR3 comprising SEQ ID NO: 24.

It is well known in the art that the CDR3 domain, independently from the CDR1 and/or CDR2 domain(s), alone can determine the binding specificity of an antibody for a cognate antigen and that multiple antibodies can predictably be generated having the same binding specificity based on a common CDR3 sequence. *See,* for example, Klimka et al., British J. of Cancer 83(2):252-260 (2000) (describing the production of a humanized anti-CD30 antibody using only the heavy chain variable domain CDR3 of murine anti-CD30 antibody Ki-4); Beiboer et al., J. Mol. Biol. 296:833-849 (2000) (describing recombinant epithelial glycoprotein-2 (EGP-2) antibodies using only the heavy chain CDR3 sequence of the parental.murine MOC-31 anti-EGP-2 antibody); Rader et al., Proc. Natl. Acad Sci. U.S.A 95:8910-8915 (1998) (describing a panel of humanized anti-integrin $\alpha_v\beta_3$ antibodies using a heavy and light chain variable CDR3 domain of a murine anti-integrin $\alpha_v\beta_3$ antibody LM609 wherein each member antibody comprises a distinct sequence outside the CDR3 domain and capable of binding the same epitope as the parent muring antibody with affinities as high or higher than the parent murine antibody); Barbas et al., J. Am. Chem. Soc. 116:2161-2162 (1994) (disclosing that the CDR3 domain provides the most significant contribution to antigen binding); Barbas et al., Proc. Natl. Acad Sci. U.S.A. 92: 2529-2533 (1995) (describing the grafting of heavy chain CDR3 seqeunces of three Fabs (SI-1, SI-40, and SI-32) against human placental DNA onto the heavy chain of an anti-tetanus toxoid Fab thereby replacing the existing heavy chain CDR3 and demonstrating that the CDR3 domain alone conferred binding specificity); and Ditzel et al., J. Immunol. 157:

739-749 (1996) (describing grafting studies wherein transfer of only the heavy chain CDR3 of a parent polyspecific Fab LNA3 to a heavy chain of a monospecific IgG tetanus toxoid-binding Fab p313 antibody was sufficient to retain binding specificity of the parent Fab).

Accordingly, the present invention provides monoclonal antibodies comprising one or more heavy and/or light chain CDR3 domains from an antibody derived from a human or non-human animal, wherein the monoclonal antibody is capable of specifically binding to CD30. Within certain aspects, the present invention provides monoclonal antibodies comprising one or more heavy and/or light chain CDR3 domain from a non-human antibody, such as a mouse or rat antibody, wherein the monoclonal antibody is capable of specifically binding to CD30. Within some embodiments, such inventive antibodies comprising one or more heavy and/or light chain CDR3 domain from a non-human antibody (a) are capable of competing for binding with; (b) retain the functional characteristics; (c) bind to the same epitope; and/or (d) have a similar binding affinity as the corresponding parental non-human antibody.

Within other aspects, the present invention provides monoclonal antibodies comprising one or more heavy and/or light chain CDR3 domain from a human antibody, such as, for example, a human antibody obtained from a non-human animal, wherein the human antibody is capable of specifically binding to CD30. Within other aspects, the present invention provides monoclonal antibodies comprising one or more heavy and/or light chain CDR3 domain from a first human antibody, such as, for example, a human antibody obtained from a non-human animal, wherein the first human antibody is capable of specifically binding to CD30 and wherein the CDR3 domain from the first human antibody replaces a CDR3 domain in a human antibody that is lacking binding specificity for CD30 to generate a second human antibody that is capable of specifically binding to CD30. Within some embodiments, such inventive antibodies comprising one or more heavy and/or light chain CDR3 domain from the first human antibody (a) are capable of competing for binding with; (b) retain the functional characteristics; (c) bind to the same epitope; and/or (d) have a similar binding affinity as the corresponding parental first human antibody. In preferred embodiments, the first human antibody is 5F11, 17G1 or 2H9.

Anti-CD30 Antibodies Lacking Fucosyl and Xylosyl Residues and Having Enhanced ADCC Activity

The present invention also relates to a defucosylated and dexylosylated anti-CD30 antibody with enhanced antibody directed cellular cytotoxicity (ADCC) against cells expressing CD30 as compared to the fucosylated and xylosylated form of the antibody. In a preferred embodiment, a defucosylated and dexylosylated antibody of the invention induces ADCC of L1236 cells *in vitro* wherein the fucosylated and xylosylated form of the antibody does not induce ADCC, under conditions of an antibody concentration of 0.1 μg/ml and a target cell to effector cell ratio of 1:50. In another preferred embodiment, a defucosylated and dexylosylated antibody of the invention enhances ADCC of L540, L428 and Karpas cells *in vitro* compared to the fucosylated and xylosylated form of the antibody, under conditions of an antibody concentration of 0.1 μg/ml and a target cell to effector cell ratio of 1:50.

The increased ADCC activity of a defucosylated and dexylosylated antibody of the invention can be quantitated, for example, as an increase in percent cell lysis, as compared to the fucosylated and xylosylated form of the antibody, when ADCC activity is measured under the same conditions for the two forms (*e.g.*, same antibody concentrations and same target to effector cell ratios). Preferably, a defucosylated and dexylosylated anti-CD30 antibody of the invention increases the percent lysis of CD30+ cells as compared to the fucosylated form of the antibody at least 1.25 fold (*i.e.*, the ratio of the % lysis of the defucosylated and dexylosylated form to the fucosylated and xylosylated form is at least 1.25), more preferably at least 2 fold, even more preferably at least 2.5 fold and even more preferably at least 3 fold. In various embodiments, the defucosylated and dexylosylated form of the antibody increases percent lysis of CD30+ cells as compared to the fucosylated form of the antibody from 1.25 to 3.25 fold, preferably 1.5 to 3.25 fold, even more preferably 1.61 to 3.25 fold, even more preferably 2.15 to 3.25 fold, and even more preferably 2.63 to 3.25 fold, preferably under conditions where the antibody is at a concentration of 25 μg/ml and the target to effector cell ratio is 1:50.

Additionally or alternatively, the increased ADCC activity of a defucosylated and dexylosylated antibody of the invention can be quantitated, for example, as an increased potency as measured by a decrease in the $EC_{50}$ value for the defucosylated and dexylosylated form, as compared to the fucosylated and xylosylated form. This can be quantitated by the ratio of the $EC_{50}$ for the fucosylated and xylosylated form to the defucosylated and dexylosylated form. Preferably, the $EC_{50}$ ratio of the fucosylated and xylosylated form to the defucosylated and dexylosylated form for ADCC of CD30+ cells is at least 3 (*i.e.,* the $EC_{50}$ of the defucosylated and dexylosylated form is 3-fold lower than the $EC_{50}$ of the fucosylated and xylosylated form), more preferably, at least 4, even more preferably at least 5, at least 7, at least 10, at least 15 or at least 20. In various embodiments, the $EC_{50}$ ratio of the fucosylated and xylosylated form to the defucosylated and dexylosylated form for ADCC of CD30+ cells is from 2 to 27.1, more preferably from 4 to 27.1, even more preferably from 4.7 to 27.1, even more preferably from 7.8 to 27.1, and even more preferably from 11.1 to 27.1. Preferably, the EC50 values are determined in ADCC assays that use a target to effector cell ratio of 1:50 and antibody concentrations from 0.0001 μg/ml to 10 μg/ml or higher.

Examples of CD30+ cell lines that can be used in the ADCC assays of the invention and against which a defucosylated and dexylosylated antibody of the invention exhibits enhanced ADCC activity, as compared to the fucosylated and xylosylated form of the antibody, include L540 cells (human Hodgkin's lymphoma; DSMZ Deposit No. ACC 72), L428 cells (human Hodgkin's lymphoma; DSMZ Deposit No. ACC 197), L1236 cells (human Hodgkin's lymphoma; DSMZ Deposit No. ACC 530), and Karpas cells (human T cell lymphoma; DSMZ Deposit No. ACC 31). The enhanced ADCC effect by defucosylated and dexylosylated anti-CD30 antibodies may result in ADCC activity on CD30+ cells at antibody concentrations where ADCC would not be observed with the fucosylated and xylosylated form of the antibody. For example, in an *in vitro* ADCC assay with a target:effector cell ratio of 1:50, ADCC due to a defucosylated and dexylosylated anti-CD30 antibody is observed with the CD30+ cell line L1236 at concentrations as low as 0.005 μg/ml, whereas no ADCC activity is detected with the fucosylated and xylosylated anti-CD30 antibody at concentrations as high as 0.1 μg/ml.

<u>Defucosylation and Dexylosylation of Anti-CD30 Antibodies</u>

Anti-CD30 antibodies (*e.g.*, murine, chimeric, humanized and human antibodies) are known in the art, and may be used in the present invention. In one embodiment, the anti-CD30 antibody of the present invention is modified such that the antibody is lacking in fucosyl residues. An antibody can be made that is lacking in fucosyl residues by one of a variety of methods. For example, the antibody can be expressed, using recombinant DNA technology, in a cell with an altered glycosylation mechanism such that addition of fucosyl residues to carbohydrate chains is inhibited. Additionally or alternatively, an antibody can be defucosylated through chemical removal of the fucosyl residue.

In one embodiment, the antibody is expressed in a cell that is lacking in a fucosyltransferase enzyme such that the cell line produces proteins lacking fucosyl in their carbohydrates. For example, the cell lines Ms704, Ms705, and Ms709 lack the fucosyltransferase gene, FUT8 (alpha (1,6) fucosyltransferase), such that antibodies expressed in the Ms704, Ms705, and Ms709 cell lines lack fucosyl on their carbohydrates. The Ms704, Ms705, and Ms709 $FUT8^{-/-}$ cell lines were created by the targeted disruption of the FUT8 gene in CHO/DG44 cells using two replacement vectors (see U.S. Patent Publication No. 20040110704 by Yamane et al. and Yamane-Ohnuki et al. (2004) Biotechnol Bioeng 87: 614-22). As another example, EP 1,176,195 by Hanai *et al.* describes a cell line with a functionally disrupted FUT8 gene, which encodes a fucosyl transferase, such that antibodies expressed in such a cell line exhibit hypofucosylation by reducing or eliminating the alpha 1,6 bond-related enzyme. Hanai *et al.* also describe cell lines which naturally have a low enzyme activity for adding fucosyl to the N-acetylglucosamine that binds to the Fc region of the antibody or does not have the enzyme activity, for example the rat myeloma cell line YB2/0 (ATCC CRL 1662). PCT Publication WO 03/035835 by Presta describes a variant CHO cell line, Lec13 cells, with reduced ability to attach fucosyl to Asn(297)-linked carbohydrates, also resulting in hypofucosylation of antibodies expressed in that host cell (see also Shields, R.L. et al. (2002) J. Biol. Chem. 277:26733-26740). PCT Publication WO 99/54342 by Umana et al. describes cell lines engineered to express glycoprotein-modifing glycosyl transferases (*e.g.*, beta(1,4)-N-acetylglucosaminyltransferase III (GnTIII)) such that antibodies expressed in the engineered cell lines exhibit increased bisecting GlcNac structures which results in increased ADCC activity of the antibodies (see also Umana et al. (1999) Nat. Biotech. 17:176-180).

In another embodiment, an anti-CD30 antibody is expressed and the fucosyl residue(s) is cleaved using a fucosidase enzyme. For example, the fucosidase alpha-L-fucosidase removes fucosyl residues from antibodies (Tarentino, A.L. et al. (1975) Biochem. 14:5516-23).

Additionally, in other embodiments, other forms of glycosylation of an antibody are also modified. For example, an aglycoslated antibody can be made (*i.e.*, the antibody lacks glycosylation). Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. Such aglycosylation may increase the affinity of the antibody for antigen. Such an approach is described in further detail in U.S. Patent Nos. 5,714,350 and 6,350,861 by Co et al.

In another embodiment, the anti-CD30 antibody of the present invention is modified such that the antibody is lacking in fucosyl and xylosyl residues. An antibody can be made that is lacking in fucosyl and xylosyl residues by one of a variety of methods. For example, the antibody can be expressed, using recombinant DNA technology, in a plant cell with an altered glycosylation mechanism such that addition of fucosyl and xylosyl residues to carbohydrate chains is inhibited. Additionally or alternatively, an antibody can be defucosylated and dexylosylated through chemical removal of the fucosyl and xylosyl residue.

A number of plant species have been targeted for use in "molecular farming" of mammalian proteins of pharmaceutical interest. Higher plants, particularly higher plants that serve as expression systems for recombinant proteins for pharmaceutical use, that have been stably transformed to produce glycoproteins with an altered *N*-glycoslyation pattern using the methods described herein may be genetically modified to produce any recombinant protein of interest. Thus, in one aspect, the invention provides methods for producing monoclonal antibodies in higher plants, wherein the monoclonal antibodies have an *N*-glycosylation pattern that reflects a reduction in the amount of β1,2-linked xylosyl residues and α1,3-linked fucosyl residues within the *N*-linked glycans, and compositions comprising recombinant monoclonal antibodies produced using plant hosts genetically modified in the manner set forth herein. Methods for production of antibodies in a plant system are known. In some embodiments, the plant host of interest is a member of the duckweed family. In some embodiments, the plant serving as the host for recombinant production of the monoclonal antibody is a member of the *Lemnaceae* family, for example, a *Lemna* plant. *Lemna* is a small, aquatic, higher plant that has been developed for the production of recombinant therapeutic proteins. The *Lemna* Expression System (LEX System$^{SM}$) enables rapid, clonal expansion of transgenic plants, secretion of transgenic proteins, high protein yields, full containment, and has the additional advantage of low operating and capital costs. Numerous proteins including mAbs have been successfully produced in *Lemna* with expression levels routinely in the range of 6-8% of the total soluble protein (TSP). These expression levels, in combination with *Lemna's* high protein content and fast growth rate (36 hr doubling time), enable production of >1g of mAb per kg biomass in a robust and well-controlled format.

Plants produce glycoproteins with complex *N*-glycans having a core bearing two N-acetylglucosamine (GlcNAc) residues that is similar to that observed in mammals. However, in plant glycoproteins this core is substituted by a β1,2-linked xylosyl residue (core xylosyl), which residue does not occur in humans, Lewis$^a$ epitopes, and an α1,3-linked fucosyl (core α[1,3]-fucosyl) instead of an α1,6-linked core fucosyl as in mammals (see, for example, Lerouge et al. (1998) Plant Mol. Biol. 38:31-48 for a review). Both the α(1,3)-fucosyl and β(1,2)-xylosyl residues reportedly are, at least partly, responsible for the immunogenicity of plant glycoproteins in mammals (see, for example, Ree et al. (2000) J Biol. Chem. 15:11451-11458; Bardor et al. (2003) Glycobiol. 13:427-434; Garcia-Casado et al. (1996) Glycobiol. 6:471-477). Therefore removal of these potentially allergenic sugar residues from mammalian glycoproteins recombinantly produced in plants would overcome concerns about the use of these proteins as pharmaceuticals for treatment of humans.

In addition, plants do not naturally contain a β1,4-galactosyltransferase (GalT) enzyme, which is responsible for transfer of Gal from UDP-Gal to GlcNAc residues in N-linked glycans. Mammalian GalT cDNA has been successfully expressed in plant cells, resulting in partially galactosylated N-glycans similar to antibodies produced by hybridoma cells (Bakker et al. (2001) Proc Nat Acad Sci, USA 98:2899-2904). Thus, in one embodiment of the invention, antibodies may be produced which lack Gal in the N-glycan structure. In another embodiment, antibodies may be produced in plant cells which contain Gal in the N-glycan struture.

Accordingly, the present invention provides methods for producing a recombinant monoclonal antibody, including a monoclonal antibody having improved effector function, wherein the antibody is recombinantly produced within a plant having an altered *N*-glycosylation pattern of endogenous and heterologous gylcoproteins produced therein such that these glycoproteins exhibit a reduction in the amount of the plant-specific β1,2-linked xylosyl residues and/or α1,3-linked fucosyl residues attached to the *N*-glycans thereof. Where the antibodies have reduced amounts α1,3-linked fucosyl residues attached to the *N*-glycans thereof, the antibodies may have increased ADCC activity relative to antibodies, produced in a control plant that has not been genetically modified to inhibit expression or function of FucT.

The transgenic higher plants of the invention are capable of producing a glycoprotein product that has a substantially homogenous glycosylation profile for the G0 glycan species, and which is characterized by its substantial homogeneity for the G0 glycoform. The G0 glycan species is also referred to as the "GnGn" or $GlcNAc_2Man_3GlcNAc_2$ *N*-glycan species. This advantageously results in plant host expression systems that have increased production consistency, as well as reduced chemical, manufacturing, and control (CMC) risk associated with the production of these glycoprotein compositions. In a preferred embodiment, the produced glycoprotein product contains at least 70% having a homogeneous glycoform. In another preferred embodiment, the produced glycoprotein product contains at least 80% having a homogeneous glycoform. In yet another preferred embodiment, the produced glycoprotein product contains at least 90%, 95%, 96%, 97%, 98%, 99% or 100% having a homogeneous glycoform. In one embodiment, the glycoprotein compositions of the invention comprise *N*-linked glycans that are predominately of the G0 glycan structure. The G0 glycoform of the antibody compositions of the present invention advantageously provides an antibody composition that has increased ADCC activity in association with the absence of fucosyl residues. Furthermore, the G0 glycoform lacks the terminal Gal residues present in antibodies having the G2 glycoform. As such, these substantially homogeneous antibody compositions of the invention having predominately the G0 glycoform have increased ADCC/CDC ratios.

Methods for altering the *N*-glycosylation pattern of proteins in higher plants include stably transforming the plant with at least one recombinant nucleotide construct that provides for the inhibition of expression of α1,3-fucosyltransferase (FucT) and β1,2-xylosyltransferase (XylT) in a plant. Methods for production of antibodies in a plant system are known. Inhibition of the expression of FucT or XylT, or both, may be obtained by a number of methods well known in the art, including, but not limited to, antisense suppression, double-stranded RNA (dsRNA) interference, hairpin RNA (hpRNA) interference or intron-containing hairpin RNA (ihpRNA) interference. For dsRNA interference, a sense RNA molecule like that described above for cosuppression and an antisense RNA molecule that is fully or partially complementary to the sense RNA molecule are expressed in the same cell, resulting in inhibition of the expression of the corresponding endogenous messenger RNA. For hpRNA interference, the expression cassette is designed to express an RNA molecule that hybridizes with itself to form a hairpin structure that comprises a single-stranded loop region and a base-paired stem. The base-paired stem region comprises a sense sequence corresponding to all or part of the endogenous messenger RNA encoding the gene whose expression is to be inhibited, and an antisense sequence that is fully or partially

complementary to the sense sequence. Thus, the base-paired stem region of the molecule generally determines the specificity of the RNA interference. hpRNA molecules are highly efficient at inhibiting the expression of endogenous genes, and the RNA interference they induce is inherited by subsequent generations of plants. See, for example, Chuang and Meyerowitz (2000) Proc. Natl. Acad Sci. USA 97:4985-4990; Stoutjesdijk et al. (2002) Plant Physiol. 129:1723-1731; and Waterhouse and Helliwell (2003) Nat. Rev. Genet. 4:29-38. Alternatively, the transgenic plants of the invention having FucT and XylT expression silenced in the manner set forth herein can be further modified in their glycosylation machinery such that they express a galactosyltransferase and efficiently attach the terminal galactose residue to the *N*-glycans of endogenous and heterologous glycoproteins produced therein.

Transformation protocols as well as protocols for introducing nucleotide sequences into plants may vary depending on the type of plant or plant cell or nodule, that is, monocot or dicot, targeted for transformation. Suitable methods of introducing nucleotide sequences into plants or plant cells or nodules include microinjection (Crossway et al. (1986) Biotechniques 4:320-334), electroporation (Riggs et al. (1986) Proc. Natl. Acad Sci. USA 83:5602-5606), *Agrobacterium*-mediated transformation (U.S. Patent Nos. 5,563,055 and 5,981,840, direct gene transfer (Paszkowski et al. (1984) EMBO J. 3:2717-2722), ballistic particle acceleration (see, *e.g.*, U.S. Patent Nos. 4,945,050; 5,879,918; 5,886,244; and 5,932,782; and Tomes et al. (1995) "Direct DNA Transfer into Intact Plant Cells via Microprojectile Bombardment," in Plant Cell, Tissue, and Organ Culture: Fundamental Methods, ed. Gamborg and Phillips (Springer-Verlag, Berlin); McCabe et al. (1988) Biotechnology 6:923-926). The cells that have been transformed may be grown into plants in accordance with conventional ways. See, for example, McCormick et al. (1986) Plant Cell Reports 5:81-84.

Characterization of Absence of Fucosyl or Xylosyl Residues on Anti-CD30 Antibodies

Antibodies of the invention lack fucosyl and xylosyl residues, for example in the Fc portion carbohydrate chain. Antibodies can be tested for the absence of fucosyl or xylosyl residues using standard techniques known in the art, such as APTS capillary electrophoresis laser induced fluorescence. Briefly, the N-linked oligosaccharides of the purified anti-CD30 antibody can be released by adding the peptide N-glycanase (Prozyme) and incubating overnight. The carbohydrates are resuspended and derivatized with 8-aminopyrene-1,3,6-trisulfonate (APTS) under mild reductive amination conditions in which desialylation and loss of fucosyl or xylosyl residues is minimized. The reaction adducts are analyzed by capillary electrophoresis with a laser-induced fluorescence detector (Beckman Coulter). An absence of fucosyl or xylosyl can be observed by a shift in the electrophoresis compared to the same antibody containing fucosyl or xylosyl. Another technique for testing the absence of fucosyl or xylosyl on anti-CD30 antibodies is a monosaccharide analysis using HPLC. Suitable assays to determine CD30 binding are further described in the Examples.

Characterization of Antibody Dependent Cell Killing of CD30+ Cells

Anti-CD30 antibodies can be tested for their ability to mediate phagocytosis and killing of cells expressing CD30. In one embodiment, a defucosylated and dexylosylated anti-CD30 antibody enhances killing of cells expressing CD30 in comparison to the same antibody containing fucosyl and xylosyl when compared at the same concentration. In another embodiment, a defucosylated and dexylosylated anti-CD30 antibody induces killing of cells expressing CD30 where the same antibody containing fucosyl and xylosyl does not induce cell killing at the same concentration.

The ADCC activity of a monoclonal antibody can be tested in established *in vitro* assays. As an example, a chromium release ADCC assay may be used. Briefly, peripheral blood mononuclear cells (PBMCs), or other effector cells, from healthy donors can be purified by Ficoll Hypaque density centrifugation, followed by lysis of contaminating erythrocytes. Washed PBMCs can be suspended in RPMI supplemented with 10% heat-inactivated fetal calf serum and mixed with $^{51}$Cr labeled cells expressing CD30, at various ratios of effector cells to tumor cells (effector cells:tumor cells). Anti-CD30 antibody can then be added at various concentrations. An isotype matched antibody can be used as a negative control. Assays can be carried out for 4-18 hours at 37° C. Samples can be assayed for cytolysis by measuring $^{51}$Cr release into the culture supernatant. Anti-CD30 monoclonal can also be tested in combinations with each other to determine whether cytolysis is enhanced with multiple monoclonal antibodies.

Fc-receptor (FcR) mediated effector cell function has been shown to be important for the *in vivo* activity of many therapeutic mAbs. The FcR expressed on NK cells and macrophages responsible for ADCC activity is Fc$\gamma$RIIIa. Macrophage mediated phagocytosis has been shown to be an important mechanism in the depletion ofB cells in vivo (Tedder et al. (2006) Springer Semin Immunol 28:351-64). The removal of fucose residues from various mAbs produced in other expression systems has been shown previously to increase FcR binding and enhance ADCC function. Thus, in one embodiment, the antibodies of the present invention have an increased binding affinity for Fc$\gamma$RIIIa.

An alternative assay that can be used to test for anti-CD30 antibody ability to mediate phagocytosis and killing of cells expressing CD30 is a time resolved fluorometry assay. Briefly, CD30 expressing cells are loaded with an acetoxymethyl ester of fluorescence enhancing ligand (BATDA), which penetrates cell membranes. Inside the cell, the ester bonds are hydrolized and the compound can no longer pass the cell membrane. Anti-CD30 antibody can then be added

at various concentrations. Following cytolysis, an europeum solution (Perkin Elmer) is added and any free ligand binds the europeum to form a highly fluorescent and stable chelate (EuTDA) that can be read on a microplate reader (Perkin Elmer). The measured signal correlates with the amount of lysed cells.

Anti-CD30 antibodies also can be tested in an *in vivo* model (*e.g.*, in mice) to determine their efficacy in mediating phagocytosis and killing of cells expressing CD30, *e.g.*, tumor cells. These antibodies can be selected, for example, based on the following criteria, which are not intended to be exclusive:

1) binding to live cells expressing CD30;
2) high affinity of binding to CD30;
3) binding to a unique epitope on CD30 (to eliminate the possibility that monoclonal antibodies with complimentary activities when used in combination would compete for binding to the same epitope);
4) opsonization of cells expressing CD30;
5) mediation *in vitro* of growth inhibition, phagocytosis and/or killing of cells expressing CD30 in the presence of human effector cells.

Preferred monoclonal antibodies of the invention meet one or more of these criteria. In a particular embodiment, the monoclonal antibodies are used in combination, *e.g.*, as a pharmaceutical composition comprising two or more anti-CD30 monoclonal antibodies or fragments thereof. For example, anti-CD30 monoclonal antibodies having different, but complementary activities can be combined in a single therapy to achieve a desired therapeutic or diagnostic effect. An illustration of this would be a composition containing an anti-CD30 monoclonal antibody that mediates highly effective killing of target cells in the presence of effector cells, combined with another anti-CD30 monoclonal antibody that inhibits the growth of cells expressing CD30.

Characterization of Binding to CD30

Antibodies of the invention can be tested for binding to CD30 by, for example, standard assays known in the art, such as ELISA, FACS analysis and/or Biacore analysis. In a typical ELISA assay, briefly, microtiter plates are coated with purified CD30 at 0.25 μg/ml in PBS, and then blocked with 5% bovine serum albumin in PBS. Dilutions of antibody are added to each well and incubated for 1-2 hours at 37°C. The plates are washed with PBS/Tween and then incubated with secondary reagent (*e.g.*, for human antibodies or a goat-anti-human IgG Fc-specific polyclonal reagent) conjugated to alkaline phosphatase for 1 hour at 37°C. After washing, the plates are developed with pNPP substrate (1 mg/ml), and analyzed at OD of 405-650.

In order to demonstrate binding of monoclonal antibodies to live cells expressing the CD30, flow cytometry can be used. In a typical (but non-limiting) example of a flow cytometry protocol, cell lines expressing CD30 (grown under standard growth conditions) are mixed with various concentrations of monoclonal antibodies in PBS containing 0.1% BSA and 20% mouse serum, and incubated at 37°C for 1 hour. After washing, the cells are reacted with Fluorescein-labeled secondary antibody (*e.g.*, anti-human IgG antibody) under the same conditions as the primary antibody staining. The samples can be analyzed by a FACScan instrument using light and side scatter properties to gate on single cells. An alternative assay using fluorescence microscopy may be used (in addition to or instead of) the flow cytometry assay. Cells can be stained exactly as described above and examined by fluorescence microscopy. This method allows visualization of individual cells, but may have diminished sensitivity depending on the density of the antigen.

Anti-CD30 antibodies can be further tested for reactivity with CD30 antigen by Western blotting. For example, cell extracts from cells expressing CD30 can be prepared and subjected to sodium dodecyl sulfate (SDS) polyacrylamide gel electrophoresis. After electrophoresis, the separated antigens are transferred to nitrocellulose membranes, blocked with 20% mouse serum, and probed with the monoclonal antibodies to be tested. Antibody binding can be detected using anti-species specific secondary antibody linked to alkaline phosphatase and developed with BCIP/NBT substrate tablets (Sigma Chem. Co., St. Louis, MO). Other techniques for evaluating the binding ability of antibodies towards CD30 are known in the art, including RIAs and Biacore analysis. Suitable assays to determine CD30 binding are described in detail in the Examples.

Antibody Physical Properties

The antibodies of the present invention may be further characterized by the various physical properties of the anti-CD30 antibodies. Various assays may be used to detect and/or differentiate different classes of antibodies based on these physical properties.

In some embodiments, antibodies of the present invention may contain one or more glycosylation sites in either the light or heavy chain variable region. The presence of one or more glycosylation sites in the variable region may result in increased immunogenicity of the antibody or an alteration of the pK of the antibody due to altered antigen binding

(Marshall et al (1972) Annu Rev Biochem 41:673-702; Gala FA and Morrison SL (2004) J Immunol 172:5489-94; Wallick et al (1988) J Exp Med 168:1099-109; Spiro RG (2002) Glycobiology 12:43R-56R; Parekh et al (1985) Nature 316:452-7; Mimura et al. (2000) Mol Immunol 37:697-706). Glycosylation has been known to occur at motifs containing an N-X-S/T sequence. Variable region glycosylation may be tested using a Glycoblot assay, which cleaves the antibody to produce a Fab, and then tests for glycosylation using an assay that measures periodate oxidation and Schiff base formation. Alternatively, variable region glycosylation may be tested using Dionex light chromatography (Dionex-LC), which cleaves saccharides from a Fab into monosaccharides and analyzes the individual saccharide content. In some instances, it is preferred to have an anti-CD30 antibody that does not contain variable region glycosylation. This can be achieved either by selecting antibodies that do not contain the glycosylation motif in the variable region or by mutating residues within the glycosylation motif using standard techniques well known in the art.

In a preferred embodiment, the antibodies of the present invention do not contain asparagine isomerism sites. A deamidation or isoaspartic acid effect may occur on N-G or D-G sequences, respectively. The deamidation or isoaspartic acid effect results in the creation of isoaspartic acid which decreases the stability of an antibody by creating a kinked structure off a side chain carboxy terminus rather than the main chain. The creation of isoaspartic acid can be measured using an iso-quant assay, which uses a reverse-phase HPLC to test for isoaspartic acid.

Each antibody will have a unique isoelectric point (pI), but generally antibodies will fall in the pH range of between 6 and 9.5. The pI for an IgG1 antibody typically falls within the pH range of 7-9.5 and the pI for an IgG4 antibody typically falls within the pH range of 6-8. Antibodies may have a pI that is outside this range. Although the effects are generally unknown, there is speculation that antibodies with a pI outside the normal range may have some unfolding and instability under *in vivo* conditions. The isoelectric point may be tested using a capillary isoelectric focusing assay, which creates a pH gradient and may utilize laser focusing for increased accuracy (Janini et al (2002) Electrophoresis 23:1605-11; Ma et al. (2001) Chromatographia 53:S75-89; Hunt et al (1998) J Chromatogr A 800:355-67). In some instances, it is preferred to have an anti-$CD_{30}$ antibody that contains a pI value that falls in the normal range. This can be achieved either by selecting antibodies with a pI in the normal range, or by mutating charged surface residues using standard techniques well known in the art.

Each antibody will have a melting temperature that is indicative of thermal stability (Krishnamurthy R and Manning MC (2002) Curr Pharm Biotechnol 3:361-71). A higher thermal stability indicates greater overall antibody stability *in vivo.* The melting point of an antibody may be measure using techniques such as differential scanning calorimetry (Chen et al (2003) Pharm Res 20:1952-60; Ghirlando et al (1999) Immunol Lett 68:47-52). $T_{M1}$ indicates the temperature of the initial unfolding of the antibody. $T_{M2}$ indicates the temperature of complete unfolding of the antibody. Generally, it is preferred that the $T_{M1}$ of an antibody of the present invention is greater than 60°C, preferably greater than 65°C, even more preferably greater than 70°C. Alternatively, the thermal stability of an antibody may be measure using circular dichroism (Murray et al. (2002) J. Chromatogr Sci 40:343-9).

In a preferred embodiment, antibodies are selected that do not rapidly degrade. Fragmentation of an anti-CD30 antibody may be measured using capillary electrophoresis (CE) and MALDI-MS, as is well understood in the art (Alexander AJ and Hughes DE (1995) Anal Chem 67:3626-32)..

In another preferred embodiment, antibodies are selected that have minimal aggregation effects. Aggregation may lead to triggering of an unwanted immune response and/or altered or unfavorable pharmacokinetic properties. Generally, antibodies are acceptable with aggregation of 25% or less, preferably 20% or less, even more preferably 15% or less, even more preferably 10% or less and even more preferably 5% or less. Aggregation may be measured by several techniques well known in the art, including size-exclusion column (SEC) high performance liquid chromatography (HPLC), and light scattering to identify monomers, dimers, trimers or multimers.

<u>Chimeric or Humanized Anti-CD30 Antibodies</u>

In certain embodiments, an anti-CD30 antibody of the invention is a chimeric or humanized antibody. Such antibodies can be prepared using mouse anti-CD30 antibodies that are available in the art and established procedures for converting a mouse antibody to a chimeric or humanized antibody. Non-limiting examples of such mouse anti-CD30 antibodies include the AC10, HeFi-1, Ber-H2, Ki-1, Ki-4, HRS-3, Irac, HRS-4, M44, M67 and Ber-H8 monoclonal antibodies. Moreover, humanized anti-CD30 antibodies are described in PCT Publication WO 02/4661.

<u>Antibodies Having Particular Germline Sequences</u>

In certain embodiment, an antibody of the invention comprises a heavy chain variable region from a particular germline heavy chain immunoglobulin gene and/or a light chain variable region from a particular germline light chain immunoglobulin gene.

For example, in a preferred embodiment, the invention provides a defucosylated monoclonal antibody, or an antigen-binding portion thereof, comprising a heavy chain variable region that is the product of or derived from a human

$V_H$ 4-34 gene, wherein the antibody specifically binds to human CD30. In another preferred embodiment, the invention provides a defucosylated monoclonal antibody, or an antigen-binding portion thereof, comprising a heavy chain variable region that is the product of or derived from a human $V_H$ 3-07 gene, wherein the antibody specifically binds CD30. In another preferred embodiment, the invention provides a defucosylated monoclonal antibody, or an antigen-binding portion thereof, comprising a light chain variable region that is the product of or derived from a human $V_K$ L15 gene, wherein the antibody specifically binds to human CD30. In another preferred embodiment, the invention provides a defucosylated monoclonal antibody, or an antigen-binding portion thereof, comprising a light chain variable region that is the product of or derived from a human $V_K$ A27 gene, wherein the antibody specifically binds to human CD30. In another preferred embodiment, the invention provides a defucosylated monoclonal antibody, or an antigen-binding portion thereof, comprising a light chain variable region that is the product of or derived from a human $V_K$ L6 gene, wherein the antibody specifically binds to human CD30.

In yet another preferred embodiment, the invention provides a defucosylated monoclonal antibody, or an antigen-binding portion thereof, wherein the antibody:

(a) comprises a heavy chain variable region that is the product of or derived from a human $V_H$ 4-34 or 3-07 gene (which encodes the amino acid sequence set forth in SEQ ID NOs: 25 and 26, respectively);
(b) comprises a light chain variable region that is the product of or derived from a human $V_k$ L15, A27, or L6 gene (which encode the amino acid sequences set forth in SEQ ID NOs: 27, 28, and 29, respectively); and
(c) specifically binds to human CD30.

A preferred $V_H$ and $V_k$ germline combination is $V_H$ 4-34 and $V_k$ L15. An example of an antibody having $V_H$ and $V_K$ of $V_H$ 4-34 and $V_k$ L15, respectively, is the 5F11 antibody. Another preferred $V_H$ and $V_k$ germline combination is $V_H$ 3-07 and $V_k$ A27. An example of an antibody having $V_H$ and $V_k$ of $V_H$ 3-07 and Vk A27, respectively, is the 17G1 antibody. Another preferred $V_H$ and $V_k$ germline combination is $V_H$ 4-34 and $V_k$ L6. An example of an antibody having $V_H$ and $V_k$ of $V_H$ 4-34 and $V_k$ L6, respectively, is the 2H9 antibody.

As used herein, a human antibody comprises heavy or light chain variable regions that is "the product of" or "derived from" a particular germline sequence if the variable regions of the antibody are obtained from a system that uses human germine immunoglobulin genes. Such systems include immunizing a transgenic mouse carrying human immunoglobulin genes with the antigen of interest or screening a human immunoglobulin gene library displayed on, phage with the antigen of interest. A human antibody that is "the product of" or "derived from" a human germline immunoglobulin sequence can be identified as such by comparing the amino acid sequence of the human antibody to the amino acid sequences of human germline immunoglobulins (eg., using the Vbase database) and selecting the human germline immunoglobulin sequence that is closest in sequence (i.e., greatest % identity) to the sequence of the human antibody. A human antibody that is "the product of" or "derived from" a particular human germline immunoglobulin sequence may contain amino acid differences as compared to the germline sequence, due to, for example, naturally-occurring somatic mutations or intentional introduction of site-directed mutation. However, a selected human antibody typically is at least 90% identical in amino acids sequence to an amino acid sequence encoded by a human germline immunoglobulin gene and contains amino acid residues that identify the human antibody as being human when compared to the germline immunoglobulin amino acid sequences of other species (e.g., murine germline sequences). In certain cases, a human antibody may be at least 95%, or even at least 96%, 97%, 98%, or 99% identical in amino acid sequence to the amino acid sequence encoded by the germline immunoglobulin gene. Typically, a human antibody derived from a particular human germline sequence will display no more than 10 amino acid differences from the amino acid sequence encoded by the human germline immunoglobulin gene. In certain cases, the human antibody may display no more than 5, or even no more than 4, 3, 2, or 1 amino acid difference from the amino acid sequence encoded by the germline immunoglobulin gene.

Homologous Antibodies

In yet another embodiment, a defucosylated antibody of the invention comprises heavy and light chain variable regions comprising amino acid sequences that are homologous to the amino acid sequences of the preferred antibodies described herein, and wherein the antibodies retain the desired functional properties of the anti-CD30 antibodies of the invention.

For example, the invention provides a defucosylated monoclonal antibody, or antigen binding portion thereof, comprising a heavy chain variable region and a light chain variable region, wherein:

(a) the heavy chain variable region comprises an amino acid sequence that is at least 80% homologous to an amino acid sequence selected from the group consisting of SEQ ID NOs: 1, 2, and 3;
(b) the light chain variable region comprises an amino acid sequence that is at least 80% homologous to an amino

acid sequence selected from the group consisting of SEQ ID NOs: 4, 5, and 6; and
(c) the antibody specifically binds to human CD30.

In other embodiments, the $V_H$ and/or $V_L$ amino acid sequences may be 85%, 90%, 95%, 96%, 97%, 98% or 99% homologous to the sequences set forth above. An antibody having $V_H$ and $V_L$ regions having high (i.e., 80% or greater) homology to the $V_H$ and $V_L$ regions of the sequences set forth above, can be obtained by mutagenesis (e.g., site-directed or PCR-mediated mutagenesis) of one or more nucleic acid molecules encoding SEQ ID NOs: 1-6, followed by testing of the encoded altered antibody for retained function (i.e., binding to CD30) using the binding assays described herein. Nucleic acid molecules encoding SEQ ID NOs: 1-6 are shown in SEQ ID NOs: 30-35.
As used herein, the percent homology between two amino acid sequences is equivalent to the percent identity between the two sequences. The percent identity between the two sequences is a function of the number of identical positions shared by the sequences *(i.e.,* % homology = # of identical positions/total # of positions x 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.
The percent identity between two nucleotide sequences can be determined using the GAP program in the GCG software package (available at www.gcg.com), using a NWSgapdna.CMP matrix and a gap weight of 40, 50, 60, 70, or 80 and a length weight of 1, 2, 3, 4, 5, or 6. The percent identity between two nucleotide or amino acid sequences can also determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. 48:444-453 (1970)) algorithm which has been incorporated into the GAP program in the GCG software package (available at www.gcg.com), using either a Blossum 62 matrix or a PAM250 matrix, and a gap weight of 16, 14, 12, 10, 8, 6, or 4 and a length weight of 1, 2, 3, 4, 5, or 6.
The percent identity between two amino acid sequences can be determined using the algorithm ofE. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. 48: 444-453 (1970)) algorithm which has been incorporated into the GAP program in the GCG software package (available at www.gcg.com), using either a Blossum 62 matrix or a PAM250 matrix, and a gap weight of 16,14,12,10,8,6, or 4 and a length weight of 1, 2, 3, 4, 5, or 6.
Additionally or alternatively, the protein sequences of the present invention can further be used as a "query sequence" to perform a search against public databases to, for example, identify related sequences. Such searches can be performed using the XBLAST program (version 2.0) of Altschul, et al. (1990) J. Mol. Biol. 215:403-10. BLAST protein searches can be performed with the XBLAST program, score = 50, wordlength = 3 to obtain amino acid sequences homologous to the antibody molecules of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (e.g., XBLAST and NBLAST) can be used. See www.ncbi.nlm.nih.gov.

Antibodies with Conservative Modifications

In certain embodiments, a defucosylated antibody of the invention comprises a heavy chain variable region comprising CDR1, CDR2 and CDR3 sequences and a light chain variable region comprising CDR1, CDR2 and CDR3 sequences, wherein one or more of these CDR sequences comprise specified amino acid sequences based on the preferred antibodies described herein (*e.g.*, 5F11, 17G1, and 2H9), or conservative modifications thereof, and wherein the antibodies retain the desired functional properties of the anti-CD30 antibodies of the invention. Accordingly, the invention provides a defucosylated monoclonal antibody, or antigen binding portion thereof, comprising a heavy chain variable region comprising CDR1, CDR2, and CDR3 sequences and a light chain variable region comprising CDR1, CDR2, and CDR3 sequences, wherein:

(a) the heavy chain variable region CDR3 sequence comprises the amino acid sequence of SEQ ID NO: 9, 12, or 15, and conservative modifications thereof;
(b) the light chain variable region CDR3 sequence comprises the amino acid sequence of SEQ ID NO: 18, 21, or 24, and conservative modifications thereof; and
(c) the antibody specifically binds to human CD30.

In a preferred embodiment, the heavy chain variable region CDR2 sequence comprises the amino acid sequence

of SEQ ID NO: 8, 11, or 14, and conservative modifications thereof; and the light chain variable region CDR2 sequence comprises the amino acid sequence of SEQ ID NO: 17, 20, or 23, and conservative modifications thereof. In another preferred embodiment, the heavy chain variable region CDR1 sequence comprises the amino acid sequence of SEQ ID NO: 7, 10, or 13, and conservative modifications thereof; and the light chain variable region CDR1 sequence comprises the amino acid sequence of SEQ ID NO: 16, 19, or 22, and conservative modifications thereof.

As used herein, the term "conservative sequence modifications" is intended to refer to amino acid modifications that do not significantly affect or alter the binding characteristics of the antibody containing the amino acid sequence. Such conservative modifications include amino acid substitutions, additions and deletions. Modifications can be introduced into an antibody of the invention by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (*e.g.*, lysine, arginine, histidine), acidic side chains (*e.g.*, aspartic acid, glutamic acid), uncharged polar side chains (*e.g.*, glycine, asparagine, glutamine, serine, threonine, tyrosine, cysteine, tryptophan), nonpolar side chains (*e.g.*, alanine, valine, leucine, isoleucine, proline, phenylalanine, methionine), beta-branched side chains (*e.g.*, threonine, valine, isoleucine) and aromatic side chains (*e.g.*, tyrosine, phenylalanine, tryptophan, histidine). Thus, one or more amino acid residues within the CDR regions of an antibody of the invention can be replaced with other amino acid residues from the same side chain family and the altered antibody can be tested for retained function (*i.e.*, the functions set forth in (c) above) using the functional assays described herein.

Alternatively, in another embodiment, mutations can be introduced randomly along all or part of an anti-CD30 antibody coding sequence, such as by saturation mutagenesis, and the resulting modified anti-CD30 antibodies can be screened for binding activity.

Antibodies that Bind to the Same Epitope as Anti-CD30 Antibodies of the Invention

In another embodiment, the invention provides defucosylated antibodies that bind to the same epitope as do the various anti-CD30 antibodies of the invention provided herein, such as other human antibodies that bind to the same epitope as the 5F11, 17G1 or 2H9 antibodies described herein. Such additional antibodies can be identified based on their ability to cross-compete (*e.g.*, to competitively inhibit the binding of, in a statistically significant manner) with other antibodies of the invention, such as 5F11, 17G1 or 2H9, in standard CD30 binding assays. The ability of a test antibody to inhibit the binding of, e.g., 5F11, 17G1 or 2H9 to human CD30 demonstrates that the test antibody can compete with that antibody for binding to human CD30; such an antibody may, according to non-limiting theory, bind to the same or a related (*e.g.*, a structurally similar or spatially proximal) epitope on human CD30 as the antibody with which it competes. In a preferred embodiment, the defucosylated antibody that binds to the same epitope on human CD30 as 5F11, 17G1 or 2H9 is a human monoclonal antibody. Such human monoclonal antibodies can be prepared and isolated as described in PCT Publication WO 03/059282.

Engineered and Modified Antibodies

A defucosylated antibody of the invention further can be prepared using an antibody having one or more of the $V_H$ and/or $V_L$ sequences disclosed herein as starting material to engineer a modified antibody, which modified antibody may have altered properties from the starting antibody. An antibody can be engineered by modifying one or more amino acid residues within one or both variable regions (*i.e.*, $V_H$ and/or $V_L$), for example within one or more CDR regions and/or within one or more framework regions. Additionally or alternatively, an antibody can be engineered by modifying residues within the constant region(s), for example to alter the effector function(s) of the antibody.

One type of variable region engineering that can be performed is CDR grafting. Antibodies interact with target antigens predominantly through amino acid residues that are located in the six heavy and light chain complementarity determining regions (CDRs). For this reason, the amino acid sequences within CDRs are more diverse between individual antibodies than sequences outside of CDRs. Because CDR sequences are responsible for most antibody-antigen interactions, it is possible to express recombinant antibodies that mimic the properties of specific naturally occurring antibodies by constructing expression vectors that include CDR sequences from the specific naturally occurring antibody grafted onto framework sequences from a different antibody with different properties (see, *e.g.*, Riechmann, L. et al. (1998) Nature 332:323-327; Jones, P. et al. (1986) Nature 321:522-525; Queen, C. et al. (1989) Proc. Natl. Acad. See. U.S.A. 86:10029-10033; U.S. Patent No. 5,225,539 to Winter, and U.S. Patent Nos. 5,530,101; 5,585,089; 5,693,762 and 6,180,370 to Queen et al.).

Accordingly, another embodiment of the invention pertains to a defucosylated monoclonal antibody, or antigen binding portion thereof, comprising a heavy chain variable region comprising CDR1, CDR2, and CDR3 sequences comprising an amino acid sequence selected from the group consisting of SEQ ID NOs: 7, 8, and 9, SEQ ID NOs: 10,

11, and 12, and SEQ ID NOs: 13, 14, and 15, respectively, and a light chain variable region comprising an amino acid sequence selected from the group consisting of SEQ ID NOs: 16, 17, and 18, SEQ ID NOs: 19, 20, and 21 and SEQ ID NOs: 22, 23, and 24, respectively. Thus, such antibodies contain the $V_H$ and $V_L$ CDR sequences of monoclonal antibodies 5F11, 17G1, or 2H9 yet may contain different framework sequences from these antibodies.

Such framework sequences can be obtained from public DNA databases or published references that include germline antibody gene sequences. For example, germline DNA sequences for human heavy and light chain variable region genes can be found in the "VBase" human germline sequence database (available on the Internet at www.mrc-cpe.cam.ac.uk/vbase), as well as in Kabat, E. A., et al. (1991) Sequences of Proteins of Immunological Interest, Fifth Edition, U.S. Department of Health and Human Services, NIH Publication No. 91-3242; Tomlinson, I. M., et al. (1992) "The Repertoire of Human Germline VH Sequences Reveals about Fifty Groups of VH Segments with Different Hypervariable Loops" J. Mol. Biol. 227:776-798; and Cox, J. P. L. et al. (1994) "A Directory of Human Germ-line VH Segments Reveals a Strong Bias in their Usage" Eur. J. Immunol. 24:827-836. As another example, the germline DNA sequences for human heavy and light chain variable region genes can be found in the Genbank database. For example, the following heavy chain germline sequences found in the HCo7 HuMAb mouse are available in the accompanying Genbank accession numbers: 1-69 (NG_0010109, NT_024637 and BC070333), 3-33 (NG_0010109 and NT_024637) and 3-7 (NG_0010109 and NT_024637). As another example, the following heavy chain germline sequences found in the HCo12 HuMAb mouse are available in the accompanying Genbank accession numbers: 1-69 (NG_0010109, NT_024637 and BC070333), 5-51 (NG_0010109 and NT_024637), 4-34 (NG_0010109 and NT_024637), 3-30.3 (CAJ556644) and 3-23 (AJ406678).

Antibody protein sequences are compared against a compiled protein sequence database using one of the sequence similarity searching methods called the Gapped BLAST (Altschul et al. (1997) Nucleic Acids Research 25: 3389-3402), which is well known to those skilled in the art. BLAST is a heuristic algorithm in that a statistically significant alignment between the antibody sequence and the database sequence is likely to contain high-scoring segment pairs (HSP) of aligned words. Segment pairs whose scores cannot be improved by extension or trimming is called a *hit*. Briefly, the nucleotide sequences of VBASE origin (*vbase.mrc-cpe.cam.ac.uk/vbase1/list2.php*) are translated and the region between and including FR1 through FR3 framework region is retained. The database sequences have an average length of 98 residues. Duplicate sequences which are exact matches over the entire length of the protein are removed. A BLAST search for proteins using the program blastp with default, standard parameters except the low complexity filter, which is turned off, and the substitution matrix of BLOSUM62, filters for top 5 hits yielding sequence matches. The nucleotide sequences are translated in all six frames and the frame with no stop codons in the matching segment of the database sequence is considered the potential hit. This is in turn confirmed using the BLAST program tblastx, which translates the antibody sequence in all six frames and compares those translations to the VBASE nucleotide sequences dynamically translated in all six frames.

The identities are exact amino acid matches between the antibody sequence and the protein database over the entire length of the sequence. The positives (identities + substitution match) are not identical but amino acid substitutions guided by the BLOSUM62 substitution matrix. If the antibody sequence matches two of the database sequences with same identity, the hit with most positives would be decided to be the matching sequence hit.

Preferred framework sequences for use in the antibodies of the invention are those that are structurally similar to the framework sequences used by selected antibodies of the invention, *e.g.*, similar to the $V_H$ 4-34 or 3-07 sequences (SEQ ID NO: 25 or 26) and/or the $V_k$ L15, A27 or L6 framework sequence (SEQ ID NO: 27, 28, or 29) used by preferred monoclonal antibodies of the invention. The $V_H$ CDR1, 2 and 3 sequences, and the $V_\kappa$ CDR1, 2 and 3 sequences, can be grafted onto framework regions that have the identical sequence as that found in the germline immunoglobulin gene from which the framework sequence derive, or the CDR sequences can be grafted onto framework regions that contain one or more mutations as compared to the germline sequences. For example, it has been found that in certain instances it is beneficial to mutate residues within the framework regions to maintain or enhance the antigen binding ability of the antibody (see *e.g.*, U.S. Patent Nos. 5,530,101; 5,585,089; 5,693,762 and 6,180,370 to Queen et al).

Another type of variable region modification is to mutate amino acid residues within the $V_H$ and/or $V_\kappa$ CDR1, CDR2 and/or CDR3 regions to thereby improve one or more binding properties (*e.g.*, affinity) of the antibody of interest. Site-directed mutagenesis or PCR-mediated mutagenesis can be performed to introduce the mutation(s) and the effect on antibody binding, or other functional property of interest, can be evaluated in *in vitro* or *in vivo* assays as described herein and provided in the Examples. Preferably conservative modifications (as discussed above) are introduced. The mutations may be amino acid substitutions, additions or deletions, but are preferably substitutions. Moreover, typically no more than one, two, three, four or five residues within a CDR region are altered.

Accordingly, in another embodiment, the invention provides defucosylated anti-CD30 monoclonal antibodies, or antigen binding portions thereof, comprising a heavy chain variable region comprising: (a) a $V_H$ CDR1 region comprising an amino acid sequence selected from the group consisting of SEQ ID NO: 7, 8, and 9, or an amino acid sequence having one, two, three, four or five amino acid substitutions, deletions or additions as compared to an amino acid sequence selected from the group consisting of SEQ ID NO: 7, 8, and 9; (b) a $V_H$ CDR2 region comprising an amino

acid sequence selected from the group consisting of SEQ ID NO: 10, 11, and 12, or an amino acid sequence having one, two, three, four or five amino acid substitutions, deletions or additions as compared to an amino acid sequence selected from the group consisting of SEQ ID NO: 10, 11, and 12; (c) a $V_H$ CDR3 region comprising an amino acid sequence selected from the group consisting of SEQ ID NO: 13, 14, and 15, or an amino acid sequence having one, two, three, four or five amino acid substitutions, deletions or additions as compared to an amino acid sequence selected from the group consisting of SEQ ID NO: 13, 14, and 15; (d) a $V_\kappa$ CDR1 region comprising an amino acid sequence selected from the group consisting of SEQ ID NO: 16, 17 and 18, or an amino acid sequence having one, two, three, four or five amino acid substitutions, deletions or additions as compared to an amino acid sequence selected from the group consisting of SEQ ID NO: 16, 17, and 18; (e) a $V_\kappa$ CDR2 region comprising an amino acid sequence selected from the group consisting of SEQ ID NO: 19, 20, and 21, or an amino acid sequence having one, two, three, four or five amino acid substitutions, deletions or additions as compared to an amino acid sequence selected from the group consisting of SEQ ID NO: 19, 20, and 21; and (f) a $V_\kappa$ CDR3 region comprising an amino acid sequence selected from the group consisting of SEQ ID NO: 22, 23, and 24, or an amino acid sequence having one, two, three, four or five amino acid substitutions, deletions or additions as compared to an amino acid sequence selected from the group consisting of SEQ ID NO: 22, 23, and 24.

Engineered antibodies of the invention include those in which modifications have been made to framework residues within $V_H$ and/or $V_\kappa$, *e.g.* to improve the properties of the antibody. Typically such framework modifications are made to decrease the immunogenicity of the antibody. For example, one approach is to "backmutate" one or more framework residues to the corresponding germline sequence. More specifically, an antibody that has undergone somatic mutation may contain framework residues that differ from the germline sequence from which the antibody is derived. Such residues can be identified by comparing the antibody framework sequences to the germline sequences from which the antibody is derived. For example, for 5F11, amino acid residue #83 (within FR3) of $V_H$ is an asparagine whereas this residue in the corresponding $V_H$ 4-34 germline sequence is a serine. To return the framework region sequences to their germline configuration, the somatic mutations can be "backmutated" to the germline sequence by, for example, site-directed mutagenesis or PCR-mediated mutagenesis (*e.g.*, residue 83 of FR3 of the $V_H$ of 5F11 can be "backmutated" from asparagine to serine. Such "backmutated" antibodies are also intended to be encompassed by the invention.

Another type of framework modification involves mutating one or more residues within the framework region, or even within one or more CDR regions, to remove T cell epitopes to thereby reduce the potential immunogenicity of the antibody. This approach is also referred to as "deimmunization" and is described in further detail in U.S. Patent Publication No. 20030153043 by Carr et al.

In addition or alternative to modifications made within the framework or CDR regions, antibodies of the invention may be engineered to include modifications within the Fc region, typically to alter one or more functional properties of the antibody, such as serum half-life, complement fixation, Fc receptor binding, and/or antigen-dependent cellular cytotoxicity. Furthermore, an antibody of the invention may be chemically modified (*e.g.*, one or more chemical moieties can be attached to the antibody) or be modified to alter its glycosylation, again to alter one or more functional properties of the antibody. Each of these embodiments is described in further detail below. The numbering of residues in the Fc region is that of the EU index of Kabat.

In one embodiment, the hinge region of CH1 is modified such that the number of cysteine residues in the hinge region is altered, *e.g.*, increased or decreased. This approach is described further in U.S. Patent No. 5,677,425 by Bodmer et al. The number of cysteine residues in the hinge region of CH1 is altered to, for example, facilitate assembly of the light and heavy chains or to increase or decrease the stability of the antibody.

In another embodiment, the Fc hinge region of an antibody is mutated to decrease the biological half life of the antibody. More specifically, one or more amino acid mutations are introduced into the CH2-CH3 domain interface region of the Fc-hinge fragment such that the antibody has impaired Staphylococcyl protein A (SpA) binding relative to native Fc-hinge domain SpA binding. This approach is described in further detail in U.S. Patent No. 6,165,745 by Ward et al.

In another embodiment, the antibody is modified to increase its biological half life. Various approaches are possible. For example, one or more of the following mutations can be introduced: T252L, T254S, T256F, as described in U.S. Patent No. 6,277,375 to Ward. Alternatively, to increase the biological half life, the antibody can be altered within the CH1 or CL region to contain a salvage receptor binding epitope taken from two loops of a CH2 domain of an Fc region of an IgG, as described in U.S. Patent Nos. 5,869,046 and 6,121,022 by Presta et al.

In yet other embodiments, the Fc region is altered by replacing at least one amino acid residue with a different amino acid residue to alter the effector function(s) of the antibody. For example, one or more amino acids selected from amino acid residues 234, 235, 236, 237, 297, 318, 320 and 322 can be replaced with a different amino acid residue such that the antibody has an altered affinity for an effector ligand but retains the antigen-binding ability of the parent antibody. The effector ligand to which affinity is altered can be, for example, an Fc receptor or the C1 component of complement. This approach is described in further detail in U.S. Patent Nos. 5,624,821 and 5,648,260, both by Winter et al..

In another example, one or more amino acids selected from amino acid residues 329, 331 and 322 can be

replaced with a different amino acid residue such that the antibody has altered C1q binding and/or reduced or abolished complement dependent cytotoxicity (CDC). This approach is described in further detail in U.S. Patent Nos. 6,194,551 by Idusogie et al.

In another example, one or more amino acid residues within amino acid positions 231 and 239 are altered to thereby alter the ability of the antibody to fix complement. This approach is described further in PCT Publication WO 94/29351 by Bodmer et al.

In yet another example, the Fc region is modified to increase the ability of the antibody to mediate antibody dependent cellular cytotoxicity (ADCC) and/or to increase the affinity of the antibody for an Fcγ receptor by modifying one or more amino acids at the following positions: 238,239, 248, 249, 252, 254, 255, 256, 258, 265, 267, 268, 269, 270, 272, 276, 278, 280, 283, 285, 286, 289, 290, 292, 293, 294, 295, 296, 298, 301, 303, 305, 307, 309, 312, 315, 320, 322, 324, 326, 327, 329, 330, 331, 333, 334, 335, 337, 338, 340, 360, 373, 376, 378, 382, 388, 389, 398, 414, 416, 419, 430, 434, 435, 437, 438 or 439. This approach is described further in PCT Publication WO 00/42072 by Presta. Moreover, the binding sites on human IgG1 for FcγR1, FcγRII, FcγRIII and FcRn have been mapped and variants with improved binding have been described (see Shields, R.L. et al. (2001) J. Biol. Chem. 276:6591-6604). Specific mutations at positions 256, 290, 298, 333, 334 and 339 were shown to improve binding to FcγRIII. Additionally, the following combination mutants were shown to improve FcγRIII binding: T256A/S298A, S298A/E333A, S298A/K224A and S298A/E333A/K334A.

Another modification of the antibodies herein that is contemplated by the invention is pegylation. An antibody can be pegylated to, for example, increase the biological (*e.g.*, serum) half life of the antibody. To pegylate an antibody, the antibody, or fragment thereof, typically is reacted with polyethylene glycol (PEG), such as a reactive ester or aldehyde derivative of PEG, under conditions in which one or more PEG groups become attached to the antibody or antibody fragment. Preferably, the pegylation is carried out via an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono (C1-C10) alkoxy- or aryloxy-polyethylene glycol or polyethylene glycol-maleimide. In certain embodiments, the antibody to be pegylated is an aglycosylated antibody. Methods for pegylating proteins are known in the art and can be applied to the antibodies of the invention. See for example, EP 0 154 316 by Nishimura et al. and EP 0 401 384 by Ishikawa et al.

<u>Methods of Engineering Antibodies</u>

As discussed above, the defucosylated anti-CD30 antibodies having $V_H$ and $V_\kappa$ sequences disclosed herein can be used to create new anti-CD30 antibodies by modifying the VH and/or $V_\kappa$ sequences, or the constant region(s) attached thereto. Thus, in another aspect of the invention, the structural features of an anti-CD30 antibody of the invention, e.g. 5F11, 17G1, or 2H9, are used to create structurally related defucosylated anti-CD30 antibodies that retain at least one functional property of the antibodies of the invention, such as binding to human CD30. For example, one or more CDR regions of 5F11, 17G1, or 2H9, or mutations thereof, can be combined recombinantly with known framework regions and/or other CDRs to create additional, recombinantly-engineered, anti-CD30 antibodies of the invention, as discussed above. Other types of modifications include those described in the previous section. The starting material for the engineering method is one or more of the $V_H$ and/or $V_\kappa$ sequences provided herein, or one or more CDR regions thereof. To create the engineered antibody, it is not necessary to actually prepare (*i.e.,* express as a protein) an antibody having one or more of the $V_H$ and/or $V_\kappa$ sequences provided herein, or one or more CDR regions thereof. Rather, the information contained in the sequence(s) is used as the starting material to create a "second generation" sequence(s) derived from the original sequence(s) and then the "second generation" sequence(s) is prepared and expressed as a protein.

Accordingly, in another embodiment, the invention provides a method for preparing an anti-CD30 antibody comprising:

(a) providing: (i) a heavy chain variable region antibody sequence comprising a CDR1 sequence selected from the group consisting of SEQ ID NOs: 7, 8, and 9, a CDR2 sequence selected from the group consisting of SEQ ID NOs: 10, 11, and 12 and/or a CDR3 sequence selected from the group consisting of SEQ ID NOs: 13, 14, and 15; and/or (ii) a light chain variable region antibody sequence comprising a CDR1 sequence selected from the group consisting of SEQ ID NOs: 16, 17, and 18, a CDR2 sequence selected from the group consisting of SEQ ID NOs: 19, 20, and 21 and/or a CDR3 sequence selected from the group consisting of SEQ ID NOs: 22, 23, and 24;
(b) altering at least one amino acid residue within the heavy chain variable region antibody sequence and/or the light chain variable region antibody sequence to create at least one altered antibody sequence; and
(c) expressing the altered antibody sequence as a protein.

Standard molecular biology techniques can be used to prepare and express the altered antibody sequence. The altered antibody sequence so prepared can then be made in defucosylated form using the methods disclosed herein

to obtain a defucosylated altered anti-CD30 antibody.

The functional properties of the altered antibodies can be assessed using standard assays available in the art and/or described herein, such as those set forth in the Examples (*e.g.*, flow cytometry, binding assays, ADCC assays).

In certain embodiments of the methods of engineering antibodies of the invention, mutations can be introduced randomly or selectively along all or part of an anti-CD30 antibody coding sequence and the resulting modified anti-CD30 antibodies can be screened for binding activity and/or other functional properties as described herein. Mutational methods have been described in the art. For example, PCT Publication WO 02/092780 by Short describes methods for creating and screening antibody mutations using saturation mutagenesis, synthetic ligation assembly, or a combination thereof. Alternatively, PCT Publication WO 03/074679 by Lazar et al. describes methods of using computational screening methods to optimize physiochemical properties of antibodies.

<u>Nucleic Acid Molecules Encoding Antibodies of the Invention</u>

Another aspect of the invention pertains to nucleic acid molecules that encode the antibodies of the invention. The term "nucleic acid molecule", as used herein, is intended to include DNA molecules and RNA molecules. A nucleic acid molecule may be single-stranded or double-stranded, but preferably is double-stranded DNA. The nucleic acids may be present in whole cells, in a cell lysate, or in a partially purified or substantially pure form. A nucleic acid is "isolated" or "rendered substantially pure" when purified away from other cellular components or other contaminants, e.g., other cellular nucleic acids or proteins, by standard techniques, including alkaline/SDS treatment, CsCl banding, column chromatography, agarose gel electrophoresis and others well known in the art. *See,* F. Ausubel, et al., ed. (1987) Current Protocols in Molecular Biology, Greene Publishing and Wiley Interscience, New York. A nucleic acid of the invention can be, for example, DNA or RNA and may or may not contain intronic sequences. In a preferred embodiment, the nucleic acid is a cDNA molecule.

Nucleic acids of the invention can be obtained using standard molecular biology techniques. For antibodies expressed by hybridomas (*e.g.*, hybridomas prepared from transgenic mice carrying human immunoglobulin genes as described further below), cDNAs encoding the light and heavy chains of the antibody made by the hybridoma can be obtained by standard PCR amplification or cDNA cloning techniques. For antibodies obtained from an immunoglobulin gene library (*e.g.*, using phage display techniques), nucleic acid encoding the antibody can be recovered from the library.

Preferred nucleic acids molecules of the invention are those encoding the VH and VL sequences of the 5F11, 17G1, and 2H9 monoclonal antibodies. The DNA sequence encoding the VH sequence of 5F11 is shown in SEQ ID NO: 30. The DNA sequence encoding the VL sequence of 5F11 is shown in SEQ ID NO: 33. The DNA sequence encoding the VH sequence of 17G1 is shown in SEQ ID NO: 31. The DNA sequence encoding the VL sequence of 17G1 is shown in SEQ ID NO: 34. The DNA sequence encoding the VH sequence of 2H9 is shown in SEQ ID NO: 32. The DNA sequence encoding the VL sequence of 2H9 is shown in SEQ ID NO: 35.

Once DNA fragments encoding VH and VL segments are obtained, these DNA fragments can be further manipulated by standard recombinant DNA techniques, for example to convert the variable region genes to full-length antibody chain genes, to Fab fragment genes or to a scFv gene. In these manipulations, a VL- or VH-encoding DNA fragment is operatively linked to another DNA fragment encoding another protein, such as an antibody constant region or a flexible linker. The term "operatively linked", as used in this context, is intended to mean that the two DNA fragments are joined such that the amino acid sequences encoded by the two DNA fragments remain in-frame.

The isolated DNA encoding the VH region can be converted to a full-length heavy chain gene by operatively linking the VH-encoding DNA to another DNA molecule encoding heavy chain constant regions (CH1, CH2 and CH3). The sequences of human heavy chain constant region genes are known in the art (see *e.g.*, Kabat, E. A., el al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The heavy chain constant region can be an IgG1, IgG2, IgG3, IgG4, IgA, IgE, IgM or IgD constant region, but most preferably is an IgG1 or IgG4 constant region. For a Fab fragment heavy chain gene, the VH-encoding DNA can be operatively linked to another DNA molecule encoding only the heavy chain CH1 constant region.

The isolated DNA encoding the VL region can be converted to a full-length light chain gene (as well as a Fab light chain gene) by operatively linking the VL-encoding DNA to another DNA molecule encoding the light chain constant region, CL. The sequences of human light chain constant region genes are known in the art (see *e.g.*, Kabat, E. A., et al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The light chain constant region can be a kappa or lambda constant region, but most preferably is a kappa constant region.

To create a scFv gene, the VH- and VL-encoding DNA fragments are operatively linked to another fragment encoding a flexible linker, *e.g.*, encoding the amino acid sequence ($Gly_4$ -Ser)$_3$, such that the VH and VL sequences can be expressed as a contiguous single-chain protein, with the VL and VH regions joined by the flexible linker (see

*e.g.*, Bird et al. (1988) Science 242:423-426; Huston et al. (1988) Proc. Natl. Acad. Sci. USA 85:5879-5883; McCafferty et al., (1990) Nature 348:552-554).

The nucleic acid compositions of the present invention, while often in a native sequence (except for modified restriction sites and the like), from either cDNA, genomic or mixtures may be mutated, thereof in accordance with standard techniques to provide gene sequences. For coding sequences, these mutations, may affect amino acid sequence as desired. In particular, DNA sequences substantially homologous to or derived from native V, D, J, constant, switches and other such sequences described herein are contemplated (where "derived" indicates that a sequence is identical or modified from another sequence).

Production of Monoclonal Antibodies of the Invention

Monoclonal antibodies (mAbs) of the present invention can be produced by a variety of techniques, including conventional monoclonal antibody methodology *e.g.*, the standard somatic cell hybridization technique of Kohler and Milstein (1975) Nature 256: 495. Although somatic cell hybridization procedures are preferred, in principle, other techniques for producing monoclonal antibody can be employed *e.g.*, viral or oncogenic transformation of B lymphocytes.

The preferred animal system for preparing hybridomas is the murine system. Hybridoma production in the mouse is a very well-established procedure. Immunization protocols and techniques for isolation of immunized splenocytes for fusion are known in the art. Fusion partners (*e.g.*, murine myeloma cells) and fusion procedures are also known.

In various embodiments, the antibody can be, for example, human antibodies, humanized antibodies or chimeric antibodies.

Chimeric or humanized antibodies of the present invention can be prepared based on the sequence of a murine monoclonal antibody prepared as described above. DNA encoding the heavy and light chain immunoglobulins can be obtained from the murine hybridoma of interest and engineered to contain non-murine (*e.g.,.* human) immunoglobulin sequences using standard molecular biology techniques. For example, to create a chimeric antibody, the murine variable regions can be linked to human constant regions using methods known in the art (see *e.g.,* U.S. Patent No. 4,816,567 to Cabilly et al.). To create a humanized antibody, the murine CDR regions can be inserted into a human framework using methods known in the art (see *e.g.*, U.S. Patent No. 5,225,539 to Winter, and U.S. Patent Nos: 5,530,101; 5,585,089; 5,693,762 and 6,180,370 to Queen et al.). A variety of mouse anti-CD30 antibodies are known in the art that can be used to create chimeric or humanized anti-CD30 antibodies, for example, AC 10, HeFi-1, Ber-H2, Ki-1, HRS-3, Irac, HRS-4, M44, M67, and Ber-H8.

In a preferred embodiment, the antibodies of the invention are human monoclonal antibodies. Such human monoclonal antibodies directed against CD30 can be generated using transgenic or transchromosomic mice carrying parts of the human immune system rather than the mouse system. These transgenic and transchromosomic mice include mice referred to herein as HuMAb mice and KM mice, respectively, and are collectively referred to herein as "human Ig mice."

The HuMAb mouse® (Medarex, Inc.) contains human immunoglobulin gene miniloci that encode unrearranged human heavy ($\mu$ and $\gamma$) and $\kappa$ light chain immunoglobulin sequences, together with targeted mutations that inactivate the endogenous $\mu$ and $\kappa$ chain loci (see e.g., Lonberg, et al. (1994) Nature 368(6474): 856-859). Accordingly, the mice exhibit reduced expression of mouse IgM or $\kappa$, and in response to immunization, the introduced human heavy and light chain transgenes undergo class switching and somatic mutation to generate high affinity human IgG$\kappa$ monoclonal (Lonberg, N. *et al.* (1994), *supra;* reviewed in Lonberg, N. (1994) Handbook of Experimental Pharmacology 113:49-101; Lonberg, N. and Huszar, D. (1995) Intern. Rev. Immunol. 13: 65-93, and Harding, F. and Lonberg, N. (1995) Ann. N.Y. Acad. Sci. 764:536-546). The preparation and use of HuMab mice, and the genomic modifications carried by such mice, is further described in Taylor, L. et al. (1992) Nucleic Acids Research 20:6287-6295; Chen, J. et al. (1993) International Immunology 5: 647-656; Tuaillon et al. (1993) Proc. Natl. Acad. Sci. USA 90:3720-3724; Choi et al. (1993) Nature Genetics 4:117-123; Chen, J. et al. (1993) EMBO J. 12: 821-830; Tuaillon et al. (1994) J. Immunol. 152:2912-2920; Taylor, L. et al. (1994) International Immunology 6: 579-591; and Fishwild, D. et al. (1996) Nature Biotechnology 14: 845-851. See further, U.S. Patent Nos. 5,545,806; 5,569,825; 5,625,126; 5,633,425; 5,789,650; 5,877,397; 5,661,016; 5,814,318; 5,874,299; and 5,770,429; all to Lonberg and Kay; U.S. Patent No. 5,545,807 to Surani et al.*;* PCT Publication Nos. WO 92/03918, WO 93/12227, WO 94/25585, WO 97/13852, WO 98/24884 and WO 99/45962, all to Lonberg and Kay; and PCT Publication No. WO 01/14424 to Korman et al.

In another embodiment, human antibodies of the invention can be raised using a mouse that carries human immunoglobulin sequences on transgenes and transchomosomes, such as a mouse that carries a human heavy chain transgene and a human light chain transchromosome. Such mice, referred to herein as "KM mice", are described in detail in PCT Publication WO 02/43478 to Ishida et al.

Still further, alternative transgenic animal systems expressing human immunoglobulin genes are available in the art and can be used to raise anti-CD30 antibodies of the invention. For example, an alternative transgenic system referred to as the Xenomouse (Abgenix, Inc.) can be used; such mice are described in, for example, U.S. Patent Nos.

5,939,598; 6,075,181; 6,114,598; 6, 150,584 and 6,162,963 to Kucherlapati et al.

Moreover, alternative transchromosomic animal systems expressing human immunoglobulin genes are available in the art and can be used to raise anti-CD30 antibodies of the invention. For example, mice carrying both a human heavy chain transchromosome and a human light chain tranchromosome, referred to as "TC mice" can be used; such mice are described in Tomizuka et al. (2000) Proc. Natl. Acad. Sci. USA 97:722-727. Furthermore, cows carrying human heavy and light chain transchromosomes have been described in the art (Kuroiwa et al. (2002) Nature Biotechnology 20:889-894) and can be used to raise anti-CD30 antibodies of the invention.

Human monoclonal antibodies of the invention can also be prepared using phage display methods for screening libraries of human immunoglobulin genes. Such phage display methods for isolating human antibodies are established in the art. See for example: U.S. Patent Nos. 5,223,409; 5,403,484; and 5,571,698 to Ladner et al.*;* U.S. Patent Nos. 5,427,908 and 5,580,717 to Dower et al.*;* U.S. Patent Nos. 5,969,108 and 6,172,197 to McCafferty et al.*;* and U.S. Patent Nos. 5,885,793; 6,521,404; 6,544,731; 6,555,313; 6,582,915 and 6,593,081 to Griffiths et al.

Human monoclonal antibodies of the invention can also be prepared using SCID mice into which human immune cells have been reconstituted such that a human antibody response can be generated upon immunization. Such mice are described in, for example, U.S. Patent Nos. 5,476,996 and 5,698,767 to Wilson et al. Immunization of Human Ig_Mice

When human Ig mice are used to raise human antibodies of the invention, such mice can be immunized with a purified or enriched preparation of CD30 antigen and/or recombinant CD30, or an CD30 fusion protein, as described by Lonberg, N. et al. (1994) Nature 368(6474): 856-859; Fishwild, D. et al. (1996) Nature Biotechnology 14:845-851; and PCT Publication WO 98/24884 and WO 01/14424. Preferably, the mice will be 6-16 weeks of age upon the first infusion. For example, a purified or recombinant preparation (5-50 $\mu$g) of CD30 antigen can be used to immunize the human Ig mice intraperitoneally.

Detailed procedures to generate fully human monoclonal antibodies to CD30 are described in PCT Publication WO 03/059282. Cumulative experience with various antigens has shown that the transgenic mice respond when initially immunized intraperitoneally (IP) with antigen in complete Freund's adjuvant, followed by every other week IP immunizations (up to a total of 6) with antigen in incomplete Freund's adjuvant. However, adjuvants other than Freund's are also found to be effective. In addition, whole cells in the absence of adjuvant are found to be highly immunogenic. The immune response can be monitored over the course of the immunization protocol with plasma samples being obtained by retroorbital bleeds. The plasma can be screened by ELISA (as described below), and mice with sufficient titers of anti-CD30 human immunoglobulin can be used for fusions. Mice can be boosted intravenously with antigen 3 days before sacrifice and removal of the spleen. It is expected that 2-3 fusions for each immunization may need to be performed. Between 6 and 24 mice are typically immunized for each antigen. Usually both HCo7 and HCo 12 strains are used. In addition, both HCo7 and HCo12 transgene can be bred together into a single mouse having two different human heavy chain transgenes (HCo7/HCo12).

Generation of Hybridomas Producing Human Monoclonal Antibodies of the Invention

To generate hybridomas producing human monoclonal antibodies of the invention, splenocytes and/or lymph node cells from immunized mice can be isolated and fused to an appropriate immortalized cell line, such as a mouse myeloma cell line. The resulting hybridomas can be screened for the production of antigen-specific antibodies. For example, single cell suspensions of splenic lymphocytes from immunized mice can be fused to one-sixth the number of P3X63-Ag8.653 nonsecreting mouse myeloma cells (ATCC, CRL 1580) with 50% PEG. Alternatively, the single cell suspension of splenic lymphocytes from immunized mice can be fused using an electric field based electrofusion method, using a CytoPulse large chamber cell fusion electroporator (CytoPulse Sciences, Inc., Glen Burnie Maryland). Cells are plated at approximately 2 x $10^5$ in flat bottom microtiter plate, followed by a two week incubation in selective medium containing 20% fetal Clone Serum, 18% "653" conditioned media, 5% origen (IGEN), 4 mM L-glutamine, 1 mM sodium pyruvate, 5mM HEPES, 0.055 mM 2-mercaptoethanol, 50 units/ml penicillin, 50 mg/ml streptomycin, 50 mg/ml gentamycin and 1X HAT (Sigma; the HAT is added 24 hours after the fusion). After approximately two weeks, cells can be cultured in medium in which the HAT is replaced with HT. Individual wells can then be screened by ELISA for human monoclonal IgM and IgG antibodies. Once extensive hybridoma growth occurs, medium can be observed usually after 10-14 days. The antibody secreting hybridomas can be replated, screened again, and if still positive for human IgG, the monoclonal antibodies can be subcloned at least twice by limiting dilution. The stable subclones can then be cultured *in vitro* to generate small amounts of antibody in tissue culture medium for characterization.

To purify human monoclonal antibodies, selected hybridomas can be grown in two-liter spinner-flasks for monoclonal antibody purification. Supernatants can be filtered and concentrated before affinity chromatography with protein A-sepharose (Pharmacia, Piscataway, N.J.). Eluted IgG can be checked by gel electrophoresis and high performance liquid chromatography to ensure purity. The buffer solution can be exchanged into PBS, and the concentration can be determined by OD280 using 1.43 extinction coefficient. The monoclonal antibodies can be aliquoted and stored at -80° C.

Generation of Transfectomas Producing Monoclonal Antibodies of the Invention

Antibodies of the invention also can be produced in a host cell transfectoma using, for example, a combination of well known recombinant DNA techniques and gene transfection methods (*e.g.*, Morrison, S. (1985) Science 229:1202).

For example, to express the antibodies, or antibody fragments thereof, DNAs encoding partial or full-length light and heavy chains, can be obtained by standard molecular biology techniques (*e.g.*, PCR amplification or cDNA cloning using a hybridoma that expresses the antibody of interest) and the DNAs can be inserted into expression vectors such that the genes are operatively linked to transcriptional and translational control sequences. In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vector or, more typically, both genes are inserted into the same expression vector. The antibody genes are inserted into the expression vector by standard methods (*e.g.*, ligation of complementary restriction sites on the antibody gene fragment and vector, or blunt end ligation if no restriction sites are present). The light and heavy chain variable regions of the antibodies described herein can be used to create full-length antibody genes of any antibody isotype by inserting them into expression vectors already encoding heavy chain constant and light chain constant regions of the desired isotype such that the $V_H$ segment is operatively linked to the $C_H$ segment(s) within the vector and the $V_K$ segment is operatively linked to the $C_L$ segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (*i.e.*, a signal peptide from a non-immunoglobulin protein).

In addition to the antibody chain genes, the recombinant expression vectors of the invention carry regulatory sequences that control the expression of the antibody chain genes in a host cell. The term "regulatory sequence" is intended to include promoters, enhancers and other expression control elements (*e.g.*, polyadenylation signals) that control the transcription or translation of the antibody chain genes. Such regulatory sequences are described, for example, in Goeddel (Gene Expression Technology. Methods in Enzymology 185, Academic Press, San Diego, CA (1990)). It will be appreciated by those skilled in the art that the design of the expression vector, including the selection of regulatory sequences, may depend on such factors as the choice of the host cell to be transformed, the level of expression of protein desired, *etc.* Preferred regulatory sequences for mammalian host cell expression include viral elements that direct high levels of protein expression in mammalian cells, such as promoters and/or enhancers derived from cytomegalovirus (CMV), Simian Virus 40 (SV40), adenovirus, (*e.g.*, the adenovirus major late promoter (AdMLP) and polyoma. Alternatively, nonviral regulatory sequences may be used, such as the ubiquitin promoter or $\beta$-globin promoter. Still further, regulatory elements composed of sequences from different sources, such as the SR$\alpha$ promoter system, which contains sequences from the SV40 early promoter and the long terminal repeat of human T cell leukemia virus type 1 (Takebe, Y. et al. (1988) Mol. Cell. Biol. 8:466-472).

In addition to the antibody chain genes and regulatory sequences, the recombinant expression vectors of the invention may carry additional sequences, such as sequences that regulate replication of the vector in host cells (*e.g.*, origins of replication) and selectable marker genes. The selectable marker gene facilitates selection of host cells into which the vector has been introduced (see, *e.g.*, U.S. Pat. Nos. 4,399,216, 4,634,665 and 5,179,017, all by Axel *et al.*). For example, typically the selectable marker gene confers resistance to drugs, such as G418, hygromycin or methotrexate, on a host cell into which the vector has been introduced. Preferred selectable marker genes include the dihydrofolate reductase (DHFR) gene (for use in dhfr- host cells with methotrexate selection/amplification) and the neo gene (for G418 selection).

For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, *e.g.*, electroporation, calcium-phosphate precipitation, DEAE-dextran transfection and the like. Although it is theoretically possible to express the antibodies of the invention in either prokaryotic or eukaryotic host cells, expression of antibodies in eukaryotic cells, and most preferably mammalian host cells, is the most preferred because such eukaryotic cells, and in particular mammalian cells, are more likely than prokaryotic cells to assemble and secrete a properly folded and immunologically active antibody. Prokaryotic expression of antibody genes has been reported to be ineffective for production of high yields of active antibody (Boss, M. A. and Wood, C. R. (1985) Immunology Today 6:12-13).

Preferred host cells for expressing the recombinant antibodies of the invention include cells which modify the fucosylation of an expressed antibody. For example, the host cell may be a cell that is lacking in a fucosyltransferase enzyme such that the host cell produces proteins lacking fucosyl in their carbohydrates, or a host cell that expresses glycoprotein-modifying glycosyl transferases such that expressed antibodies in the host cell have increased bisecting

GlcNac structures that prevents fucosylation. Other mammalian host cells for expressing the recombinant antibodies include Chinese Hamster Ovary (CHO cells) (including dhfr- CHO cells, described in Urlaub and Chasm, (1980) Proc. Natl. Acad. Sci. USA 77:4216-4220. used with a DHFR selectable marker, *e.g.*, as described in R. J. Kaufman and P. A. Sharp (1982) Mol. Biol. 159:601-621), NSO myeloma cells, COS cells and SP2 cells. In particular, for use with NS0 myeloma cells, another preferred expression system is the GS gene expression system disclosed in WO 87/04462, WO 89/01036 and EP 338,841. When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can be recovered from the culture medium using standard protein purification methods.

Other preferred hosts for expressing the recombinant antibodies of the invention include cells which modify the fucosylation and xylosylation of an expressed antibody. For example, the host cell may be a plant cell that is lacking in a fucosyltransferase and xylosyltransferase enzyme such that the host cell produces proteins lacking fucosyl and xylosyl in their carbohydrates. Methods for altering the *N*-glycosylation pattern of proteins in higher plants include stably transforming the plant with at least one recombinant nucleotide construct that provides for the inhibition of expression of $\alpha$1,3-fucosyltransferase (FucT) and $\beta$1,2-xylosyltransferase (XylT) in a plant. Methods for production of antibodies in a plant system are known.

Pharmaceutical Compositions

In another aspect, the present invention provides a composition, *e.g.*, a pharmaceutical composition, containing one or a combination of monoclonal antibodies of the present invention, formulated together with a pharmaceutically acceptable carrier. Such compositions may include one or a combination of (*e.g.*, two or more different) antibodies of the invention. For example, a pharmaceutical composition of the invention can comprise a combination of antibodies tat bind to different epitopes on the target antigen or that have complementary activities.

Pharmaceutical compositions of the invention also can be administered in combination therapy, *i.e.*, combined with other agents. For example, the combination therapy can include a defucosylated anti-CD30 antibody of the present invention combined with at least one other anti-neoplastic, anti-inflammatory or immunosuppressive agent. Such therapeutic agents include, among others, steroidal and nonsteroidal anti-inflammatory drugs (NSAIDS), *e.g.*, aspirin and other salicylates, such as ibuprofen (Motrin, Advil), naproxen (Naprosyn), sulindac (Clinoril), diclofenac (Voltaren), piroxicam (Feldene), ketoprofen (Orudis), diflunisal (Dolobid), nabumetone (Relafen), etodolac (Lodine), oxaprozin (Daypro), indomethacin (Indocin), and aspirin in high doses. Other examples of therapeutic agents that can be used in combination therapy are described in greater detail below in the section on uses of the antibodies of the invention.

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (*e.g.*, by injection or infusion). Depending on the route of administration, the active compound, *i.e.*, antibody or immunoconjuage, may be coated in a material to protect the compound from the action of acids and other natural conditions that may inactivate the compound.

The pharmaceutical compounds of the invention may include one or more pharmaceutically acceptable salts. A "pharmaceutically acceptable salt" refers to a salt that retains the desired biological activity of the parent compound and does not impart any undesired toxicological effects (see *e.g.*, Berge, S.M., *et al.* (1977) *J. Pharm. Sci.* 66:1-19). Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

A pharmaceutical composition of the invention also may include a pharmaceutically acceptable anti-oxidant. Examples ofpharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Examples of suitable aqueous and nonaqueous carriers that may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate.

Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of presence of microorganisms may be ensured both by sterilization procedures, *supra*, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the composition which produces a therapeutic effect. Generally, out of one hundred per cent, this amount will range from about 0.01 per cent to about ninety-nine percent of active ingredient, preferably from about 0.1 per cent to about 70 per cent, most preferably from about 1 per cent to about 30 per cent of active ingredient in combination with a pharmaceutically acceptable carrier.

Dosage regimens are adjusted to provide the optimum desired response (*e.g.*, a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

For administration of the antibody, the dosage ranges from about 0.0001 to 100 mg/kg, and more usually 0.01 to 5 mg/kg, of the host body weight. For example dosages can be 0.3 mg/kg body weight, 1 mg/kg body weight, 3 mg/kg body weight, 5 mg/kg body weight or 10 mg/kg body weight or within the range of 1-10 mg/kg. An exemplary treatment regime entails administration once per week, once every two weeks, once every three weeks, once every four weeks, once a month, once every 3 months or once every three to 6 months. Preferred dosage regimens for a defucosylated anti-CD30 antibody of the invention include 1 mg/kg body weight or 3 mg/kg body weight via intravenous administration, with the antibody being given using one of the following dosing schedules: (i) every four weeks for six dosages, then every three months; (ii) every three weeks; (iii) 3 mg/kg body weight once followed by 1 mg/kg body weight every three weeks.

In some methods, two or more monoclonal antibodies with different binding specificities are administered simultaneously, in which case the dosage of each antibody administered falls within the ranges indicated. Antibody is usually administered on multiple occasions. Intervals between single dosages can be, for example, weekly, monthly,

every three monthgs or yearly. Intervals can also be irregular as indicated by measuring blood levels of antibody to the target antigen in the patient. In some methods, dosage is adjusted to achieve a plasma antibody concentration of about 1-1000 μg /ml and in some methods about 25-300 μg /ml.

Alternatively, antibody can be administered as a sustained release formulation, in which case less frequent administration is required. Dosage and frequency vary depending on the half-life of the antibody in the patient. In general, human antibodies show the longest half life, followed by humanized antibodies, chimeric antibodies, and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A "therapeutically effective dosage" of an anti-CD30 antibody of the invention preferably results in a decrease in severity of disease symptoms, an increase in frequency and duration of disease symptom-free periods, or a prevention of impairment or disability due to the disease affliction. For example, for the treatment of cancerous tumors, a "therapeutically effective dosage" preferably inhibits cell growth or tumor growth by at least about 20%, more preferably by at least about 40%, even more preferably by at least about 60%, and still more preferably by at least about 80% relative to untreated subjects. The ability of a compound to inhibit tumor growth can be evaluated in an animal model system predictive of efficacy in human tumors. Alternatively, this property of a composition can be evaluated by examining the ability of the compound to inhibit, such inhibition *in vitro* by assays known to the skilled practitioner. A therapeutically effective amount of a therapeutic compound can decrease tumor size, or otherwise ameliorate symptoms in a subject. One of ordinary skill in the art would be able to determine such amounts based on such factors as the subject's size, the severity of the subject's symptoms, and the particular composition or route of administration selected.

A composition of the present invention can be administered via one or more routes of administration using one or more of a variety of methods known in the art. As will be appreciated by the skilled artisan, the route and/or mode of administration will vary depending upon the desired results. Preferred routes of administration for antibodies of the invention include intravenous, intramuscular, intradermal, intraperitoneal, subcutaneous, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Alternatively, a defucosylated antibody of the invention can be administered via a non-parenteral route, such as a topical, epidermal or mucosal route of administration, for example, intranasally, orally, vaginally, rectally, sublingually or topically.

The active compounds can be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. *See, e.g.*, Sustained and Controlled Release Drug Delivery Systems, J.R. Robinson, ed., Marcel Dekker, Inc., New York, 1978.

Therapeutic compositions can be administered with medical devices known in the art. For example, in a preferred embodiment, a therapeutic composition of the invention can be administered with a needleless hypodermic injection device, such as the devices disclosed in U.S. Patent Nos. 5,399,163; 5,383,851; 5,312,335; 5,064,413; 4,941,880; 4,790,824; or 4,596,556. Examples of well-known implants and modules useful in the present invention include: U.S. Patent No. 4,487,603, which discloses an implantable micro-infusion pump for dispensing medication at a controlled rate; U.S. Patent No. 4,486,194, which discloses a therapeutic device for administering medicants through the skin; U.S. Patent No. 4,447,233, which discloses a medication infusion pump for delivering medication at a precise infusion rate; U.S. Patent No. 4,447,224, which discloses a variable flow implantable infusion apparatus for continuous drug delivery; U.S. Patent No. 4,439,196, which discloses an osmotic drug delivery system having multi-chamber compartments; and

U.S. Patent No. 4,475,196, which discloses an osmotic drug delivery system. Many other such implants, delivery systems, and modules are known to those skilled in the art.

In certain embodiments, the defucosylated antibodies of the invention can be formulated to ensure proper distribution *in vivo.* For example, the blood-brain barrier (BBB) excludes many highly hydrophilic compounds. To ensure that the therapeutic compounds of the invention cross the BBB (if desired), they can be formulated, for example, in liposomes. For methods of manufacturing liposomes, see, *e.g.*, U.S. Patents 4,522,811; 5,374,548; and 5,399,331. The liposomes may comprise one or more moieties which are selectively transported into specific cells or organs, thus enhance targeted drug delivery (*see*, *e.g.*, V.V. Ranade (1989) J. Clin. Pharmacol. 29:685). Exemplary targeting moieties include folate or biotin (see, *e.g.*, U.S. Patent 5,416,016 to Low et al.); mannosides (Umezawa et al., (1988) Biochem. Biophys. Res. Commun. 153:1038); antibodies (P.G. Bloeman et al. (1995) FEBS Lett. 357:140; M. Owais et al. (1995) Antimicrob. Agents Chemother. 39:180); surfactant protein A receptor (Briscoe et al. (1995) Am. J Physiol. 1233:134); p120 (Schreier et al. (1994) J. Biol. Chem. 269:9090); see also K. Keinanen; M.L. Laukkanen (1994) FEBS Lett. 346: 123; J.J. Killion; I.J. Fidler (1994) Immunomethods 4:273.

## Uses and Methods of the Invention

The antibodies, antibody compositions and methods of the present invention have numerous *in vitro* and *in vivo* diagnostic and therapeutic utilities involving the diagnosis and treatment of disorders involving CD30 expression. For example, these molecules can be administered to cells in culture, *e.g. in vitro* or *ex vivo,* or to human subjects, *e.g.*, *in vivo*, to treat, prevent and to diagnose a variety of disorders. As used herein, the term "subject" is intended to include human and non-human animals. Non-human animals includes all vertebrates, *e.g.*, mammals and non-mammals, such as non-human primates, sheep, dogs, cats, cows, horses, pigs, chickens, avians, amphibians, and reptiles. Preferred subjects include human patients having disorders characterized by CD30 expression. When antibodies to CD30 are administered together with another agent, the two can be administered in either order or simultaneously.

Other antibodies can be used in combination with anti-CD30 antibodies of the present invention to produce an additive or synergistic effect. These include molecules on the surface of dendritic cells which activate DC function and antigen presentation. Anti-CD40 antibodies are able to substitute effectively for T cell helper activity (Ridge, J. et al. (1998) Nature 393: 474-478) and can be used in conjuction with CD30 antibodies. Activating antibodies to T cell costimulatory molecules such as CTLA-4 (e.g., US Patent No. 5,811,097), OX-40 (Weinberg, A. et al. (2000) Immunol 164: 2160-2169), 4-1BB (Melero, I. et al. (1997) Nature Medicine 3: 682-685 (1997), and ICOS (Hutloff, A. et al. (1999) Nature 397: 262-266) may also provide for increased levels of T cell activation.

Suitable routes of administering the antibody compositions (*e.g.*, antibody or immunoconjugate) of the invention *in vivo* and *in vitro* are well known in the art and can be selected by those of ordinary skill. For example, the antibody compositions can be administered by injection (*e.g.*, intravenous or subcutaneous). Suitable dosages of the molecules used will depend on the age and weight of the subject and the concentration and/or formulation of the antibody composition.

In one embodiment, the antibodies of the invention can be initially tested for binding activity associated with therapeutic or diagnostic use *in vitro.* For example, compositions of the invention can be tested using ELISA and flow cytometric assays. Moreover, the activity of these molecules in triggering at least one effector-mediated effector cell activity, including inhibiting the growth of and/or killing of cells expressing CD30 can be assayed. Protocols for assaying for effector cell-mediated ADCC are described in the Examples below.

### *A. Detection Methods*

In one embodiment, the antibodies of the invention can be used to detect levels of CD30, or levels of cells which contain CD30 on their membrane surface, which levels can then be linked to certain disease symptoms.

In a particular embodiment, the invention provides methods for detecting the presence of CD30 antigen in a sample, or measuring the amount of CD30 antigen, comprising contacting the sample, and a control sample, with a defucosylated antibody, or an antigen binding portion thereof, which specifically binds to CD30, under conditions that allow for formation of a complex between the antibody or portion thereof and CD30. The formation of a complex is then detected, wherein a difference complex formation between the sample compared to the control sample is indicative the presence of CD30 antigen in the sample. For example, standard detection methods, well-known in the art, such as ELISA and flow cytometic assays, can be performed using the compositions of the invention.

Accordingly, in one aspects, the invention further provides methods for detecting the presence of CD30 (*e.g.*, human CD30 antigen) in a sample, or measuring the amount of CD30, comprising contacting the sample, and a control sample, with an antibody of the invention, or an antigen binding portion thereof, which specifically binds to CD30, under conditions that allow for formation of a complex between the antibody or portion thereof and CD30. The formation of a complex is then detected, wherein a difference in complex formation between the sample compared to the control sample

is indicative of the presence of CD30 in the sample.

The compositions of the invention can also be used to target cells expressing CD30, for example for labeling such cells. For such use, the binding agent can be linked to a molecule that can be detected. Thus, the invention provides methods for localizing *ex vivo* or *in vitro* cells expressing CD30. The detectable label can be, *e.g.*, a radioisotope, a fluorescent compound, an enzyme, or an enzyme co-factor.

*B. Inhibition of Growth of CD30+ Cells*

The antibodies can be used to inhibit or block CD30 function which, in turn, can be linked to the prevention or amelioration of certain disease symptoms, thereby implicating CD30 as being involved in the disease. Differences in CD30 expression during a disease state as compared to a non-disease state can be determined by contacting a test sample from a subject suffering from the disease and a control sample with the anti-CD30 antibody under conditions that allow for the formation of a complex between the antibody and CD30. Any complexes formed between the antibody and CD30 are detected and compared in the sample and the control.

For example, the antibodies can be used to elicit *in vivo* or *in vitro* one or more of the following biological activities: to inhibit the growth of and/or kill a cell expressing CD30; to mediate phagocytosis or ADCC of a cell expressing CD30 in the presence of human effector cells; to inhibit shedding of soluble CD30, to block CD30 ligand binding to CD30, to inhibit IL-4 expression or to mediate expression of the Th2 phenotype, *e.g.*, at low dosages. As discussed herein, the defucosylated antibodies of the invention exhibit enhanced ADCC activity as compared to the fucosylated form of the antibody.

Accordingly, in another aspect, the invention provides a method of inhibiting growth of $CD30^+$ cells comprising contacting said cells with a defucosylated anti-CD30 antibody under conditions sufficient to induce antibody-dependent cellular cytoxicity (ADCC) of said cells. The cells can be, for example, tumor cells. In a preferred embodiment, the anti-CD30 antibody is a human antibody.

In one embodiment, the antibodies, or binding portions thereof, of the present invention can be used to modulate CD30 levels on target cells, such as by capping and eliminating receptors on the cell surface. Mixtures of anti-Fc receptor antibodies can also be used for this purpose.

Target-specific effector cells, *e.g.*, effector cells linked to compositions of the invention can also be used as therapeutic agents. Effector cells for targeting can be human leukocytes such as macrophages, neutrophils or monocytes. Other cells include eosinophils, natural killer cells and other IgG- or IgA-receptor bearing cells. If desired, effector cells can be obtained from the subject to be treated. The target-specific effector cells, can be administered as a suspension of cells in a physiologically acceptable solution. The number of cells administered can be in the order of $10^8$-$10^9$ but will vary depending on the therapeutic purpose. In general, the amount will be sufficient to obtain localization at the target cell, *e.g.*, a tumor cell expressing CD30, and to effect cell killing by, *e.g.*, phagocytosis. Routes of administration can also vary.

Therapy with target-specific effector cells can be performed in conjunction with other techniques for removal of targeted cells. For example, anti-tumor therapy using the compositions of the invention and/or effector cells armed with these compositions can be used in conjunction with chemotherapy. Additionally, combination immunotherapy may be used to direct two distinct cytotoxic effector populations toward tumor cell rejection.

*C. Use of Immunoconjugates and Combination Therapy*

In one embodiment, immunoconjugates of the invention can be used to target compounds (*e.g.*, therapeutic agents, labels, cytotoxins, radiotoxins immunosuppressants, etc.) to cells which have CD30 cell surface receptors by linking such compounds to the antibody. For example, an anti-CD30 antibody can be conjugated to any of the toxin compounds described in US Patent Nos. 6, 281, 354 and 6,548,530, US patent publication Nos. 20030050331, 20030064984, 20030073852, and 20040087497, published in WO 03/022806, WO05/112919 or disclosed in [the U.S. Patent application corresponding to Darby & Darby LLP attorney docket No. 0203496-WO0, filed on April 7, 2006]. Thus, the invention also provides methods for localizing *ex vivo* or *in vitro* cells expressing CD30 (*e.g.*, with a detectable label, such as a radioisotope, a fluorescent compound, an enzyme, or an enzyme co-factor). Alternatively, the immunoconjugates can be used to kill cells which have CD30 cell surface receptors by targeting cytotoxins or radiotoxins to CD30, such as to CD30-expressing tumor cells to thereby eliminate the tumor cell, or to CD30-expressing antigen-presenting cells to thereby eliminate the APCs as a means to inhibit immune responses (*e.g.*, in autoimmune disorders).

In other embodiments, the subject can be additionally treated with an agent that modulates, *e.g.*, enhances or inhibits, the expression or activity of Fcγ or Fcγ receptors by, for example, treating the subject with a cytokine. Preferred cytokines for administration during treatment include of granulocyte colony-stimulating factor (G-CSF), granulocyte-macrophage colony-stimulating factor (GM-CSF), interferon-γ (IFN-γ), and tumor necrosis factor (TNF).

In another embodiment, the subject can be additionally treated with a lymphokine preparation. Cancer cells

which do not highly express CD30 can be induced to do so using lymphokine preparations. Lymphokine preparations can cause a more homogeneous expression of CD30 among cells of a tumor which can lead to a more effective therapy. Lymphokine preparations suitable for administration include interferon-gamma, tumor necrosis factor, and combinations thereof. These can be administered intravenously. Suitable dosages of lymphokine are 10,000 to 1,000,000 units/patient.

In another embodiment, patients treated with antibody compositions of the invention can be additionally administered (prior to, simultaneously with, or following administration of an antibody of the invention) with another therapeutic agent, such as a cytotoxic or radiotoxic agent, which enhances or augments the therapeutic effect of the human antibodies, or another antibody. The antibody can be linked to the agent (as an immunocomplex) or can be administered separate from the agent. In the latter case (separate administration), the antibody can be administered before, after or concurrently with the agent or can be co-administered with other known therapies, e.g., an anti-cancer therapy, e.g., radiation. Such therapeutic agents include, among others, anti-neoplastic agents such as doxorubicin (adriamycin), cisplatin bleomycin sulfate, carmustine, chlorambucil, and cyclophosphamide hydroxyurea which, by themselves, are only effective at levels which are toxic or subtoxic to a patient. Cisplatin is intravenously administered as a 100 mg/ml dose once every four weeks and adriamycin is intravenously administered as a 60-75 mg/ml dose once every 21 days. Co-administration of the anti-CD30 antibodies, or antigen binding fragments thereof, of the present invention with chemotherapeutic agents provides two anti-cancer agents which operate via different mechanisms which yield a cytotoxic effect to human tumor cells. Such co-administration can solve problems due to development of resistance to drugs or a change in the antigenicity of the tumor cells which would render them unreactive with the antibody.

*D. Treatment of Autoimmune Diseases*

The compositions can be used *in vitro* or *in vivo* to treat diseases mediated by or involving CD30, for example, diseases characterized by expression, typically overexpression, of CD30 such as autoimmune diseases mediated by macrophages, activated neutrophils, dendritic cells or NK cells, such as thyroid autoimmune diseases, such as Graves' Disease and Hashimoto's thyroiditis, autoimmune diabetes and multiple sclerosis (Ruggeri et al. (2006) Histol Histolpathol. 21:249-56; Chiarle et al. (2003) Pathologica 95:229-30; Chakrabarty et al. (2003) Clin Exp Immunol 133:318-225; Watanabe et al. (2003) Thyroid 13:259-63; Pellegrini et al. (2005) Neuroimmunomodulation 12:220-34). Soluble CD30 is regularly shed from the surface of cells expressing CD30 and elevated sCD30 levels have been reported in the serum of patients with a variety of tumorigenic and autoimmune disorders. Accordingly, yet another use for the antibodies of the invention includes the prevention or treatment of diseases involving blocking or inhibiting of shedding of sCD30.

By contacting the antibody with CD30 (*e.g.*, by administering the antibody to a subject), the ability of CD30 to induce such activities is inhibited and, thus, the associated disorder is treated. The antibody composition can be administered alone or along with another therapeutic agent, such as an immunosuppressant which acts in conjunction with or synergistically with the antibody composition to treat or prevent the CD30 mediated disease. Preferred antibodies bind to epitopes which are specific to CD30 and, thus, advantageously inhibit CD30 induced activities, but do not interfere with the activity of structurally related surface antigens. The compositions can be used to treat any diseases mediated by CD30 expressing cells, including, but not limited to, autoimmune hemolytic anemia (AIHA), rheumatoid arthritis (RA), systemic lupus-erythematosus (SLE), Systemic Sclerosis, Atopic Dermatitis, Graves' disease, Hashimoto's thyroiditis, Wegner's granulomatosis, Omen's syndrome, chronic renal failure, idiopathic thrombocytopenic purpura (ITP), inflammatory bowel disease (IBD; including Crohn's Disease, Ulcerative Colitis and Celiac's Disease), insulin dependent diabetes mellitus (IDDM), acute infectious mononucleosis, HIV, herpes virus associated diseases, multiple sclerosis (MS), transplantation rejection, allergy or Graft versus Host Disease (GVHD), hemolytic anemia, thyroiditis, stiff man syndrome, pemphigus vulgaris and myasthenia gravis (MG).

*E. Treatment of Cancer*

In another embodiment, the present invention provides a method of inhibiting the growth of CD30+ tumor cells (*i.e.*, tumor cells expressing CD30) in a subject, in which a defucosylated anti-CD30 antibody of the invention is administered to the subject such that growth of the CD30+ tumor cells is inhibited. For human subjects, the antibody preferably is a humanized or human antibody. In a preferred embodiment, the tumor cells are Hodgkin's Disease tumor cells. In another preferred embodiment, the tumor cells are anaplastic large-cell lymphomas (ALCL) tumor cells. In other embodiments, the tumor cells may be from a disease selected from the group consisting of non-Hodgkin's lymphoma, Burkitt's lymphoma, cutaneous T-cell lymphomas, nodular small cleaved-cell lymphomas, lymphocytic lymphomas, peripheral T-cell lymphomas, Lennert's lymphomas, immunoblastic lymphomas, T-cell leukemia/lymphomas (ATLL), adult T-cell leukemia (T-ALL), entroblastic/centrocytic (cb/cc) follicular lymphomas cancers, diffuse large cell lymphomas of B lineage, angioimmunoblastic lymphadenopathy (AILD)-like T cell lymphoma, adult T-cell lymphoma (ATL), HIV associated body cavity based lymphomas, Embryonal Carcinomas, undifferentiated carcinomas of the rhino-pharynx (*e.g.*, Schmincke's tumor), Castleman's disease, Kaposi's Sarcoma and other CD30+ T-cell lymphomas and CD30+ B-cell

lymphomas.

The method involves administering to a subject an antibody composition of the present invention in an amount effective to treat or prevent the disorder. The antibody composition can be administered alone or along with another therapeutic agent, such as a cytotoxic or a radiotoxic agent which acts in conjunction with or synergistically with the antibody composition to treat or prevent the disease associated with CD30 expression.

Kits

Also within the scope of the invention are kits comprising an antibody of the invention and instructions for use. The kit can further contain one or more additional reagents, such as an immunostimulatory reagent, a cytotoxic agent or a radiotoxic agent, or one or more additional antibodies of the invention (*e.g.*, an antibody having a complementary activity which binds to an epitope in the CD30 antigen distinct from the first antibody). Kits typically include a label indicating the intended use of the contents of the kit. The term label includes any writing, or recorded material supplied on or with the kit, or which otherwise accompanies the kit.

The present invention is further illustrated by the following examples.

**EXAMPLES**

**Example 1: Preparation and Characterization of Defucosylated Anti-CD30 Monoclonal Antibody**

In this example, a fully human anti-CD30 monoclonal antibody was expressed in a cell line lacking a fucosyl transferase enzyme such that the cell line produces proteins lacking fucosyl in their carbohydrates. The defucosylated antibody was tested against a fucosylated anti-CD30 antibody (expressed in a different cell line that contains the fucosyl transferase enzyme) to determine structural and characteristic differences between the antibodies, using a variety of chemical analysis techniques, including capillary electrophoresis, comparison of amino acid sequence, mass differences by mass spectroscopy and charge variation by capillary isoelectric focusing.

The anti-CD30 fully human monoclonal antibody 5F11 was originally described in PCT Publication WO 03/059282. The amino acid and nucleotide sequences of the 5F11 heavy chain is shown in Figure 1A and the amino acid and nucleotide sequences of the SF11 light chain are shown in Figure 1B. The 5F11 heavy and light chain variable sequences were subcloned into an expression vector. The 5F11 kappa variable region cDNA, including its signal sequence and an optimal Kozak sequence, was subcloned in frame with the human kappa constant region. The 5F11 heavy chain variable region cDNA, including its signal sequence and an optimal Kozak sequence, was subcloned in frame with the human γ1 heavy constant region. Both light and heavy chain expression were driven by human ubiquitin C promoters (Nenoi, M. et al. Gene 175;179, 1996). This expression vector is described in further detail in U.S. Patent Publication US 2005 153394.

The expression vector was transfected into the $FUT8^{-/-}$ host cell line Ms704 by DNA electroporation. The Ms704 $FUT8^{-/-}$ cell line was created by the targeted disruption of the FUT8 gene in CHO/DG44 cells using two replacement vectors, and is more fully described in U.S. Patent Publication 20040110704 by Yamane et al. and Yamane-Ohnuki et al. (2004) Biotechnol Bioeng 87:614-22. The Ms704 cells were adapted to growth in suspension culture in growth medium, EX-CELL™ 325 PF CHO Medium (JRH #14335) supplemented with 100 μM hypoxanthine with 16 μM thymidine (Invitrogen #11067-030) and 6 mM L-glutamine (Invitrogen #25030-081).

The vector DNA to be used for electroporation was ethanol precipitated and resuspended in 10 mM Tris 7.6, 1 mM EDTA. 1, 5, 10, 15 or 20 μg DNA was utilized for twenty electroporations, four electroporations per DNA concentration. The Ms704 cells were prepared for transfection by washing the cells in a sucrose-buffered solution (SBS) and resuspending the cells at 1 X $10^7$ cells/ml SBS solution. 400 μl cells were mixed with construct DNA and electroporated utilizing settings at 230 volts, 400 microfaradays capacitance and 13 ohms resistance (BTX Molecular Delivery Systems #600 electro cell manipulator). The cells were removed from the electroporation cuvettes and 20 ml growth medium was added. The cells were plated into a 96 well dish using 200 μl cells per well, approximately 4 X $10^4$ cells/well. 2 days after the electroporation, 150 μl of medium was removed from each well and replaced with 150 μl selection medium, growth medium with 400 μg/ml G418 (Invitrogen #10131-035). Every three to seven days, 150 μl of selection medium per well was replaced with fresh selection medium. CHO DG44 host cells (FUT 8 +/+) were electroporated with the identical 5F11 construct using a similar procedure and CHO DG44 transfectants expressing recombinant 5F11 antibody containing fucosylated carbohydrates were established.

The highest producing Ms704 and CHO DG44 clones were expanded and recombinant 5F11 antibody was purified from cell culture supernatants by Protein A affinity chromatography.

Comparative analysis of N-linked oligosaccharides derived from the Ms704 and the CHO DG44 derived anti-CD30 monoclonal antibody samples was done by capillary electrophoresis laser induced fluorescence (cLIF) (Chen and Evangelista (1998) *Electrophoresis* 15:1892). The N-linked oligosaccharides of the purified antibody were released by

adding the peptide N-glycanase (Prozyme) and incubating overnight. The protein was ethanol precipitated, and the carbohydrate containing supernatant was transferred to a new tube and dried using a Speedvac. The carbohydrates were resuspended and derivatized with 8-aminopyrene-1,3,6-trisulfonate (APTS) under mild reductive amination conditions in which desialylation and loss of fucosyl residues was minimized. The reaction adducts were analyzed by capillary electrophoresis with a laser-induced fluorescence detector (Beckman Coulter) (Ma and Nashabeh (1999) *Anal. Chem.* 71:5185). Differences in the oligosaccharide profile were observed between the antibody obtained from the Ms704 cell line as compared to the CHO DG44 cell line, consistent with an absence of fucosyl residues in the Ms704 derived anti-CD30 antibodies.

To confirm the absence of fucosyl residues on the antibody expressed in Ms704 cells, monosaccharide composition analysis was performed. The results are shown below in Table 1:

**Table 1: Monosaccharide Analysis**

| Antibody | Protein Amount (μg) | Monosaccharide | Amount Found (pmol) | mol Sugar/ mol Protein |
|---|---|---|---|---|
| Anti-CD30 + fucosyl | 29 μg | Fucosyl | 206.0 | 1.0 |
| | | Galactosamine | 0.0 | 0.0 |
| | | Glucosamine | 847.6 | 4.4 |
| | | Galactose | 85.8 | 0.5 |
| | | Mannose | 547.0 | 2.9 |
| Anti-CD30 - fucosyl | 23 μg | Fucosyl | 0.0 | 0.0 |
| | | Galactosamine | 0.0 | 0.0 |
| | | Glucosamine | 655.2 | 4.3 |
| | | Galactose | 89.7 | 0.6 |
| | | Mannose | 488.8 | 3.2 |

The results of the monosaccharide analysis confirm that the antibody expressed in Ms704 cells lacks fucosyl residues.

Aside from the difference in oligosaccharides shown by capillary electrophoresis and monosaccharide analysis, the Ms704 and CHO DG44 derived anti-CD30 antibody protein samples were essentially identical. Analysis of N-terminal protein sequence revealed an identical N-terminal amino acid sequence. Mass spectroscopy of the light chain of the Ms704 and CHO DG44 derived anti-CD30 antibodies yielded masses of 23,740 and 23,742, respectively, which were within the error of the instrument. The two antibodies were also tested using a standard capillary isoelectric focusing kit assay (Beckman Coulter) and showed that the two antibody samples had an identical isoelectric point at 8.6. These studies indicate that the protein component of the antibody samples derived from the Ms704 and the CHO DG44 cells are essentially identical with the exception of the defucosylation of the carbohydrate component of the Ms704 derived antibodies.

**Example 2: Assessment of ADCC Activity of Defucosylated Anti-CD30 Antibody**

The anti-CD30 monoclonal antibody 5F11 is capable of killing CD30+ cells through the recruitment of an effector cell population via antibody dependent cellular cytotoxicity (ADCC). In this example, defucosylated 5F11 (defuc-5F11) monoclonal antibodies were tested for the ability to kill CD30+ cell lines in the presence of effector cells in a cytotoxicity chromium release assay.

Human effector cells were prepared from whole blood as follows. Human peripheral blood mononuclear cells were purified from heparinized whole blood by standard Ficoll-paque separation. The cells were resuspended in RPMI1640 media containing 10% FBS and 200 U/ml of human IL-2 and incubated overnight at 37°C. The following day, the cells were collected and washed once in culture media and resuspended at 1 x $10^7$ cells/ml. Two million target CD30+ cells were incubated with 200 μCi $^{51}$Cr in 1 ml total volume for 1 hour at 37° C. The target cells were washed once, resuspended in 1ml of media, and incubated at 37° C for an additional 30 minutes. After the final incubation, the target cells were washed once and brought to a final volume of $1 \times 10^5$ cells/ml.

The CD30+ cell lines L540 (human Hodgkin's lymphoma; DSMZ Deposit No. ACC 72), L428 (human Hodgkin's lymphoma; DSMZ Deposit No. ACC 197), L1236 (human Hodgkin's lymphoma; DSMZ Deposit No. ACC 530) and Karpas (human T cell lymphoma; DSMZ Deposit No. ACC 31) cell lines were initially tested for binding to both the fucosylated 5F11 (fuc-5F11) and defuc-5F11 using a standard FACS analysis. Each target cell displayed similar binding profiles through a range of antibody concentrations for both fuc-5F11 and defuc-SF1 1. The level of CD30 expression, as determined by mean fluorescence intensity, was highest in L540, followed by Karpas, L428, and the lowest CD30 expression was on L1236 cells.

The L540, L428, L1236 and Karpas cells were tested in a modified $^{51}$Cr antibody dependent cellular cytotoxicity (ADCC) assay as follows. Each target cell line (100 μl of labeled CD30+ cells) was incubated with 50 μl of effector cells and 50 μl of antibody. A target to effector ratio of 1:50 was used throughout the experiments. In all studies, the following negative controls were also run: a) target and effector cells without antibody, b) target cells without effector cells, c) wells containing target and effector cells in the presence of 1% Triton X-100, and d) human IgG1 isotype control. Following a 4 hour incubation at 37° C, the supernatants were collected and counted on a gamma Counter (Cobra II auto-gamma from Packard Instruments) with a reading window of 240-400 keV. The counts per minute were plotted as a function of antibody concentration and the data was analyzed by non-linear regression, sigmoidal dose response (variable slope) using Prism software (San Diego, CA). Cell cytotoxicity curves for the L540, L428, L1236 and Karpas cell lines using varying concentrations of fuc-SF1 and defuc-5F11 are shown in Figures 4-7, respectively.

The percent lysis was determined by the following equation:

**% Lysis = (Sample CPM- no antibody CPM)/TritonX CPM-No antibody CPM) X 100**

The % Lysis was tested at an antibody concentration of 25 μg/ml and a target to effector cell ratio of 1:50. $EC_{50}$ values also were calculated for each target cell. The results are summarized in Table 2 below.

**Table 2: Cytotoxic Ability of Defucosylated Anti-CD30 Monoclonal Antibody**

| Target cell | % Lysis | % Lysis | % Lysis ratio fucosyl - : fucosyl + | $EC_{50}$ (μg/ml) | $EC_{50}$ (μg/ml) | $EC_{50}$ ratio fucosyl+: fucosyl- |
|---|---|---|---|---|---|---|
| | Fucosyl + | Fucosyl- | | Fucosyl + | Fucosyl - | |
| L540 | 42 | 68 | 1.61 | 0.042 | 0.009 | 4.7 |
| Karpas | 19 | 50 | 2.63 | 0.250 | 0.032 | 7.8 |
| L428 | 20 | 43 | 2.15 | 0.100 | 0.009 | 11.1 |
| L1236 | 4 | 13 | 3.25 | 1.218 | 0.045 | 27.1 |

Defuc-5F11 showed from 1.61 times (for L540 cells) to 3.25 times (for L1236 cells) greater percent cell lysis as compared to the fuc-5F11 antibody. This increased potency of the defuc-5F11 results in measurable cell lysis at antibody concentrations where the fuc-SF11 has no measurable effect. For example, on L1236 cells, which have a low level of expression of CD30, defuc-5F11 at 0.1 μg/ml results in a 10% specific lysis, whereas fuc-5F11 at the same concentration has no measurable effect (see Figure 6). Defuc-5F11 was 4.7 times (for L540 cells) to 27.1 times (for L1236 cells) more potent in ADCC activity than the fuc-SF11 antibody, as measured by ratio of $EC_{50}$ values.

**Example 3: Assessment of ADCC Activity of Anti-CD30 Antibody**

In this example, anti-CD30 monoclonal antibodies were tested for the ability to kill CD30+ cell lines in the presence of effector cells via antibody dependent cellular cytotoxicity (ADCC) in a fluorescence cytotoxicity assay. Human effector cells were prepared as described above and the ADCC assay performed as indicated above. As can be seen in Figure 9, when using the defucosylated anti-CD30 antibody there was increased ADCC activity as compared with parental anti-CD30 antibody. In addition, the defucosylated anti-CD30 antibody was more potent than the parental antibody as evidenced by the reduced EC50 as compared to the parental anti-CD30 antibody. The antibody was also more efficacious as evidenced by the fact that the maximum percent lysis was higher for the defucosylated anti-CD30 antibody. With either antibody, the anti-CD16 (3G8) antibody effectively inhibited the ADCC suggesting that this lysis was mediated by CD16.

**Example 4: Increased ADCC With Human Effector Cells.**

ADCC assays were performed as described above. In this experiment, however, mouse effector cells were compared with human effector cells. As can be seen in Figure 10, while there was no increased ADCC comparing parental anti-CD30 antibody with defucosylated antibody when mouse effector cells were used, when human effector cells were examined, there was a notable increase in ADCC with the defucosylated antibody as compared to the parental anti-CD30 antibody.

**Example 5: ADCC assay comparing parental and defucosylated antibody using effector cells from cynomolgus monkeys**

Whole blood was obtained from cynomolgus monkeys. Red blood cell lysed cynomolgus peripheral blood cells were stimulated with 50 U/ml rIL-2 and cultured in RPMI1640 media containing 10% FBS overnight at 37°C. On the day of the study, cynomolgus cells were resuspended in assay buffer (RPMI1640, 10% FBS, 2.5 mM probenecid) at $1x16^7$ cells/mL. CD30 positive target cells, Karpas 299, were labeled, washed three times with wash buffer (PBS, 2.5mM probenecid, 20mM HEPES), and adjusted to $1x10^5$ cells/mL for 1:50 target to effector cell ratio. The ADCC assay was performed as described above. We compared the activity of parental anti-CD30 antibody to defucosylated antibody using effector cells purified from cynomolgus blood. Modest ADCC activity was seen with the parental antibody (from around 7-10% cell lysis at 10 μg/mL). In contrast, the defucosylated antibody induced significantly higher percent lysis (from around 10-30% cell lysis at 10 μg/mL) and a reduced EC50 (see Figure 11).

**Example 6: Scatchard analysis of binding affinity of anti-CD30 monoclonal antibodies to L540 cells, activated human and cynomolgus peripheral blood cells**

The binding affinity of the parental and defucosylated anti-CD30 antibodies was determined. We compared the binding affinity of the two antibodies to CD30 positive L540 cells as well as PHA/Con A-activated human or cynomolgus peripheral blood mononuclear cells.

Human or cynomolgus peripheral blood cells were stimulated with 2μg/ml PHA, 10 μg/ml Con A, and 50 U/ml rIL-2 and cultured in RPMI1640 media containing 10% fetal bovine serum (FBS) at $1x10^6$ cells/ml density for 3 days. On the day of the study, the cells were washed and adjusted to $2x10^7$ cells/ml in binding buffer (RPMI1640 +10% FBS). As a control, CD30 positive L540 cells (adjusted to $4\text{-}8x10^6$ cells/ml) were used in these studies since they express high levels of the antigen. The cells were placed on ice until the initiation of the experiment. Millipore glass fiber filter plates (MAFBN0B50) were coated with 1% nonfat dry milk in water and stored a 4°C overnight. The plates were washed three times with 0.2 ml of binding buffer. Fifty microliters of buffer alone was added to the maximum binding wells (total binding). Twenty-five microliters of buffer alone was added to the control wells. Varying concentration of $^{125}$I-anti-CD30 antibody was added to all wells in a volume of 25 μl. Varying concentrations of unlabeled antibody at 300-400 fold excess were added in a volume of 25 μl to control wells (non-specific binding) and 25 μl of CD30 positive L540 cells or stimulated human or cynomolgus peripheral blood cells in binding buffer were added to all wells. The plates were incubated for 2 hours at 200 RPM on a shaker at 4°C. At the completion of the incubation the Millipore plates were washed twice with 0.2 ml of cole wash buffer (RPMI1640, 10% FBS, 500 mM NaCl). The filters were removed and counted in a gamma counter. Evaluation of equilibrium binding was performed using single site binding parameters with the Prism software (San Diego, CA).

Using the above Scatchard binding assay, the $K_D$ of the parental CD30 antibody for L540 cells was approximately 1.4 nM while the defucosylated antibody had a $K_D$ of 1.9 nM (Table 3). This indicates that there was little change in affinity with removal of fucosyl. These studies were repeated using primary cells rather than a cell line. In addition, the affinity on cells which express significantly fewer receptors per cell was tested. Activated human peripheral blood cells were prepared as indicated above and the $K_D$ was found to be1.1 and 2.7 nM for parental and defucosylated anti-CD30 antibody, respectively.

Finally, the binding affinity of the parental and defucosylated antibody for PHA, Con A, and rIL-2 activated cynomolgus peripheral blood mononuclear cells was compared. The $K_D$ was found to be approximately 0.47 nM and 0.83 nM for parental and defucosylated antibody, respectively.

**Table 3 Scatchard Analysis**

| Sample | | L540 | Human | Cynomolgus |
|---|---|---|---|---|
| Parental CD30 | KD (nM ave) | 1.37 | 1.08 | 0.47 |
| | Receptors Per Cell (ave) | 2496082 | 45654 | 72781 |
| Defucosylated CD30 | KD (nM ave) | 1.93 | 2.66 | 0.83 |
| | Receptors Per Cell (ave) | 3024600 | 74258 | 108824 |

**Example 7: Production of Anti-CD30 Monoclonal Antibody Having Improved Receptor Binding and Increased ADCC Activity**

This example outlines the expression of human anti-CD30 mAbs in *Lemna.* Anti-CD30 fully human monoclonal

antibodies were originally described in PCT Publication WO 03/059282, which is hereby incorporated by reference. Optimization of anti-CD30 mAb glycosylation was accomplished by co-expression with an RNAi construct targeting the endogenous expression of α-1,3-fucosyltransferase (FucT) and β-1,2-xylosyltransferase (XylT) genes in a manner similar to that noted in the examples above for mAbI. The resultant anti-CD30 mAb produced in *Lemna* having its native glycosylation machinery engineered to suppress FucT and XylT expression contained a single major *N*-glycan species without any trace of plant-specific *N*-glycans. In addition to the *N*-glycan homogeneity, glyco-optimized anti-CD30 mAbs were also shown to have enhanced antibody-dependent cell-mediated cytotoxicity (ADCC) and effector cell receptor binding activity when compared to CHO-expressed anti-CD30 nabs.

METHODS

*Strains and Reagents.*

Novablue competent *Escherichia coli* cells were used for all recombinant DNA work (EMD Biosciences, San Diego, CA). Restriction endonucleases and DNA modification enzymes were obtained from New England Biolabs (Ipswich, MA). Oligonucleotides were obtained from Integrated DNA technologies (Coralville, IA). Waters Oasis HLB and MCX columns (1 cc), 2,5-dihydroxybenzoic acid (DHB), and α-cyano-4-hydroxycinnamic acid (CHCA) were from Waters Corporation (Milford, MA). Purified dabsylated, tetrapeptide, GnGn *N*-glycan acceptors (GnGn-dabsyl-peptide) and *N*-glycosidase A were from EMD Biosciences. Carbograph SPE columns (4 cc) were from Grace Davidson Discovery Sciences (Deerfield, IL). Uridine-5'-diphospho-D-xylosyl (UDP-Xyl) was purchased from Carbosource Services (Athens, GA). Acetonitrile (Optima grade) was from Fisher Scientific (Summerville, NY). Ammonium acetate was from MP Biochemicals (Irvine, CA). Maltooligosaccharides (MD6-1) were from V-Labs Inc. (Covington, CA). Monosaccharide standards were from Dionex (Sunnyvale, CA). BATDA (bis(acetoxymethyl)2,2':6',2"-terpyridine-6,6"-dicarboxylate) and Europium solution were from Perkin-Elmer (Wellesley, MA). Guanosine-5'-diphospbo-L-fucasyl (GDP-Fuc), *N*-acetylglucosamine (GlcNAc), 2-aminobenzoic acid (2-AA) and all other materials were from Sigma (St. Louis, MO).

*Construction of mAb and RNAi expression vectors.*

The heavy (H) and light (L) chain variable region cDNA sequences of fully human mAb1 kappa antibody 5F11 derived from a transgenic Medarex HuMAb-Mouse® were determined and the full length 5F11 human mAb antibody was produced recombinantly by a Chinese hamster ovary cell line, CHO DG44, using standard techniques. Optimized genes for H and L chains were designed to have *Lemna*-preferred codon usage (63%-67% GC content) and contain the rice α-amylase signal sequence (GenBank M24286) fused to the 5' end of their coding sequences. Restriction endonuclease sites were added for cloning into *Agrobacterium* binary vectors (EcoRI (5') / SacI (3'), H-chain) and (SalI (5') / HindIII (3'), L-chain). Synthetic genes were constructed and provided by Picoscript (Houston, TX).

A chimeric hairpin RNA was designed to target silencing of endogenous *Lemna* genes encoding α-1,3-fucosyltransferase (based on the coding sequence for *L. minor* FucT isoform #1, see GenBank DQ789145) and β-1,2-xylosyltransferase (based on the coding sequence for *L. minor* XylT isoform #2, see GenBank DQ789146). The chimeric FucT + XylT hairpin RNA was designed to have 602 bp of double stranded FucT sequence, 626 bp of double stranded XylT sequence, and 500 bp of spacer sequence. The sense strand portion of the hairpin RNA cassette encompasses the FucT forward fragment and XylT forward fragment, a spacer sequence. The antisense strand portion of the hairpin RNA encompasses the XylT reverse fragment and FucT reverse fragment. The chimeric hairpin RNA was constructed by PCR amplifying FucT and XylT forward and reverse gene fragments from *Lemna minor* cDNA and sequentially cloning them into pT7blue (EMD Biosciences) creating plasmid XF02 in T7-4. The FucT forward gene fragment was amplified with DNA primers BLX 686 (5'-ATGGTCGACTGCTGCTGGTGCTC TCAAC-3') (SEQ ID NO:36) and BLX690 (5'- ATGTCTAGAATG CAGCAGCAAGTGCACC-3') (SEQ ID NO:37) producing a 620 bp product with terminal SalI (5') and XbaI (3') cloning sites. The XylT forward gene fragment was amplified with DNA primers BLX 700 (5'-ATGACTAGTTGC GAAGCCTACTTCGGCAACAGC3') (SEQ ID NO:38) and BLX694 (5'-ATGGGATCCGAATCTCAAGA ACAACTGTCG-3') (SEQ ID NO:39) producing a 1144 bp product with terminal SpeI (5') and BamHI (3') cloning sites. The XylT reverse gene fragment was amplified with DNA primers BLX 695 (5'-ATGGGTACCTGCGAAGCCTACTTCGGCAA CAGC-3') (SEQ ID NO:40) and BLX696 (5'- ATGGGA TCCACTGGCTGGGAGAAGTTCTT-3') (SEQ ID NO:41) producing a 644 bp product with terminal BamHI (5') and KpnI (3') cloning sites. The FucT reverse gene fragment was amplified with DNA primers BLX 691 (5'-ATGGAGCTCTGCTGCTGGTGCT CTCAAC-3') (SEQ ID NO:42) and BLX692 (5'-ATGGGTACCATGCAGCAGCAAGTGCACC-3') (SEQ ID NO:43) producing a 620 bp product with terminal KpnI (5') and SacI (3') cloning sites.

Independent expression cassettes containing promoter, gene of interest, and Nos terminator were created for the optimized 5F11 H and L chains and the chimeric RNAi. Expression cassettes were cloned into a modification of the *Agrobacterium* binary vector pBMSP3 (obtained from Dr. Stan Gelvin, Purdue University) with the appropriate restriction

sites. The H chain was fused to the modified chimeric octopine and mannopine synthase promoter with *Lemna gibba* 5' RbcS leader[36]. The L-chain was fused to the high expression, constitutive *Lemna minor* polyubiquitin promoter (LmUbq). The chimeric RNAi cassette, taken from plasmid XF02 in T7-4, was fused to the high expression, constitutive *Spirodela polyrhiza* polyubiquitin promoter (SpUbq). The three expression cassettes were cloned into the modified pBMSP3 binary vector in tandem orientation creating plasmid MDXA04.

*Transformation and plant line screening.*

Using *Agrobacterium tumefaciens* CS8Z707, transgenic plants representing individual clonal lines were generated from rapidly growing *Lemna minor* nodules according to the procedure of Yamamoto *et al*. For transgenic screening, individual clonal lines were preconditioned for 1 week at 150 to 200 μmol $m^{-2}s^{-2}$ in vented plant growth vessels containing SH media without sucrose. Fifteen to twenty preconditioned fronds were then placed into vented containers containing fresh SH media, and allowed to grow for two weeks. Tissue and media samples from each line were frozen and stored at -70°C.

*ELISA analysis of mAb produced in Lemna.*

*Lemna* tissue (100 mg) was homogenized using a FastPrep FP120 bead mill (Thermo Electron Corporation). Supernatants were diluted to 1 μg/mL and assayed using the IgG Quantitation ELISA kit (Bethyl Laboratories). For the assay, microtiter plates were coated with goat anti-human IgG at a concentration of 10 μg/mL, and mAb was detected by horseradish peroxidase (HRP)-conjugated goat anti-human IgG diluted 1:100,000. Standard curves were created with Human Reference IgG supplied with the ELISA kit. The sensitivity of the ELISA was 7.8 ng/mL. All samples were analyzed in duplicate.

*Preparation of Lemna microsomal membranes and assaying for core β-1,2-xylosyltransferase and α-1,3-fucosyltransferase activities.*

*Lemna* tissue (100 mg) from each line was homogenized in 1 mL of cold homogenization buffer (50 mM 4-[2-hydroxyethyl]-1-piperazineethanesulfonic acid [HEPES], pH 7.5, 0.25 M sucrose, 2 mM ethylenediaminetetraacetic acid [EDTA], 1 mM 1,4-dithiothreitol [DTT]) for 40 s in a FastPrep FP120 bead mill (Thermo Electron Corporation, Waltham, MA). The homogenate was centrifuged at 1,000 g for 5 min at 4°C. The supernatant was removed and centrifuged at 18,000g for 90 min at 4°C. The resulting pellet was resuspended in 20 μL of cold reaction buffer (0.1 M 2-[4-morpholino] ethanesulfonic acid [Mes], pH 7.0, 0.1% [v/v] Triton X-100, 10 mM $MnCl_2$) and kept on ice or stored at -80°C until use.

Core β-1,2-xylosyltransferase and α-1,3-fucosyltransferase activities were measured simultaneously in 4 μL of microsomal membranes prepared from each RNAi line by incubating with 125 mM GlcNAc, 6.25 mM UDP-Xyl, 6.25 mM GDP-Fuc, 12.5 mM $MnCl_2$, and 1.5 nmol of GnGn-dabsyl-peptide acceptor for 2 h at 37°C as described previously. The reaction was terminated by a brief centrifugation and incubation at 4°C and the products were analyzed by positive reflectron mode MALDI-TOF MS.

*Purification of 5F11 LEX and $LFX^{Opt}$ mAbs.*

Plant tissue was homogenized with 50 mM sodium phosphate, 0.3 M sodium chloride, and 10 mM EDTA, pH 7.2 using a Silverson high shear mixer. The homogenate was acidified to pH 4.5 with 1 M citric acid, and centrifuged at 7,500g for 30 min at 4°C. The pH of the supernatant was adjusted to pH 7.2 with 2 M Tris, prior to filtration using 0.22 μm filters. The material was loaded directly on mAbSelect SuRe protein A resin (GE Healthcare) equilibrated with a solution containing 50 mM sodium phosphate, 0.3 M sodium chloride, and 10 mM EDTA, pH 7.2. After loading, the column was washed to baseline with the equilibration buffer followed by an intermediate wash with 5 column volumes of 0.1 M sodium acetate, pH 5.0. Bound antibody was eluted with 10 column volumes of 0.1 M sodium acetate, pH 3.0. The protein A eluate was immediately neutralized with 2 M 2-amino-2-[hydroxymethyl]-1,3-propanediol (Tris). For aggregate removal, the protein A eluate was adjusted to pH 5.5 and applied to a ceramic hydroxyapatite type I (Bio-Rad) column equilibrated with 25 mM sodium phosphate, pH 5.5. After washing the column with 5 column volumes of equilibration buffer, the antibody was eluted in a single step-elution using 0.25 M sodium phosphate, pH 5.5. Fractions containing antibody by $A_{280}$ were pooled and stored at -80°C.

Tissue extract and protein A flow through samples were prepared for SDS-PAGE under reducing and non-reducing conditions by addition of 2x SDS sample buffer ± 5% [v/v] 2-mercaptoethanol. Protein A eluate and hydroxyapatite eluate samples were diluted to a protein concentration of 0.5mg/mL followed by addition of 2x SDS sample buffer ± 5% [v/v] 2-mercaptoethanol . Samples were incubated at 95°C for 2 minutes prior to electrophoresis using 4-20% Tris-Glycine gradient gels (Invitrogen, Carlsbad, CA). Mark12 Molecular weight markers (Invitrogen) and a 5F11 reference

standard were included on the gels. Gels were stained with Colloidal Blue stain.

*Purification of N-linked glycans.*

Protein A purified monoclonal antibodies (1 mg) from wild-type and RNAi *Lemna* plant lines were dialyzed extensively against water and lyophilized to dryness. Samples were resuspended in 100 μL of 5% (v/v) formic acid, brought to 0.05 mg/ml pepsin, and incubated at 37°C overnight. The samples were heat inactivated at 95°C for 15 min and dried. Pepsin digests were resuspended in 100 μL of 100 mM sodium acetate, pH 5.0 and incubated with 1 mU of *N*-glycosidase A at 37°C overnight. The released N-glycans were isolated using 4 cc Carbograph SPE columns and dried.

Dried *N*-glycans were further purified using 1 cc Waters Oasis MCX cartridges. Columns were prepared by washing with 3 column volumes of methanol followed by 3 column volumes of 5% (v/v) formic acid. *N*-glycans, resuspended in 1 mL of 5% (v/v) formic acid, were loaded onto the prepared columns. The unbound fraction as well as 2 additional column volume washes of 5% (v/v) formic acid were collected, pooled, and dried.

*Derivatization of oligosaccharides with 2-aminobenzoic acid (2-AA).*

Purified *N*-glycans or maltooligosaccharides were labeled with 2-AA and purified using 1 cc Waters Oasis HLB cartridges according to Anumula and Dhume, 1998[50]. Labeled *N*-glycans and maltooligosaccharides were resuspended in 50 μL of water and analyzed by negative mode MALDI-TOF MS and NP-HPLC-QTOF MS.

*MALDI-TOF Mass Spectrometry.*

MALDI-TOF MS was conducted using a Waters MALDI Micro MX (Millford, MA). Analysis of β-1,2-xylosyltransferase/α-1,3-fucosyltransferase reaction products was conducted by mixing 0.5 μL of each reaction supernatant with 0.5 μL of 10 mg/mL CHCA in 0.05% (v/v) TFA, 50% (v/v) acetonitrile on a target plate. Xylosylated ($[M+H]^+$ = 2192.85 Da) or fucosylated ($[M+H]^+$ = 2206.87 Da) GnGn-dabsyl-peptide products were detected in positive reflectron mode. Ion counts of 200 combined spectra from each sample were normalized against that of β-1,4-galactosylated, GnGn-dabsyl-peptide ($[M+H]^+$= 2222.87 Da) present as a contaminant (<5%) in the original GnGn-dabsyl-peptide mixture from EMD Biosciences.

2-AA labeled *N*-glycans or maltooligosaccharides (0.5 μL) were diluted with water, mixed with 0.5 μL of 10 mg/ml DHB matrix in 70% (v/v) acetonitrile, spotted onto a target plate and analyzed in negative reflectron mode.

*NP-HPLC-Q-TOF MS analysis of 2-AA labeled N-glycans.*

2-AA labeled *N*-glycans or maltooligosaccharides were brought to 80% (v/v) acetonitrile and separated on a Waters 2695 HPLC system fitted with a TSK-Gel Amide-80 (2 mm x 25 cm, 5 μm) column (Tosoh Biosciences, Montgomeryville, PA). 2-AA labeled carbohydrates were detected and analyzed using a Waters 2475 fluorescence detector (230 nm excitation, 425 nm emission) and a Waters Q-TOF API US quadropole-time of flight (QTOF) mass spectrometer fitted on-line with the HPLC system.

Separations were conducted at 0.2 mL/min, 40°C, using 10 mM ammonium acetate, pH 7.3 (solvent A) and 10 mM ammonium acetate, pH 7.3, 80% (v/v) acetonitrile (solvent B). Sample elution was carried out at 0% A isocratic for 5 min, followed by a linear increase to 10% A at 8 min, and a linear increase to 30% A at 48 min. The column was washed with 100% A for 15 min and equilibrated at 0% A for 15 min prior to the next injection.

QTOF analysis was conducted in negative ion mode with source and desolvation temperatures of 100°C and 300°C, respectively, and capillary and cone voltages of 2,100 and 30 V, respectively. Mass spectra shown are the result of combining ≥40 individual scans per labeled *N*-glycan.

*Monosaccharide analysis by HPAEC-PAD.*

mAb samples (200 μg) were subjected to acid hydrolysis using 2 N TFA at 100°C for 3 h. Samples were dried by vacuum centrifugation at ambient temperature and reconstituted in 150 μL water prior to analysis by HPAE-PAD (Dionex). An aliquot (25 μL) of the reconstituted sample was applied to a CarboPac PA10 column (4 x 250 mm) with a pre-column Amino Trap (Dionex). Separation of monosaccharides was accomplished with a mobile phase of 3.5 mM KOH, using an EG40 eluent generator. Monosaccharide peak identity and relative abundance were determined using monosaccharide standards.

*Thermal stability of mAb.*

A MicroCal (Northampton, MA) VP-Capillary differential scanning calorimetry (DSC) instrument was used to determine thermal stability of glycol-optimized and wild-type mAbs. Purified mAb samples were dialyzed in 20 mM $NaH_2PO_4$, pH 7.4, 150 mM NaCl (PBS) overnight. Thermal denaturation data was collected by heating the samples at a concentration of 300 $\mu$g/mL from 35 to 95 °C at a scan rate of 1°C/min using PBS as the reference buffer. The feedback and gain were set to low. The baseline-corrected and normalized data was fit to a non-2-state model using Origin v7.0 software.

*FcR binding activity of mab.*

The experiment was conducted using a BIACORE (Biacore AB, Uppsala, Sweden) instrument using surface plasmon resonance technology. mAbs, 2 $\mu$g/mL, were captured on the antigen coated surface (recombinant human CD30). Several concentrations of both the $Val^{158}$ and $Phe^{158}$ allotypes of FcRγIIIa, starting from 6 $\mu$M, were flowed over the captured antibodies for 3 min. The binding signal as a function of FcRγIIIa was fit to a one-site binding model using GraphPad Prism (v4.01) software to obtain the $K_D$ values. HBS-EP buffer (10 mM HEPES, 0.15 M NaCl, 3 mM EDTA and 0.005% (v/v) P20 at pH 7.4) was used throughout the experiment. Binding of the mAbs to buffer or FcRγIIIa to blank surfaces were used as negative controls.

*Assay for antigen binding affinity.*

CD30-expressing L540 cells (DSMZ Cell Culture Collection # ACC 72) were used as antigen positive cells to assay for binding. Aliquots of 2 x $10^5$ cells/well were incubated for 30 min at 4°C with 100 $\mu$L of primary antibody at the indicated concentrations. Cells were washed twice in PBS with 2% (v/v) fetal bovine serum (FBS) before addition of goat anti-human mAb, FITC-labeled secondary antibody (Jackson ImmunoResearch, West Grove, PA) at 1:500 dilution in 100 $\mu$L/well for 30 min at 4°C. Cells were washed twice in PBS with 2% (v/v) FBS and assayed by flow cytometry using a FACS Calibur instrument (Becton Dickinson, Franklin Lakes, NJ). $EC_{50}$ values of 5F11 CHO, LEX and $LEX^{Opt}$ mAb binding to CD30 on L540 cells were determined from binding curves utilizing GraphPad Prism 3.0 software.

*ADCC assay.*

Human peripheral blood mononuclear effector cells were purified from heparinized whole blood by standard Ficoll-Paque separation. Cells (2x$10^6$) were washed in PBS and sent for genotyping. The remaining effector cells were then resuspended at 1x$10^6$ cells/mL in RPMI 1640 medium containing 10% (v/v) FBS and 50 U/mL of human IL-2 (Research Diagnostics, Concord, MA) and incubated overnight at 37°C. The effector cells were washed once in culture medium and resuspended at 1x$10^7$ cells/mL prior to use. L540 target cells at 1 x $10^6$ cells/mL in RPMI 1640 medium containing 10% (v/v) FBS and 5 mM probenecid were labeled with 20 $\mu$M BATDA (bis(acetoxymethyl)2,2':6',2"-terpyridine-6,6"-dicarboxylate) for 20 min at 37°C. Target cells were washed three times in PBS supplemented with 20 mM HEPES and 5 mM probenecid, resuspended at 1 x $10^5$ cells/mL and added to effector cells in 96-well plates (1x$10^4$ target cells and 5x$10^5$ effector cells/well) at a final target to effector ratio of 1:50. Maximal release was obtained by incubation of target cells in 3% (v/v) Lysol and spontaneous release obtained by incubation in cell culture medium alone. After 1 h incubation at 37°C, 20 $\mu$L of supernatant was harvested from each well and added to wells containing 180 $\mu$L of Europium solution. The reaction was read with a Perkin Elmer Fusion Alpha TRF reader using a 400 $\mu$sec delay and 330/80, 620/10 excitation and emission filters respectively. The counts per second were plotted as a function of antibody concentration and the data was analyzed by non-linear regression, sigmoidal dose response (variable slope) using GraphPad Prism 3.0 software. The percent specific lysis was calculated as: (experimental release - spontaneous release) /(maximal release- spontaneous release) x 100. In all studies, human mAb1 isotype control was included and compared to 5F11 CHO, LEX, and $LEX^{Opt}$ mAbs. Other controls included target and effector cells with no mAb, target cells with no effector cells and target and effector cells in the presence of 3% (v/v) Lysol.

RESULTS

*Expression of 5F11 mAb in the LEX system.*

5F11 (also known as MDX-060) is an anti-CD30 antibody being developed for the treatment of Hodgkins lymphoma and anaplastic large cell lymphoma. Two binary vectors were constructed for the expression of 5F11 in the LEX system. Expression vector MDXA01 contained codon optimized genes encoding heavy (H) and light (L) chains of 5F11 while vector MDXA04 contained genes encoding H and L chains in addition to a chimeric FucT/XylT RNAi gene.

Independent transgenic lines were generated for both the MDXA01 (165 lines) and MDXA04 (195 lines) expression vectors. For simplicity, MDXA01 derived mAbs (wild-type *N*-glycosylation), and MDXA04 derived mAbs (containing the FucT/XylT RNAi construct) will be referred to as 5F11 LEX and 5F11 $LEX^{Opt}$, respectively, in the discussions below.

Transgenic plant lines were first screened for mAb expression with an IgG ELISA. $LEX^{Opt}$ lines with high levels of mAb expression were assayed further for FucT and XylT activity. Transferase activities in the majority of the high expressing 5F11 $LEX^{Opt}$ lines were reduced to levels of the negative control indicating effective silencing in the majority of the assayed lines (Figure 12). 5F11 $LEX^{Opt}$ lines did not exhibit any morphological or growth differences compared to wild-type *Lemna* plants (data not shown). These results suggest that RNAi silencing of the FucT/XylT genes has no effect on plant viability.

Thermal stabilities of the 5F11 CHO, LEX, and $LEX^{Opt}$ mAbs were determined using differential scanning calorimetry (DSC). All three mAbs exhibited similar melting curve kinetics (data not shown) and melting transition point temperatures (Table 4 below), further demonstrating the structural integrity of the *Lemna*-produced 5F11 LEX and $LEX^{Opt}$ mAbs compared to the 5F11 CHO mAb. SDS-PAGE analysis under non-reducing (Figure 13A) and reducing conditions (Figure 13B) showed that mAbs from the 5F11 $LEX^{Opt}$ and 5F11 CHO lines had similar protein profiles.

Table 4. Comparison of the thermal stabilities of 5F11 CHO, 5F11 LEX, and glyco-optimized 5F11 $LEX^{Opt}$ mAbs by differential scanning calorimetry (DSC).

| Antibody | $T_{m1}$ (°C) | $T_{m2}$ (°C) | $T_{m3}$(°C) |
|---|---|---|---|
| **5F11 CHO** | 72 | 75 | 84 |
| **5F11 LEX** | 71 | 75 | 84 |
| **5F11 $LEX^{Opt}$** | 72 | 76 | 84 |

*N-glycan structures of 5F11 CHO, LEX, and $LEX^{Opt}$ mAbs.*

N-glycan oligosaccharides were released from 5F11 CHO, 5F11 LEX, and 5F11 $LEX^{Opt}$ derived mAbs and analyzed by MALDI-TOF and normal phase (NP) HPLC-QTOF MS. Negative reflectron mode MALDI-TOF MS analysis of 2-AA derivatized *N*-glycans from 5F11 CHO lines indicated the presence of four major *N*-glycans with m/z values corresponding to 2-AA labeled $GnGnF^6$ (nomenclature derived from www.proglycan.com), Man5, $GnA_{iso}F^6$, and $AAF^6$ (Figure 14). NP-HPLC separated the $GnA_{iso}F^6$ *N*-glycan into its two isoforms (Gal attached to the α-1,6-Man or α-1,3-Man arm) bringing the total number of major *N*-glycans found on 5F11 CHO to five (Figure 15). MS/MS fragmentation of the peaks was not conducted to confirm the identity of each isoform; however, the higher abundance of the earlier peak suggested that Gal was attached to the α-1,6-Man arm of this *N*-glycan[3,38]. On-line negative mode QTOF MS analysis gave m/z values corresponding to doubly charged $GnGnF^6$, Man5, $GnA_{iso}F^6$ (both isoforms), and $AAF^6$, confirming the MALDI-TOF MS results (Table 5 below). Peak integration of the fluorescent trace revealed that $GnGnF^6$, Man5, $AGnF^6$, $GnAF^6$, and $AAF^6$ constituted 50.8, 2.5, 26.1, 10.7 and 6.8%, respectively, of the total *N*-glycan structures from 5F11 CHO. The remaining 3.1% *of N*-glycans were found to be a mixture of GnGn, $GnM_{iso}F^6$, $GnM_{iso}$, and MM with no structure higher than 1.2% of the total (data not shown).

Table 5. Summary of observed MALDI-TOF and QTOF MS masses of the major 2-AA labeled *N*-glycans from MDXA-060 mAbs produced by CHO cells (CHO), wild-type *Lemna* (LEX) or glyco-optimized *Lemna* lines expressing the RNAi construct ($LEX^{Opt}$).

| ***N*-glycan name[a]** | **Proposed Structure [b]** | **Theoretical m/z** | **Observed MALDI-TOF[c]** | **Observed Q-TOF[c]** | **% Peak Area[c]** |
|---|---|---|---|---|---|
| ***CHO*** | | $[M-H]^-/[M-2H]^{2-}$ | $[M-H]^-$ | $[M-2H]^{2-}$ | |
| $GnGnF^6$-2AA | 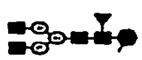 | 1582.590<br>790.7911 | 1582.455 | 790.7825 | 50.8 |
| Man5-2AA | 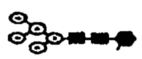 | 1354.479<br>676.7436 | 1354.392 | 676.7343 | 2.50 |
| $GnA_{iso}F^6$-2AA | 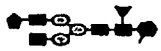<br>or<br>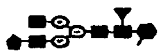 | 1744.642<br>871.8175 | 1744.492 | 871.7970 | 36.8 |

(continued)

| *N*-glycan name[a] | Proposed Structure [b] | Theoretical m/z | Observed MALDI-TOF[c] | Observed Q-TOF[c] | % Peak Area[c] |
|---|---|---|---|---|---|
| AAF[6]-2AA | 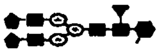 | 1906.695<br>952.8438 | 1906.567 | 952.8181 | 6.80 |
| ***LEX*** | | | | | |
| GnGn-2AA | 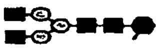 | 1436.532<br>717.7622 | 1436.549 | 717.7894 | 8.40 |
| GnGnX-2AA | 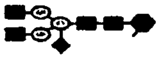 | 1568.574<br>783.7833 | 1568.581 | 783.8150 | 17.2 |
| GnGnXF[3]-2AA | 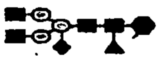 | 1714.632<br>856.8122 | 1714.615 | 856.853 | 67.4 |
| ***LEx[Opt]*** | | | | | |
| GnGn-2AA | 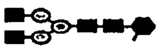 | 1436.532<br>717.7622 | 1436.523 | 717.7993 | 95.8 |

[a]*N*-glycan names are based on Proglycan (www.proglycan.com) nomenclature. 2AA, 2-aminobenzoic acid.

[b]The symbols of the proposed *N*-glycan structures are as follows:■, *N*-acetylglucosamine; 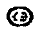 , mannose; 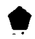 , galactose; ♦, xylosyl; ▲, α-1,3-fucosyl; ▼, α-1,6-fucosyl; ●, 2-aminobenzoic acid.

MALDI-TOF MS analysis of wild-type 5F11 LEX mAb revealed the presence of three major species with m/z values corresponding to $GnGnXF^3$, GnGnX and GnGn (Figure 14). NP-HPLC followed by on-line QTOF MS analysis showed three major fluorescent peaks with m/z values corresponding to doubly charged $GnGnXF^3$, GnGnX and GnGn, again confirming the MALDI-TOF MS results (Figure 15; Table 5). Integration of the fluorescent peaks indicated that $GnGnXF^3$, GnGnX and GnGn constituted 67.4, 17.2 and 8.4%, respectively, of the total *N*-glycans derived from 5F11 LEX mAb's. The remaining 7% of *N*-glycans were determined to be a mixture of MM, $GnM_{iso}$, $MMXF^3$, $GnGnF^3$, $GnM_{iso}XF^3$, Man6, Man7, $Gn(FA)_{iso}XF^3$, Man8 and Man9 with no *N*-glycan greater than 2% of the total (data not shown). Similar results were seen with mAbs isolated from two independently transformed 5F11 LEX lines (data not shown).

In contrast to the 5F11 LEX mAb, *N*-glycans from the 5F11 $LEX^{Opt}$ mAb possessed GnGn as the major *N*-glycan species by both MALD-TOF and NP-HPLC-QTOF MS analysis (Figures 14 and 15; Table 5). GnGn comprised 95.8% of the total *N*-glycans with the remaining 4.2% of *N*-glycans determined to be MM, $GnM_{iso}$, $GnA_{iso}$, Man6, Man7 and Man8 with no one structure greater than 1.2% of the total *N*-glycans. None of the $LEX^{Opt}$ *N*-glycans contained fucosyl (Fuc) or xylosyl (Xyl). These results demonstrated that co-expression of an RNAi construct targeting *Lemna* FucT and XylT resulted in the complete elimination of Fuc and Xyl-containing *N*-glycans from 5F11 $LEX^{Opt}$ mAbs and produced highly homogeneous mAb glycoforms. Similar results were obtained with two independent 5F11 $LEX^{Opt}$ mAbs (data not shown).

The absence of Fuc or Xyl on 5F11 $LEX^{Opt}$ mAb *N*-glycans was further confirmed by monosaccharide analysis (Table 6 below). Monosaccharides were released from 5F11 CHO, LEX and $LEX^{Opt}$ mAbs by acid hydrolysis and analyzed by high performance anion exchange chromatography (HPAEC) coupled to pulsed amperometric detection (PAD). The monosaccharide ratios for Man and GlcNAc residues were similar for CHO and wild-type LEX mAbs and correlated well with expected values. LEX mAbs were significantly decreased in Gal and Fuc content and had a significant increase in Xyl when compared to CHO-derived mAbs. Monosaccharide analysis of *Lemna* derived mAbs revealed that while Fuc and Xyl were present on wild-type LEX *N*-glycans, they were not detected on $LEX^{Opt}$ mAbs. These results confirmed the oligosaccharide profiling data and further suggested that RNAi silencing of *Lemna* XylT and FucT activity changed the *Lemna* *N*-glycosylation pathway to produce mAbs devoid of plant-specific *N*-glycans.

Table 6. Monosaccharides released from 5F11 CHO, LEX and $LEX^{Opt}$ mAbs by acid hydrolysis and analyzed by HPAEC-PAD. The monosaccharide content from each mAb was determined by normalizing against carbohydrate controls.

| Monosaccharide | 5F11 CHO pmol (% total) | 5F11 LEX pmol (% total) | 5F11 $LEX^{Opt}$ pmol (% total) |
|---|---|---|---|
| **Fuc** | 254 (20) | 232 (13) | 0 |
| **GlcNAc** | 605(47) | 773 (45) | 1,003 (67) |

(continued)

| Monosaccharide | 5F11 CHO pmol (% total) | 5F11 LEX pmol (% total) | 5F11 LEX$^{Opt}$ pmol (% total) |
|---|---|---|---|
| Gal | 75(6) | 0 | 0 |
| Man | 355(27) | 491 (29) | 501 (33) |
| Xyl | 0 | 226 (13) | 0 |
| Total | 1,289 (100) | 1,722 (100) | 1,504(100) |

*Functional activity of 5F11 CHO, LEX and LEX$^{Opt}$ mAbs.*

Antigen binding properties of the 5F11 CHO, 5F11 LEX, and 5F11 LEX$^{Opt}$ mAbs were determined using CD30 expressing L540 cells. All three mAbs had nearly identical binding curves (Figure 15). $EC_{50}$ concentrations were determined to be 0.180 μg/mL, 0.227 μg/mL, and 0.196 μg/mL for 5F11 CHO, LEX, and LEX$^{Opt}$, respectively (Figure 16), indicating that antigen binding for all three mAbs were similar.

FcR binding of CHO, LEX and LEX$^{Opt}$ mAbs was determined by incubating mAbs with effector cells expressing two different human FcRγIIIa allotypes (Phe$^{158}$ or Val$^{158}$). 5F11 LEX had a 1.7-fold increase in FcRγIIIaPhe$^{158}$ and a 0.4-fold decrease in FcRγIIIaVal$^{158}$ binding compared to the CHO-derived mAb, demonstrating that receptor binding for CHO and LEX mAbs were similar. In contrast, LEX$^{Opt}$ mAbs had a 27 and 15-fold higher affinity for FcRγIIIaPhe$^{158}$ and FcRγIIIaVal$^{158}$, respectively, than CHO mAbs (Figure 17). These results suggested that RNAi silencing of the *Lemna* FucT and XylT activities in LEX$^{Opt}$ lines produced mAbs with enhanced FcR binding.

ADCC activities of the CHO, LEX and LEX$^{Opt}$ mAbs were determined by incubating mAbs with either homozygous (FcRγIIIaPhe$^{158}$) or heterozygous (FcRγIIIaPhe/Val$^{158}$) human effector cells and BATDA (bis(acetoxymethyl) 2,2':6',2"-terpyridine-6,6' = dicarboxylate) labeled L540 target cells (Figure 17). 5F11 LEX mAbs (31%) had the same maximal percent cell lysis as CHO mAbs (31%) using heterozygous FcRγIIIaPhe/Val$^{158}$ human effector cells (Figure 18) with similar $EC_{50}$ values (0.04210 and 0.05887, respectively). Maximal percent cell lysis for LEX mAbs (27%) was slightly increased compared to CHO mAbs (15%) using homozygous FcγRIIIa Phe/Phe$^{158}$ effector cells, with $EC_{50}$ values of 0.05759 and 0.03368, respectively. Importantly, LEX$^{Opt}$ mAbs had significantly increased ADCC activity compared to 5F11 CHO and LEX mAbs, irrespective of the donor genotype. This was assessed by both an increase in potency and efficacy. Maximal percent lysis for 5F11 Lex$^{Opt}$ was 45% for both experiments. The $EC_{50}$ value of 0.01306 was 3 to 5 times lower than 5F11 LEX and 5F11 CHO mAbs, respectively, for FcγRIIIa Val/Phe$^{158}$ effector cells. The $EC_{50}$ value of 0.01990 was 2 to 3 times lower for the FcγRIIIa Phe/Phe$^{158}$ effector cells. These results demonstrate that removal of Fuc and Xyl-containing *N*-glycans from 5F11 LEX$^{Opt}$ mAbs caused an enhancement in ADCC activity and hence can improve their therapeutic potential.

*RP-HPLC-Q-TOF MS analysis of intact IgG for 5F11 LEX and 5F11 LEX$^{Opt}$*

Protein A purified IgG's (50 μg) were desalted using the Waters 2695 HPLC system fitted with a Poros R1-10 column (2 mm x 30 mm; Applied Biosystems). IgG's were detected and analyzed using a Waters 2487 dual wavelength UV detector (280 nm) and the Waters Q-TOF API US. Separations were conducted at 0.15 mL/min, 60°C, using 0.05% (v/v) trifluoroacetic acid (TFA; solvent A) and 0.05% (v/v)TFA, 80% (v/v) acetonitrile (solvent B). Sample elution was carried out using a linear increase from 30 to 50% B for 5 min, an increase to 80% B for 5 min. The solvent ratio remained at 80% B for an additional 4 min, followed by a wash with 100% B for 1 min and equilibration of the column with 30% B for 15 min prior to the next run.

Q-TOF analysis was conducted in positive ion mode with source and desolvation temperatures of 100°C and 300°C, respectively, and capillary and cone voltages of 3.0 and 60 V, respectively. Data are the result of combining ≥100 individual scans and deconvolution to the parent mass spectrum using MaxEnt 1.

See also Triguero et al. (2005) Plant Biotechnol. J. 3: 449-457; Takahashi et al. (1998) Anal. Biochem. 255: 183-187; Dillon et al. (2004) J. Chromatogr. A. 1053: 299-305.

Figure 19 shows intact mass analysis of the 5F11 LEX mAb compositions produced in wild-type *L. minor* comprising the MDXA01 construct. When XylT and FucT expression are not suppressed in *L. minor,* the recombinantly produced 5F11 LEX mAb composition comprises at least 7 different glycoforms, with the GOXF$^3$ glycoform being the predominate species present. Note the absence of a peak representing the G0 glycoform.

Figure 20 shows glycan mass analysis of the heavy chain of the 5F11 LEX mAb produced in wild-type *L. minor* comprising the MDXA01 construct. When XylT and FucT expression are not suppressed in *L. minor,* the predominate *N*-glycan species present is GOXF$^3$, with additional major peaks reflecting the G0X species. Note the minor presence of the G0 glycan species.

Figure 21 shows intact mass analysis of the 5F11 LEX$^{Opt}$ mAb compositions produced in transgenic *L. minor* comprising the MDXA04 construct. When XylT and FucT expression are suppressed in *L. minor,* the intact mAb composition contains only G0 *N*-glycans. In addition, the composition is substantially homogeneous for the G0 glycoform (peak 2), wherein both glycosylation sites are occupied by the G0 *N*-glycan species, with two minor peaks reflecting trace amounts of precursor glycoforms (peak 1, showing mAb having an Fc region wherein the $C_H2$ domain of one heavy chain has a G0 glycan species attached to Asn 297, and the $C_H2$ domain of the other heavy chain is unglycosylated; and peak 3, showing mAb having an Fc region wherein the Asn 297 glycosylation site on each of the $C_H2$ domains has a G0 glycan species attached, with a third G0 glycan species attached to an additional glycosylation site within the mAb structure).

Figure 22 shows glycan mass analysis of the heavy chain of the 5F11 LEX$^{Opt}$ mAb produced in transgenic *L. minor* comprising the MDXA04 construct. When XylT and FucT expression are suppressed in *L. minor,* the only readily detectable *N*-glycan species attached to the Asn 297 glycosylation sites of the $C_H2$ domains of the heavy chains is G0.

DISCUSSION

Glyco-optimization of 5F11 was accomplished by co-expression with an RNAi cassette aimed at silencing the endogenous *Lemna* FucT and XylT genes. This simultaneous silencing of both FucT and XylT genes was achieved using a single RNAi transcript. The absence of Fuc and Xyl on the LEX$^{Opt}$ mAb was confirmed by MALDI-TOF, NP-HPLC-QTOF MS, and monosaccharide analysis of *N*-glycans purified from the 5F11 LEX$^{Opt}$ mAb. These analyses corroborate the lack of transferase activity observed in microsomal membranes. Importantly, >95% of the *N*-glycans released from LEX$^{Opt}$ mAbs were of a single structure, GnGn, indicating that this strategy had the added benefit of producing mAbs with a homogeneous *N*-glycan profile. 5F11 LEX and LEX$^{Opt}$ mAbs were found to be indistinguishable with regard to thermal stability and antigen binding compared to 5F11 CHO. Electrophoretic analysis was also found to be similar for all three mAbs. In fact, the only structural differences detected were in the mAb *N*-glycan profiles.

Without being bound by theory, the ability of the 5F11 LEX$^{Opt}$ lines to produce mAbs with a single major *N*-glycan species may be based on the more uniform mAb glycoform distribution found in wild-type *Lemna*. *N*-glycans released from mAbs purified from wild-type tobacco, alfalfa, and moss show that mAb glycoform heterogeneity in plants with wild-type *N*-glycosylation can range from five (alfalfa) to eight (tobacco) different major structures. 5F11 LEX possesses only three major *N*-glycan structures (GnGn, GnGnX and GnGnXF). This simple array of *N*-glycans on mAbs produced by wild-type *Lemna* may provide a more amenable starting point for glyco-optimization leading to greater homogeneity than that observed in other systems.

Fc-receptor mediated effector cell function has been shown to be important for the *in vivo* activity of many therapeutic mAbs. In this study, the ADCC activity of 5F11 CHO, 5F11 LEX, and 5F11 LEX$^{Opt}$ mAbs was compared. Since the FcR expressed on NK cells and macrophages responsible for ADCC activity is Fc$\gamma$RIIIa, the binding of the various mAbs to this receptor was also compared. The results discussed above show that 5F11 LEX$^{Opt}$ mAb has an increased binding affinity (15-25 fold) and maximal binding (4-5 fold) to Fc$\gamma$RIIIa as well as enhanced ADCC activity compared to 5F11 CHO and 5F11 LEX mAbs. The removal of $\alpha$-1,6-linked Fuc from various mAbs produced in other expression systems has been shown previously to increase FcR binding and enhance ADCC function. The results presented herein suggest that removal of the $\alpha$-1,3-linked Fuc from the 5F11 LEX$^{Opt}$ mAbs has the same effect on mAb function as the removal of $\alpha$-1,6-linked Fuc.

In this study, two naturally occurring polymorphic isoforms of Fc$\gamma$RIIIa at residue 158[41], Val$^{158}$ and Phe$^{158}$, were evaluated. 5F11 LEX$^{Opt}$ shows a higher binding affinity to Fc$\gamma$RIIIa-Val$^{158}$ compared to Fc$\gamma$RIIIa-Phe$^{158}$ as has been observed with other IgG1 mAbs. The fact that an increase in binding with 5F11 LEX$^{Opt}$ was observed with both isoforms is important since differential binding to Val$^{158}$ over Phe$^{158}$ was found to be predictive of the clinical and immunological responsiveness of certain patient groups receiving anti-CD20 treatment. This increase in binding could lead to more positive clinical outcomes over a broad population base.

A similar increase in ADCC activity was also observed. In this study, the 5F11 LEX$^{Opt}$ mAb showed an increase in cell lysis and a decrease in the $EC_{50}$ value, resulting in an increase in efficacy and potency when compared to 5F11 CHO. This corresponds to a 20-fold increase in activity, determined by taking the maximum percent lysis of 5F11 CHO and calculating the concentration of 5F11 LEX$^{Opt}$ mAb giving rise to the same percent cell lysis. As with the Fc$\gamma$RIIIa binding study, the increase in ADCC activity was observed with both a homozygous Fc$\gamma$RIIIaPhe/Phe$^{158}$ and a heterozygous Fc$\gamma$RIIIa Phe/Val$^{158}$ effector cell donor.

The robustness of this glyco-optimization strategy has been demonstrated with multiple independent *Lemna* plant lines expressing the 5F11 LEX$^{Opt}$ mAb as well as with other mAbs expressed in the *Leman* expression system. Furthermore, there is no apparent difference in plant phenotype or growth rate compared with wild-type *Lemna* plants. Unlike mammalian cell culture systems where *N*-glycan heterogeneity can change with culture conditions, growth scale and growth period, the glycan uniformity observed with LEX$^{Opt}$ mAbs has been shown to be consistent under a variety of growth conditions and scales (data not shown).

In conclusion, an RNAi strategy was used to produce a glyco-optimized anti-CD30 antibody in the *Lemna* expression system. The resulting mAb consists of a single, major *N*-glycan structure; without any evidence of Fuc and Xyl. In addition, the resulting optimized mAb has increased ADCC activity and FcγRIIIa binding activity compared to a CHO-derived mAb. The homogeneous glycosylation profile obtained on mAbs produced in a *Lemna* expression system having this FucT+XylT gene knockout strategy makes it possible to express mAbs with increased production consistency.

SUMMARY OF SEQUENCE LISTING

| SEQ ID NO: | SEQUENCE | SEQ ID NO: | SEQUENCE |
|---|---|---|---|
| 1 | VH a.a. 5F11 | 19 | VK CDR2 a.a. 5F11 |
| 2 | VH a.a. 17G1 | 20 | VK CDR2 aa. 17G1 |
| 3 | VH a.a. 2H9 | 21 | VK CDR2 a.a. 2H9 |
| | | | |
| 4 | VK a.a. 5F11 | 22 | VK CDR3 a.a. 5F11 |
| 5 | VK a.a. 17G1 | 23 | VK CDR3 a.a. 17G1 |
| 6 | VK a.a. 2H9 | 24 | VK CDR3 a.a. 2H9 |
| | | | |
| 7 | VH CDR1 a.a. 5F11 | | |
| 8 | VH CDR1 a.a. 17G1 | 25 | VH 4-34 germline aa |
| 9 | VH CDR1 a.a. 2H9 | 26 | VH 3-07 germline aa |
| | | | |
| 10 | VH CDR2 a.a. 5F11 | 27 | VK L15 germline aa |
| 11 | VH CDR2 a.a. 17G1 | 28 | VK A27 germline aa |
| 12 | VH CDR2 a.a. 2H9 | 29 | VK L6 germline aa |
| | | | |
| 13 | VH CDR3 a.a. 5F11 | 30 | VH n.t. 5F11 |
| 14 | VH CDR3 a.a. 17G1 | 31 | VH n.t. 17G1 |
| 15 | VH CDR3 a.a. 2H9 | 32 | VH n.t. 2H9 |
| | | | |
| 16 | VK CDR1 a.a. 5F11 | 33 | VK n.t. 5F11 |
| 17 | VK CDR1 a.a. 17G1 | 34 | VK n.t. 17G1 |
| 18 | VK CDR1 a.a. 2H9 | 35 | VK n.t. 2H9 |

## Claims

1. An isolated glycosylated anti-CD30 antibody, where at least 90% is a single glycoform, which lacks fucosyl and xylosyl residues.

2. An isolated glycosylated anti-CD30 antibody according to claim 1 which does not contain galactosyl residues.

3. A glycoprotein composition comprising an anti-CD30 antibody composition comprising an N-glycosylation profile wherein at least 90% of the N-glycans species present in said profile are GlcNAc2Man3GlcNAc2 (G0), said profile comprising a trace amount of precursor N-glycan species, wherein said precursor N-glycan species is selected from Man3GlcNAc2, GlcNac1Man3GlcNAc2 wherein GlcNac1 is attached to the 1,3 mannose arm (MGn), GlcNac1Man3GlcNAc2 wherein GlcNac1 is attached to the 1,6 mannose arm (GnM), and any combination thereof.

4. The glycoprotein composition of claim 3, wherein said antibody composition:

(a) comprises an Fc region selected from the group consisting of an IgG1, IgG2, IgG3, and IgG4 region;
(b) is a human antibody;
(c) is a humanized or chimeric antibody; such as a humanized or chimeric antibody prepared from a mouse anti-CD30 antibody selected from AC10, HeFi-1, Ber-H2, Ki-1, Ki-4, HRS-3, Irac, HRS-4, M44, M67 and Ber-H8;
(d) is a monoclonal antibody;
(e) exhibits increased binding affinity for an FcγRIII;
(f) enhances antibody dependent cellular cytotoxicity of cells expressing cell surface CD30; or
(g) exhibits increased macrophage mediated phagocytosis.

**5.** The glycoprotein composition of claim 3 or 4, wherein the antibody composition comprises:

(a) a human heavy chain variable region CDR1 comprising an amino acid sequence selected from SEQ ID NOs: 7, 8, and 9;
(b) a human heavy chain variable region CDR2 comprising an amino acid sequence selected from SEQ ID NOs: 10, 11, and 12;
(c) a human heavy chain variable region CDR3 comprising an amino acid sequence selected from SEQ ID NOs: 13, 14, and 15;
(d) a human light chain variable region CDR1 comprising an amino acid sequence selected from SEQ ID NOs: 16, 17, and 18;
(e) a human light chain variable region CDR2 comprising an amino acid sequence selected from SEQ ID NOs: 19, 20, and 21; and
(f) a human light chain variable region CDR3 comprising an amino acid sequence selected from SEQ ID NOs: 22, 23, and 24.

**6.** The glycoprotein composition of claim 5, wherein the antibody composition comprises:

(a) a human heavy chain variable region CDR1 comprising SEQ ID NO:7, a human heavy chain variable region CDR2 comprising SEQ ID NO:10, a human heavy chain variable region CDR3 comprising SEQ ID NO:13, a human light chain variable region CDR1 comprising SEQ ID NO:16, a human light chain variable region CDR2 comprising SEQ ID NO:19 and a human light chain variable region CDR3 comprising SEQ ID NO:22;
(b) a human heavy chain variable region CDR1 comprising SEQ ID NO:8, a human heavy chain variable region CDR2 comprising SEQ ID NO:11, a human heavy chain variable region CDR3 comprising SEQ ID NO:14, a human light chain variable region CDR1 comprising SEQ ID NO:17, a human light chain variable region CDR2 comprising SEQ ID NO:20 and a human light chain variable region CDR3 comprising SEQ ID NO:23;
(c) a human heavy chain variable region CDR1 comprising SEQ ID NO:9, a human heavy chain variable region CDR2 comprising SEQ ID NO:12, a human heavy chain variable region CDR3 comprising SEQ ID NO:15, a human light chain variable region CDR1 comprising SEQ ID NO:18, a human light chain variable region CDR2 comprising SEQ ID NO:21 and a human light chain variable region CDR3 comprising SEQ ID NO:24; or (d) a human heavy chain variable region comprising an amino acid sequence selected from SEQ ID NOs: 1, 2 and 3 and a human light chain variable region comprising an amino acid sequence selected from SEQ ID NOs: 4, 5 and 6.

**7.** The glycoprotein composition of claim 6, wherein:

(a) the antibody composition heavy chain variable region comprises the amino acid sequence of SEQ ID NO: 1 and the light chain variable region comprises the amino acid sequence of SEQ ID NO: 4;
(b) the antibody composition heavy chain variable region comprises the amino acid sequence of SEQ ID NO: 2 and the light chain variable region comprises the amino acid sequence of SEQ ID NO: 5; or
(c) the antibody composition heavy chain variable region comprises the amino acid sequence of SEQ ID NO: 3 and the light chain variable region comprises the amino acid sequence of SEQ ID NO: 6.

**8.** The glycoprotein composition of claim 4, wherein the antibody composition comprises a heavy chain variable region that is a product of or derived from a human $V_H$ 4-34 or $V_H$ 3-07 gene and/or a light chain variable region that is a product of or derived from a human $V_k$ L15, A27 or L6 gene.

**9.** A pharmaceutical composition comprising the antibody of claim 1 or 2 or the glycoprotein composition of any one of claims 3 to 8.

10. A host cell comprising the glycoprotein composition of claim 4, wherein the host cell is optionally a plant host cell such as a duckweed cell.

11. A host cell comprising immunoglobulin heavy and light chain genes encoding an anti-CD30 antibody, wherein said host cell lacks a fucosyltransferase and a xylosyltransferase such that the anti-CD30 antibody expressed by said host cell lacks fucosyl and xylosyl residues, wherein the host cell is optionally a plant cell, wherein the plant cell is optionally a member of the *Lemnaceae* family, such as *Lemna minor.*

12. The host cell of claim 11, wherein:

(a) the immunoglobulin heavy and light chain genes are human immunoglobulin heavy and light chain genes; or
(b) said fucosyltransferase is FucT and said xylosyltransferase is XylT.

13. An *in vitro* method of inhibiting growth of $CD30^+$ cells such as tumor cells comprising contacting said cells with an anti-CD30 antibody, where at least 90% is a single glycoform, which lacks fucosyl and xylosyl residues, under conditions sufficient to induce antibody-dependent cellular cytoxicity (ADCC) of said cells.

14. An anti-CD30 antibody, where at least 90% is a single glycoform, which lacks fucosyl and xylosyl residues, for use in a method of inhibiting growth of tumor cells expressing CD30 or in a method of treating an autoimmune disorder.

15. The method of claim 13 or the antibody of claim 14, wherein said anti-CD30 antibody is a human antibody.

16. The antibody of claim 14 or 15, wherein the tumor cells are of from a disease selected from Hodgkin's Disease (HD), anaplastic large-cell lymphoma (ALCL), non-Hodgkin's lymphoma, Burkitt's lymphoma, cutaneous T-cell lymphomas, nodular small cleaved-cell lymphomas, lymphocytic lymphomas, peripheral T-cell lymphomas, Lennert's lymphomas, immunoblastic lymphomas, T-cell leukemia/lymphomas (ATLL), adult T-cell leukemia (T-ALL), entroblastic/centrocytic (cb/cc) follicular lymphomas cancers, diffuse large cell lymphomas of B lineage, angioimmunoblastic lymphadenopathy (AILD)-like T cell lymphoma, adult T-cell lymphoma (ATL), HIV associated body cavity based lymphomas, Embryonal Carcinomas, undifferentiated carcinomas of the rhinopharynx (e.g., Schmincke's tumor), Castleman's disease, Kaposi's Sarcoma, CD30+ T-cell lymphomas and CD30+ B-cell lymphomas.

**Patentansprüche**

1. Isolierter glykosylierter Anti-CD30-Antikörper, wobei wenigstens 90% eine einzelne Glykoform sind, dem Fucosyl- und Xylosylreste fehlen.

2. Isolierter glykosylierter Anti-CD30-Antikörper gemäß Anspruch 1, der keine Galaktosylreste enthält.

3. Glykoprotein-Zusammensetzung, umfassend eine Anti-CD30-Antikörper-Zusammensetzung, umfassend ein N-Glykosylierungsprofil, bei dem wenigstens 90% der N-Glykan-Spezies, die in dem Profil vorliegen, GlcNAc2Man3GlcNAc2 (G0) sind, wobei das Profil eine Spurenmenge von Vorläufer-N-Glykan-Spezies umfasst, wobei die Vorläufer-N-Glykan-Spezies aus Man3GlcNAc2, GlcNac1Man3GlcNAc2, worin GlcNac1 an den 1,3-Mannosearm (MGn) gebunden ist, GlcNac1Man3GlcNAc2, worin GlcNac1 an den 1,6-Mannosearm (GnM) gebunden ist, und einer beliebigen Kombination davon ausgewählt ist.

4. Glykoprotein-Zusammensetzung gemäß Anspruch 3, wobei die Antikörperzusammensetzung:

(a) eine FC-Region, ausgewählt aus der Gruppe, bestehend aus einer IgG1-, IgG2-, IgG3- und IgG4-Region, umfasst;
(b) ein humaner Antikörper ist;
(c) ein humanisierter oder chimärer Antikörper ist; zum Beispiel ein humanisierter oder chimärer Antikörper, hergestellt aus einem Maus-Anti-CD30-Antikörper, ausgewählt aus AC10, HeFi-1, Ber-H2, Ki-1, Ki-4, HRS-3, Irac, HRS-4, M44, M67 und Ber-H8;
(d) ein monoklonaler Antikörper ist;
(e) verstärkte Bindungsaffinität für ein FcγRIII aufweist;
(f) Antikörper-abhängige zelluläre Cytotoxizität von Zellen, die Zelloberflächen-CD30 exprimieren, verstärkt oder
(g) verstärkte Makrophagen-vermittelte Phagozytose aufweist.

**5.** Glykoprotein-Zusammensetzung gemäß Anspruch 3 oder 4, wobei die Antikörperzusammensetzung umfasst:

(a) eine CDR1 der variablen Region der humanen schweren Kette, umfassend eine Aminosäuresequenz, ausgewählt aus SEQ ID NOs: 7, 8 und 9;
(b) eine CDR2 der variablen Region der humanen schweren Kette, umfassend eine Aminosäuresequenz, ausgewählt aus SEQ ID NOs: 10, 11 und 12;
(c) eine CDR3 der variablen Region der humanen schweren Kette, umfassend eine Aminosäuresequenz, ausgewählt aus SEQ ID NOs: 13, 14 und 15;
(d) eine CDR1 der variablen Region der humanen leichten Kette, umfassend eine Aminosäuresequenz, ausgewählt aus SEQ ID NOs: 16, 17 und 18;
(e) eine CDR2 der variablen Region der humanen leichten Kette, umfassend eine Aminosäuresequenz, ausgewählt aus SEQ ID NOs: 19, 20 und 21, und
(f) eine CDR3 der variablen Region der humanen leichten Kette, umfassend eine Aminosäuresequenz, ausgewählt aus SEQ ID NOs: 22, 23 und 24.

**6.** Glykoprotein-Zusammensetzung gemäß Anspruch 5, wobei die Antikörperzusammensetzung umfasst:

(a) eine CDR1 der variablen Region der humanen schweren Kette, umfassend SEQ ID NO:7,
eine CDR2 der variablen Region der humanen schweren Kette, umfassend SEQ ID NO:10,
eine CDR3 der variablen Region der humanen schweren Kette, umfassend SEQ ID NO:13,
eine CDR1 der variablen Region der humanen leichten Kette, umfassend SEQ ID NO:16,
eine CDR2 der variablen Region der humanen leichten Kette, umfassend SEQ ID NO:19 und
eine CDR3 der variablen Region der humanen leichten Kette, umfassend SEQ ID NO:22;,
(b) eine CDR1 der variablen Region der humanen schweren Kette, umfassend SEQ ID NO:8,
eine CDR2 der variablen Region der humanen schweren Kette, umfassend SEQ ID NO:11,
eine CDR3 der variablen Region der humanen schweren Kette, umfassend SEQ ID NO:14,
eine CDR1 der variablen Region der humanen leichten Kette, umfassend SEQ ID NO:17,
eine CDR2 der variablen Region der humanen leichten Kette, umfassend SEQ ID NO:20 und
eine CDR3 der variablen Region der humanen leichten Kette, umfassend SEQ ID NO:23;,
(c) eine CDR1 der variablen Region der humanen schweren Kette, umfassend SEQ ID NO:9,
eine CDR2 der variablen Region der humanen schweren Kette, umfassend SEQ ID NO:12,
eine CDR3 der variablen Region der humanen schweren Kette, umfassend SEQ ID NO:15,
eine CDR1 der variablen Region der humanen leichten Kette, umfassend SEQ ID NO:18,
eine CDR2 der variablen Region der humanen leichten Kette, umfassend SEQ ID NO:21 und
eine CDR3 der variablen Region der humanen leichten Kette, umfassend SEQ ID NO:24, oder
(d) die variable Region der humanen schweren Kette, umfassend eine Aminosäuresequenz, ausgewählt aus SEQ ID NOs: 1, 2 und 3, und die variable Region der humanen leichten Kette, umfassend eine Aminosäuresequenz, ausgewählt aus SEQ ID NOs: 4, 5 und 6.

**7.** Glykoprotein-Zusammensetzung gemäß Anspruch 6, in der:

(a) die variable Region der schweren Kette der Antikörperzusammensetzung die Aminosäuresequenz von SEQ ID NO:1 umfasst und die variable Region der leichten Kette die Aminosäuresequenz von SEQ ID NO:4 umfasst;
(b) die variable Region der schweren Kette der Antikörperzusammensetzung die Aminosäuresequenz von SEQ ID NO:2 umfasst und die variable Region der leichten Kette die Aminosäuresequenz von SEQ ID NO:5 umfasst oder
(c) die variable Region der schweren Kette der Antikörperzusammensetzung die Aminosäuresequenz von SEQ ID NO:3 umfasst und die variable Region der leichten Kette die Aminosäuresequenz von SEQ ID NO:6 umfasst.

**8.** Glykoprotein-Zusammensetzung gemäß Anspruch 4, wobei die Antikörperzusammensetzung die variable Region einer schweren Kette, die ein Produkt von einem humanen $V_H$-4-34- oder $V_H$-3-07-Gen ist oder davon abgeleitet ist, und/oder die variable Region einer leichten Kette, die ein Produkt eines humanen $V_k$L15-, -A27 oder -L6-Gens ist oder davon abgeleitet ist, umfasst.

**9.** Pharmazeutische Zusammensetzung, die den Antikörper gemäß Anspruch 1 oder 2 oder die Glykoprotein-Zusammensetzung gemäß einem der Ansprüche 3 bis 8 umfasst.

**10.** Wirtszelle, die die Glykoprotein-Zusammensetzung gemäß Anspruch 4 umfasst, wobei die Wirtszelle gegebenenfalls

eine Pflanzenwirtszelle, zum Beispiel eine Wasserlinsenzelle ist.

**11.** Wirtszelle, die Gene der schweren und leichten Immunglobulinkette umfasst, die einen Anti-CD30-Antikörper codieren, wobei der Wirtszelle eine Fucosyltransferase und eine Xylosyltransferase fehlt, sodass dem Anti-CD30-Antikörper, der durch die Wirtszelle exprimiert wird, Fucosyl- und Xylosylreste fehlen, wobei die Wirtszelle gegebenenfalls eine Pflanzenzelle ist, wobei die Pflanzenzelle gegebenenfalls ein Mitglied der Lemnaceae-Familie ist, zum Beispiel *Lemna minor.*

**12.** Wirtszelle gemäß Anspruch 11, wobei:

(a) die Gene der schweren und leichten Immunglobulinkette Gene der humanen schweren und leichten Immunglobulinkette sind, oder
(b) die Fucosyltransferase FucT ist und die Xylosyltransferase XylT ist.

**13.** In vitro-Verfahren zur Inhibierung des Wachstums von $CD30^+$-Zellen, zum Beispiel Tumorzellen, das ein in Kontakt bringen der Zellen mit einem Anti-CD30-Antikörper umfasst, in dem wenigstens 90% eine einzelne Glykoform sind, dem Fucosyl- und Xylosylreste fehlen, unter Bedingungen, die ausreichen, um eine Antikörper-abhängige zelluläre Toxizität (ADCC) der Zellen zu induzieren.

**14.** Anti-CD30-Antikörper, in dem wenigstens 90% eine einzelne Glykoform sind, dem Fucosyl- und Xylosylreste fehlen, zur Verwendung in einem Verfahren zur Inhibierung des Wachstums von Tumorzellen, die CD30 exprimieren, oder in einem Verfahren zum Behandeln einer Autoimmunstörung.

**15.** Verfahren gemäß Anspruch 13 oder Antikörper gemäß Anspruch 14, wobei der Anti-CD30-Antikörper ein humaner Antikörper ist.

**16.** Antikörper gemäß Anspruch 14 oder 15, wobei die Tumorzellen aus einer Krankheit stammen, die ausgewählt ist aus Hodgkinscher Krankheit (HD), anaplastischem großzelligem Lymphom (ALCL), Nicht-Hodgkin-Lymphom, Burkitts Lymphom, kutanen T-Zell-Lymphomen, nodulären, gespaltenen klein-zelligen Lymphomen, lymphozytischen Lymphomen, peripheren T-Zell-Lymphomen, Lennerts Lymphomen, immunoblastischen Lymphomen, T-Zell-Leukämie/Lymphomen (ATLL), Erwachsenen-T-Zell-Leukämie (T-ALL), enteroblastischen/zentro-zytischen (cb/cc) follikulären Lymphom-Krebsarten, diffusen großzelligen Lymphomen der B-Lineage, angioimmunoblastischer Lymphadenopathie (AILD)-artigem T-Zell-Lymphom, Erwachsenen-T-Zell-Lymphom (ATL), mit HIV-assoziierten Lymphomen basierend auf einer Körperhöhle, embryonalen Karzinomen, undifferenzierten Karzinomen des Rhinopharynx (z.B. Schminckes Tumor), Castelmans Krankheit, Kaposi-Sarcom, CD30+-T-Zell-Lymphomen und CD30+-B-Zell-Lymphomen.

## Revendications

**1.** Anticorps anti-CD30 glycosylé isolé, dont une fraction d'au moins 90 % représente une glycoforme unique et qui est dépourvu de résidus fucosyle et xylosyle.

**2.** Anticorps anti-CD30 glycosylé isolé, conforme à la revendication 1, qui ne contient pas de résidus galactosyle.

**3.** Composition de glycoprotéine comprenant une composition d'anticorps anti-CD30 dotée d'un profil de N-glycosylation dans lequel au moins 90 % des espèces de type N-glycanne présentes dans ledit profil sont des espèces $GlcNAc_2Man_3GlcNAc_2$ (G0), lequel profil comprend des traces d'une espèce précurseur de type N-glycanne, laquelle espèce précurseur de type N-glycanne est choisie parmi $Man_3GlcNAc_2$, $GlcNac_1Man_3GlcNAc_2$ dont le résidu $GlcNac_1$ est attaché à la branche Man-1,3-Man (MGn), et $GlcNac_1Man_3GlcNAc_2$ dont le résidu $GlcNac_1$ est attaché à la branche Man-1,6-Man (GnM), et toute combinaison de ces espèces.

**4.** Composition de glycoprotéine, conforme à la revendication 3, dans laquelle ladite composition d'anticorps :

a) comprend un fragment Fc choisi dans l'ensemble constitué par les fragments correspondants d'IgG1, d'IgG2, d'IgG3 et d'IgG4 ;
b) ou est un anticorps humain ;
c) ou est un anticorps humanisé ou chimérique, comme un anticorps humanisé ou chimérique préparé à partir

d'un anticorps anti-CD30 de souris choisi parmi les anticorps AC10, HeFi-1, Ber-H2, Ki-1, Ki-4, HRS-3, Irac, HRS-4, M44, M67 et Ber-H8 ;
d) ou est un anticorps monoclonal ;
e) ou a une affinité de liaison accrue pour un fragment FcγRIII ;
f) ou renforce la cytotoxicité cellulaire dépendante des anticorps vis-à-vis des cellules exprimant CD30 à leur surface ;
g) ou renforce la phagocytose par macrophages.

**5.** Composition de glycoprotéine conforme à la revendication 3 ou 4, dans laquelle la composition d'anticorps comprend :

a) une région variable CDR1 de chaîne lourde humaine, comprenant une séquence d'acides aminés choisie parmi les Séquences N° 7, N° 8 et N° 9 ;
b) une région variable CDR2 de chaîne lourde humaine, comprenant une séquence d'acides aminés choisie parmi les Séquences N° 10, N° 11 et N° 12 ;
c) une région variable CDR3 de chaîne lourde humaine, comprenant une séquence d'acides aminés choisie parmi les Séquences N° 13, N° 14 et N° 15 ;
d) une région variable CDR1 de chaîne légère humaine, comprenant une séquence d'acides aminés choisie parmi les Séquences N° 16, N° 17 et N° 18 ;
e) une région variable CDR2 de chaîne légère humaine, comprenant une séquence d'acides aminés choisie parmi les Séquences N° 19, N° 20 et N° 21 ;
f) une région variable CDR3 de chaîne légère humaine, comprenant une séquence d'acides aminés choisie parmi les Séquences N° 22, N° 23 et N° 24 ;

**6.** Composition de glycoprotéine conforme à la revendication 5, dans laquelle la composition d'anticorps comprend :

a) une région variable CDR1 de chaîne lourde humaine comprenant la Séquence N° 7, une région variable CDR2 de chaîne lourde humaine comprenant la Séquence N° 10, une région variable CDR3 de chaîne lourde humaine comprenant la Séquence N° 13, une région variable CDR1 de chaîne légère humaine comprenant la Séquence N° 16, une région variable CDR2 de chaîne légère humaine comprenant la Séquence N° 19, et une région variable CDR3 de chaîne légère humaine comprenant la Séquence N° 22 ;
b) une région variable CDR1 de chaîne lourde humaine comprenant la Séquence N° 8, une région variable CDR2 de chaîne lourde humaine comprenant la Séquence N° 11, une région variable CDR3 de chaîne lourde humaine comprenant la Séquence N° 14, une région variable CDR1 de chaîne légère humaine comprenant la Séquence N° 17, une région variable CDR2 de chaîne légère humaine comprenant la Séquence N° 20, et une région variable CDR3 de chaîne légère humaine comprenant la Séquence N° 23 ;
c) une région variable CDR1 de chaîne lourde humaine comprenant la Séquence N° 9, une région variable CDR2 de chaîne lourde humaine comprenant la Séquence N° 12, une région variable CDR3 de chaîne lourde humaine comprenant la Séquence N° 15, une région variable CDR1 de chaîne légère humaine comprenant la Séquence N° 18, une région variable CDR2 de chaîne légère humaine comprenant la Séquence N° 21, et une région variable CDR3 de chaîne légère humaine comprenant la Séquence N° 24 ;
d) ou une région variable de chaîne lourde humaine comprenant une séquence d'acides aminés choisie parmi les Séquences N° 1, N° 2 et N° 3, et une région variable de chaîne légère humaine comprenant une séquence d'acides aminés choisie parmi les Séquences N° 4, N° 5 et N° 6.

**7.** Composition de glycoprotéine conforme à la revendication 6, dans laquelle

a) la région variable de chaîne lourde de la composition d'anticorps comprend la séquence d'acides aminés de la Séquence N° 1, et la région variable de chaîne légère comprend la séquence d'acides aminés de la Séquence N° 4 ;
b) la région variable de chaîne lourde de la composition d'anticorps comprend la séquence d'acides aminés de la Séquence N° 2, et la région variable de chaîne légère comprend la séquence d'acides aminés de la Séquence N° 5 ;
c) la région variable de chaîne lourde de la composition d'anticorps comprend la séquence d'acides aminés de la Séquence N° 3, et la région variable de chaîne légère comprend la séquence d'acides aminés de la Séquence N° 6.

**8.** Composition de glycoprotéine conforme à la revendication 4, dans laquelle la composition d'anticorps comprend

une région variable de chaîne lourde qui est un produit ou qui dérive d'un gène humain $V_H$ 4-34 ou $V_H$ 3-07, et/ou une région variable de chaîne légère qui est un produit ou qui dérive d'un gène humain $V_k$ L15, A27 ou L6.

**9.** Composition pharmaceutique comprenant un anticorps conforme à la revendication 1 ou 2 ou une composition de glycoprotéine conforme à l'une des revendications 3 à 8.

**10.** Cellule hôte comprenant une composition de glycoprotéine conforme à la revendication 4, laquelle cellule hôte, en option, est une cellule hôte végétale comme une cellule de lentille d'eau.

**11.** Cellule hôte comprenant des gènes de chaîne lourde et de chaîne légère d'immunoglobuline qui codent un anticorps anti-CD30, laquelle cellule hôte est dépourvue de fucosyl-transférase et de xylosyl-transférase, de telle sorte que l'anticorps anti-CD30 exprimé par ladite cellule hôte est dépourvu de résidus fucosyle et xylosyle, et laquelle cellule hôte, en option, est une cellule végétale qui, en option, provient d'un membre de la famille des Lemnacées, tel que *Lemna minor.*

**12.** Cellule hôte conforme à la revendication 11, dans laquelle :

a) les gènes de chaîne lourde et de chaîne légère d'immunoglobuline sont des gènes humains de chaîne lourde et de chaîne légère d'immunoglobuline ;
b) ou ladite fucosyl-transférase est l'enzyme FucT et ladite xylosyl-transférase est l'enzyme XylT.

**13.** Procédé *in vitro* d'inhibition de la prolifération de cellules CD30+, telles que des cellules tumorales, comprenant le fait de mettre lesdites cellules en contact avec un anticorps anti-CD30 dont une fraction d'au moins 90 % représente une glycoforme unique et qui est dépourvu de résidus fucosyle et xylosyle, dans des conditions suffisantes pour induire vis-à-vis desdites cellules une cytotoxicité cellulaire dépendante des anticorps (ADCC).

**14.** Anticorps anti-CD30 glycosylé isolé, dont une fraction d'au moins 90 % représente une glycoforme unique et qui est dépourvu de résidus fucosyle et xylosyle, pour utilisation dans un procédé d'inhibition de la prolifération de cellules tumorales exprimant CD30 ou dans un procédé de traitement d'une affection auto-immune.

**15.** Procédé conforme à la revendication 13, ou anticorps conforme à la revendication 14, dans lequel ledit anticorps anti-CD30 est un anticorps humain.

**16.** Anticorps conforme à la revendication 14 ou 15, pour lequel les cellules tumorales proviennent d'un cas d'une maladie choisie parmi les suivantes : maladie de Hodgkin (HD), lymphome anaplasique à grandes cellules (ALCL), lymphome non hodgkinien, lymphome de Burkitt, lymphomes cutanés à cellules T, lymphomes nodulaires à petites cellules clivées, lymphomes lymphoïdes, lymphomes périphériques à cellules T, lymphomes de Lennert, lymphomes immunoblastiques, lymphome/leucémie à cellules T de l'adulte (ATLL), leucémie aigüe lymphoblastique à cellules T (T-ALL), cancers lymphomes folliculaires centroblastiques-centrocytiques (cb/cc), lymphomes diffus à grandes cellules B, lymphome à cellules T de type lymphadénopathie angio-immunoblastique (aILD), lymphome à cellules T de l'adulte (ATL), lymphomes des cavités corporelles associés au VIH, carcinomes embryonnaires, carcinomes indifférenciés du rhinopharynx (par exemple, tumeur de Schmincke), maladie de Castleman, sarcome de Kaposi, lymphomes à cellules T CD30+ et lymphomes à cellules B CD30+.

Anti-CD30 5F11 VH
V-segment: Locus: 4-34
D segment: Locus - 7-27
J segment: JH4b

```
       Q   V   Q   L   Q   Q   W   G   A   G   L   L   K   P   S   E   T   L
1     CAG GTG CAG CTA CAG CAG TGG GGC GCA GGA CTG TTG AAG CCT TCG GAG ACC CTG

                                                            CDR1
                                                   ~~~~~~~~~~~~~~~~~~~~
       S   L   T   C   A   V   Y   G   G   S   F   S   A   Y   Y   W   S   W
55    TCC CTC ACC TGC GCT GTC TAT GGT GGG TCC TTC AGT GCT TAC TAC TGG AGC TGG

                                                                CDR2
                                                       ~~~~~~~~~~~~~~~~~~~~
       I   R   Q   P   P   G   K   G   L   E   W   I   G   D   I   N   H   G
109   ATC CGC CAG CCC CCA GGG AAG GGG CTG GAG TGG ATT GGG GAC ATC AAT CAT GGT

                 CDR2
      ~~~~~~~~~~~~~~~~~~~~~~~~~~~~~~~~~~~~~~~~~~~~~
       G   G   T   N   Y   N   P   S   L   K   S   R   V   T   I   S   V   D
163   GGA GGC ACC AAC TAC AAC CCG TCC CTC AAG AGT CGA GTC ACC ATA TCA GTA GAC

       T   S   K   N   Q   F   S   L   K   L   N   S   V   T   A   A   D   T
217   ACG TCC AAG AAC CAG TTC TCC CTG AAG CTG AAC TCT GTA ACC GCC GCG GAC ACG

                                         CDR3
                                  ~~~~~~~~~~~~~~~~
       A   V   Y   Y   C   A   S   L   T   A   Y   W   G   Q   G   S   L   V
271   GCT GTG TAT TAC TGT GCG AGC CTA ACT GCC TAC TGG GGC CAG GGA AGC CTG GTC
                                 D7-27/DHQ52  └→ JH4b

       T   V   S   S
325   ACC GTC TCC TCA
```

*Fig. 1A*

```
Anti-CD30 5F11 VL
     V-segment:       Locus: L15
     J segment:       JK5

        D   I   Q   M   T   Q   S   P   T   S   L   S   A   S   V   G   D   R
  1    GAC ATC CAG ATG ACC CAG TCT CCA ACC TCA CTG TCT GCA TCT GTA GGA GAC AGA

                                          CDR1
                               ~~~~~~~~~~~~~~~~~~~~~~~~~~~~~~~~~~~~~~~~~~~~~
        V   T   I   T   C   R   A   S   Q   G   I   S   S   W   L   T   W   Y
 55    GTC ACC ATC ACT TGT CGG GCG AGT CAG GGT ATT AGC AGC TGG TTA ACC TGG TAT

                                                                CDR2
                                                           ~~~~~~~~~~~~~~~~~~~
        Q   Q   K   P   E   K   A   P   K   S   L   I   Y   A   A   S   S   L
109    CAG CAG AAA CCA GAG AAA GCC CCT AAG TCC CTG ATC TAT GCT GCA TCC AGT TTG

        CDR2
       ~~~~~~~~
        Q   S   G   V   P   S   R   F   S   G   S   G   S   G   T   D   F   T
163    CAA AGT GGG GTC CCA TCA AGG TTC AGC GGC AGT GGA TCT GGG ACA GAT TTC ACT

                                                                       CDR3
                                                                     ~~~~~~~
        L   T   I   S   S   L   Q   P   E   D   F   A   T   Y   Y   C   Q   Q
217    CTC ACC ATC AGC AGC CTG CAG CCT GAA GAT TTT GCA ACT TAT TAC TGC CAA CAG

             CDR3
       ~~~~~~~~~~~~~~~~~~~~~~~~~~~~~
        Y   D   S   Y   P   I   T   F   G   Q   G   T   R   L   E   I   K
271    TAT GAT AGT TAC CCT ATC ACC TTC GGC CAA GGG ACA CGA CTG GAG ATT AAA
                            └→ JK5
```

*Fig. 1B*

```
Anti-CD30 17G1 VH
     V-segment:        Locus: 3-07
     D segment:        Not Found
     J segment:        JH2

      E   V   Q   L   V   E   S   G   G   G   L   V   Q   P   G   G   S   L
  1   GAG GTG CAG TTG GTG GAG TCT GGG GGA GGC TTG GTC CAG CCT GGG GGG TCC CTG

                                                          CDR1
                                                  ~~~~~~~~~~~~~~~~~~~~
      R   L   S   C   V   A   S   G   F   T   F   S   N   S   W   M   S   W
 55   AGA CTC TCC TGT GTA GCC TCT GGA TTC ACC TTT AGT AAC TCT TGG ATG AGC TGG

                                                              CDR2
                                                      ~~~~~~~~~~~~~~~~~~~~
      V   R   Q   A   P   G   K   G   L   E   W   V   A   N   I   N   E   D
109   GTC CGC CAG GCT CCA GGG AAA GGG CTG GAG TGG GTG GCC AAC ATA AAC GAA GAT

                  CDR2
      ~~~~~~~~~~~~~~~~~~~~~~~~~~~~~~~~~~~~~~~~~~~~~~~
      G   S   E   K   F   Y   V   D   S   V   K   G   R   F   T   F   S   R
163   GGA AGT GAG AAA TTC TAT GTG GAC TCT GTG AAG GGC CGA TTC ACC TTC TCC AGA

      D   N   A   E   N   S   L   Y   L   Q   M   N   S   L   R   A   E   D
217   GAC AAC GCC GAG AAC TCA CTG TAT CTG CAA ATG AAC AGC CTG AGA GCC GAG GAC

                                             CDR3
                                      ~~~~~~~~~~~~~~~~~~~~~~~~~~
      T   A   V   Y   Y   C   A   R   V   H   W   Y   F   H   L   W   G   R
271   ACG GCT GTG TAT TAC TGT GCG AGG GTT CAT TGG TAC TTC CAT CTC TGG GGC CGT
                                            |
                                            └─► JH2
      G   T   L   V   T   V   S   S
325   GGC ACC CTG GTC ACT GTC TCC TCA
```

*Fig. 2A*

```
Anti-CD30 17G1 VL
     V-segment:      Locus: A27
     J segment:      JK1

      E   I   V   L   T   Q   S   P   G   T   L   S   L   S   P   G   E   R
  1  GAA ATT GTG TTG ACG CAG TCT CCA GGC ACC CTG TCT TTG TCT CCA GGG GAA AGA

                                                CDR1
                                ~~~~~~~~~~~~~~~~~~~~~~~~~~~~~~~~~~~~~~~~~~~~~~~~~~~~
      A   T   L   S   C   R   A   S   Q   S   V   S   S   S   Y   L   A   W
 55  GCC ACC CTC TCC TGC AGG GCC AGT CAG AGT GTT AGC AGC AGC TAC TTA GCC TGG

                                                                   CDR2
                                                              ~~~~~~~~~~~~~~~
      Y   Q   Q   K   P   G   Q   A   P   R   L   L   I   Y   G   A   S   S
109  TAC CAG CAG AAA CCT GGC CAG GCT CCC AGG CTC CTC ATC TAT GGT GCA TCC AGC

       CDR2
     ~~~~~~~~~~~~
      R   A   T   G   I   P   D   R   F   S   G   S   G   S   G   T   D   F
163  AGG GCC ACT GGC ATC CCA GAC AGG TTC AGT GGC AGT GGG TCT GGG ACA GAC TTC

                                                                         CDR3
                                                                          ~~~
      T   L   T   I   S   S   L   E   P   E   D   F   A   V   Y   Y   C   Q
217  ACT CTC ACC ATC AGC AGC CTG GAG CCT GAA GAT TTT GCA GTG TAT TAC TGT CAG

               CDR3
     ~~~~~~~~~~~~~~~~~~~~~~~~~~~~~~
      Q   Y   G   S   S   P   W   T   F   G   Q   G   T   K   V   E   I   K
271  CAG TAT GGT AGC TCA CCG TGG ACG TTC GGC CAA GGG ACC AAG GTG GAA ATC AAA
                          |
                          L-> JK1
```

*Fig. 2B*

```
Anti-2H9 CD30 VH
     V-segment:        Locus: 4-34
     D segment:        Locus - 5-12
     J segment:        JH2

       Q   V   Q   L   Q   Q   W   G   A   G   L   L   K   P   S   E   T   L
  1    CAG GTG CAG CTA CAG CAG TGG GGC GCA GGA CTG TTG AAG CCT TCG GAG ACC CTG

                                                               CDR1
                                                         ~~~~~~~~~~~~~~~~~~~~
       S   L   T   C   A   V   Y   G   G   S   F   S   G   Y   Y   W   S   W
 55    TCC CTC ACC TGC GCT GTC TAT GGT GGG TCC TTC AGT GGT TAC TAC TGG AGC TGG

                                                                   CDR2
                                                             ~~~~~~~~~~~~~~~~~~~~
       I   R   Q   P   P   G   K   G   L   E   W   I   G   E   I   N   H   S
109    ATC CGC CAG CCC CCA GGG AAG GGG CTG GAG TGG ATT GGG GAA ATC AAT CAT AGT

                   CDR2
     ~~~~~~~~~~~~~~~~~~~~~~~~~~~~~~~~~~~~~~~~~~~~
       G   S   T   K   Y   T   P   S   L   K   S   R   V   T   I   S   V   D
163    GGA AGC ACC AAG TAC ACC CCG TCC CTC AAG AGC CGA GTC ACC ATA TCA GTA GAC

       T   S   K   H   Q   F   S   L   K   L   S   S   V   T   A   A   D   T
217    ACG TCC AAG CAC CAA TTC TCC CTG AAG CTG AGC TCT GTG ACC GCC GCG GAC ACG

                                           CDR3
                                 ~~~~~~~~~~~~~~~~~~~~~~~~~~~~~~~
       A   V   Y   Y   C   A   R   E   T   V   Y   Y   F   D   L   W   G   R
271    GCT GTG TAT TAC TGT GCG AGA GAG ACT GTC TAC TAC TTC GAT CTC TGG GGC CGT
                                              |
                                              └► JH2

       G   T   L   V   T   V   S   S
325    GGC ACC CTG GTC ACT GTC TCC TCA
```

*Fig. 3A*

```
Anti-2H9 CD30 VL
     V-segment:        Locus: L6
     J segment:        JK1

      E   I   V   L   T   Q   S   P   A   T   L   S   L   S   P   G   E   R
  1  GAA ATT GTG TTG ACA CAG TCT CCA GCC ACC CTG TCT TTG TCT CCA GGG GAA AGA

                                   CDR1
                          ~~~~~~~~~~~~~~~~~~~~~~~~~~~~~~~~~~~~~~~~~~~~~
      A   T   L   S   C   R   A   S   Q   S   V   S   S   N   L   A   W   Y
 55  GCC ACC CTC TCC TGC AGG GCC AGT CAG AGT GTA AGC AGC AAC TTA GCC TGG TAC

                                                           CDR2
                                                      ~~~~~~~~~~~~~~~~~~~
      Q   Q   K   P   G   Q   A   P   R   L   L   I   Y   D   A   S   N   R
109  CAA CAG AAA CCT GGC CAG GCT CCC AGG CTC CTC ATC TAT GAT GCA TCC AAC AGG

     CDR2
     ~~~~~~~~
      A   T   G   I   P   A   R   L   S   G   S   G   S   G   T   D   F   T
163  GCC ACT GGC ATC CCA GCC AGG CTC AGT GGC AGT GGG TCT GGG ACA GAC TTC ACT

                                                                    CDR3
                                                                  ~~~~~~~
      L   T   I   S   S   L   E   P   E   D   F   A   V   Y   Y   C   Q   Q
217  CTC ACC ATC AGC AGC CTA GAG CCT GAA GAT TTT GCA GTT TAT TAC TGT CAA CAG

           CDR3
     ~~~~~~~~~~~~~~~~~~~~~~~~~~~
      R   S   N   W   P   W   T   F   G   Q   G   T   K   V   E   I   K
271  CGT AGC AAC TGG CCG TGG ACG TTC GGC CAA GGG ACC AAG GTG GAA ATC AAA
                     |
                     └─> JK1
```

*Fig. 3B*

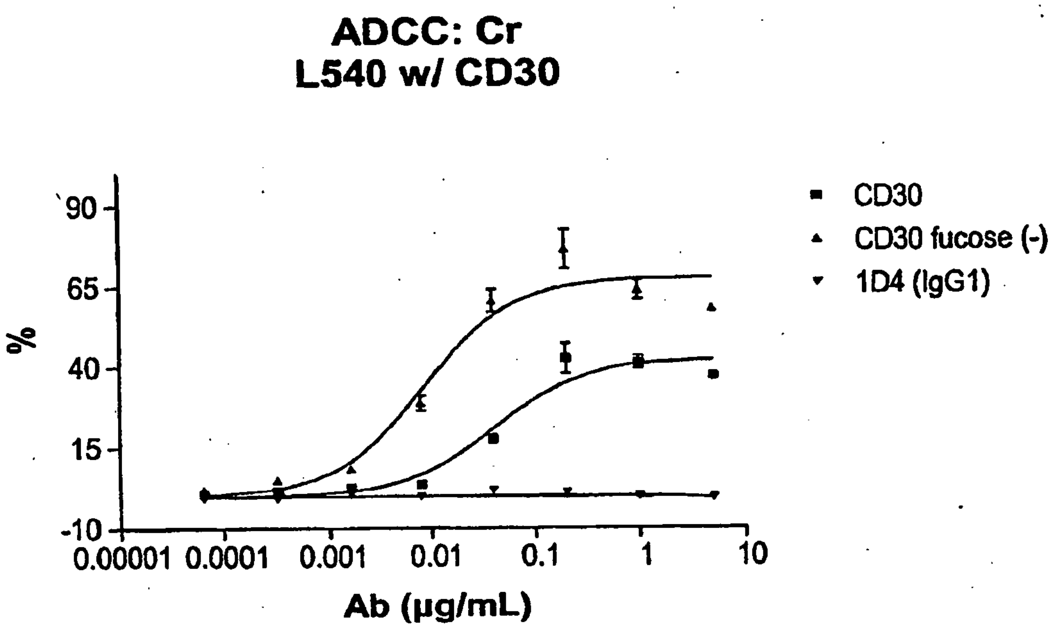
ADCC: Cr
L540 w/ CD30
%
90
65
40
15
-10
0.00001 0.0001 0.001 0.01 0.1 1 10
Ab (µg/mL)
CD30
CD30 fucose (-)
1D4 (IgG1)

*Fig. 4*

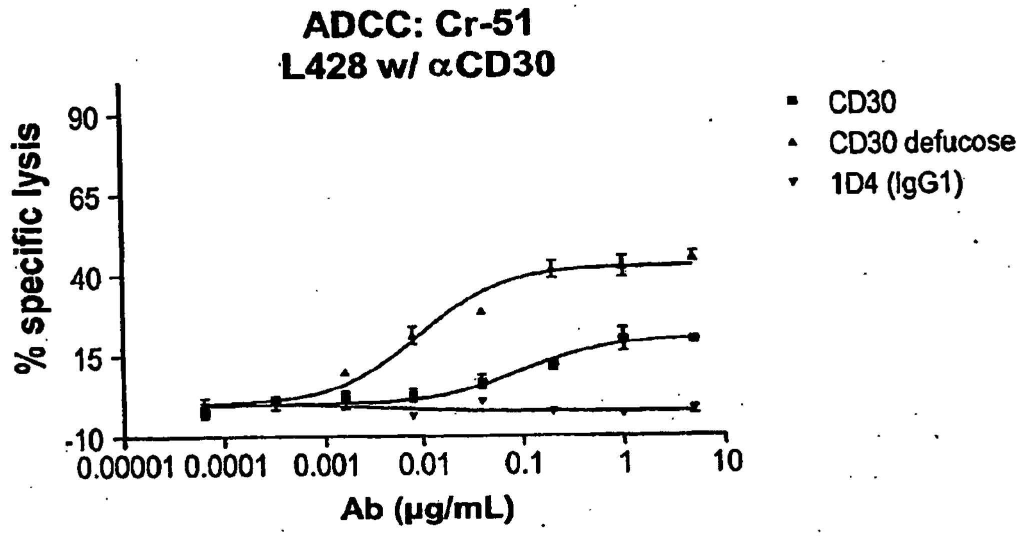
ADCC: Cr-51
L428 w/ αCD30
% specific lysis
90
65
40
15
-10
0.00001
0.0001
0.001
0.01
0.1
1
10
Ab (µg/mL)
CD30
CD30 defucose
1D4 (IgG1)

*Fig. 5*

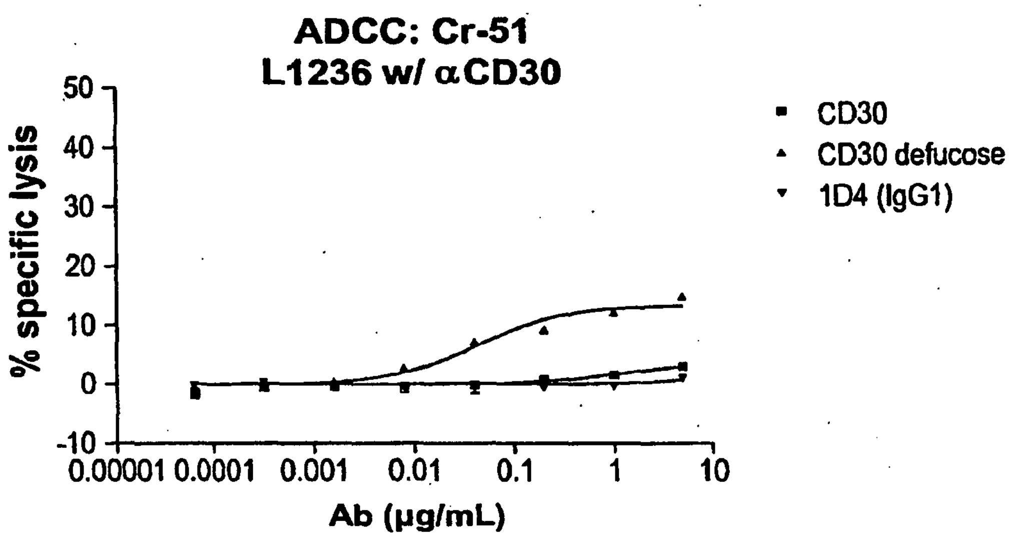
ADCC: Cr-51
L1236 w/ αCD30
% specific lysis
50
40
30
20
10
0
-10
0.00001 0.0001 0.001 0.01 0.1 1 10
Ab (μg/mL)
CD30
CD30 defucose
1D4 (IgG1)

*Fig. 6*

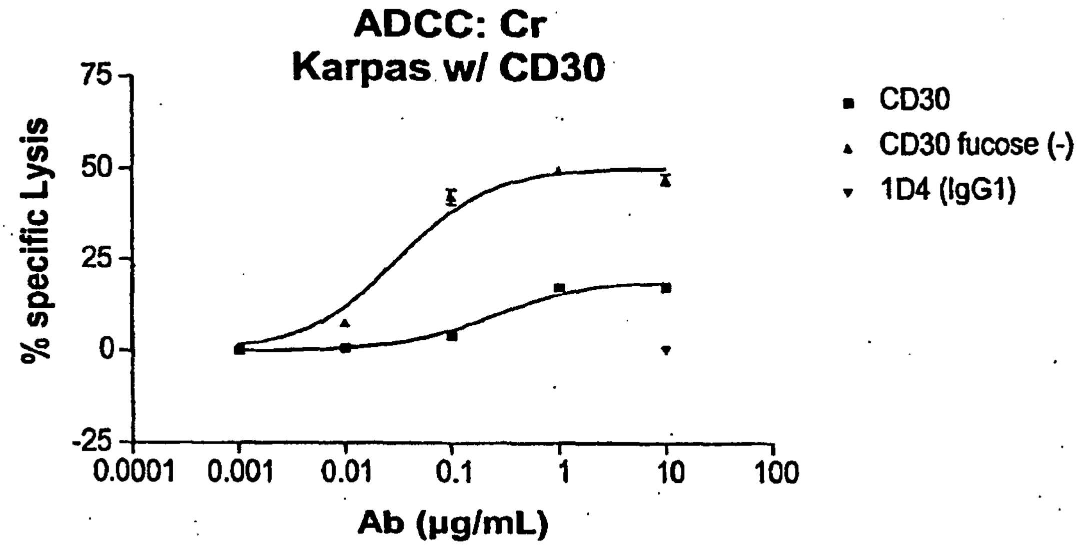
ADCC: Cr
Karpas w/ CD30
% specific Lysis
75
50
25
0
-25
0.0001
0.001
0.01
0.1
1
10
100
Ab (µg/mL)
CD30
CD30 fucose (-)
1D4 (IgG1)

*Fig. 7*

**Heavy Chain Germline Sequences**

4-34 Germline: Q V Q L Q Q W G A G L L K P S E T L S L T C A V Y G G S F S G Y Y W S (CDR1: G Y Y W S)

W I R Q P P G K G L E W I G E I N H S G S T N Y N P S L K S R V T I S (CDR2: G S T N Y N P S L K S)

V D T S K N Q F S L K L S S V T A A D T A V Y Y C A R (CDR3: C A R)

3-07 Germline: E V Q L V E S G G G L V Q P G G S L R L S C A A S G F T F S S Y W M S W (CDR1: S Y W M S)

V R Q A P G K G L E W V A N I K Q D G S E K Y Y V D S V K G R F T I S R (CDR2: N I K Q D G S E K Y Y V D S V K G)

D N A K N S L Y L Q M N S L R A E D T A V Y Y C A R

*Fig. 8A*

**Light Chain Germline Sequences**

**L6 germline:**

CDR1

E I V L T Q S P A T L S L S P G E R A T L S C R A S Q S V S S Y L A

CDR2

W Y Q Q K P G Q A P R L L I Y D A S N R A T G I P A R F S G S G S G

CDR3

T D F T L T I S S L E P E D F A V Y Y C Q Q R S N W

**A27 Germline:**

EIVLTQSPGTLSLSPGERATLSC RASQSVSSSYLA (CDR1) WYQQKPGQAPRLLIY GASSRAT (CDR2)

GIPDRFSGSGSGTDFTLTISRLEPEDFAVYYC QQYGSS (CDR3)

**L15 Germline:**

DIQMTQSPSSLSASVGDRVTITC RASQGISSWLA (CDR1) WYQQKPEKAPKSLIY AASSLQS (CDR2)

GVPSRFSGSGSGTDFTLTISSLQPEDFATYYC QQYNSY (CDR3)

*Fig. 8B*

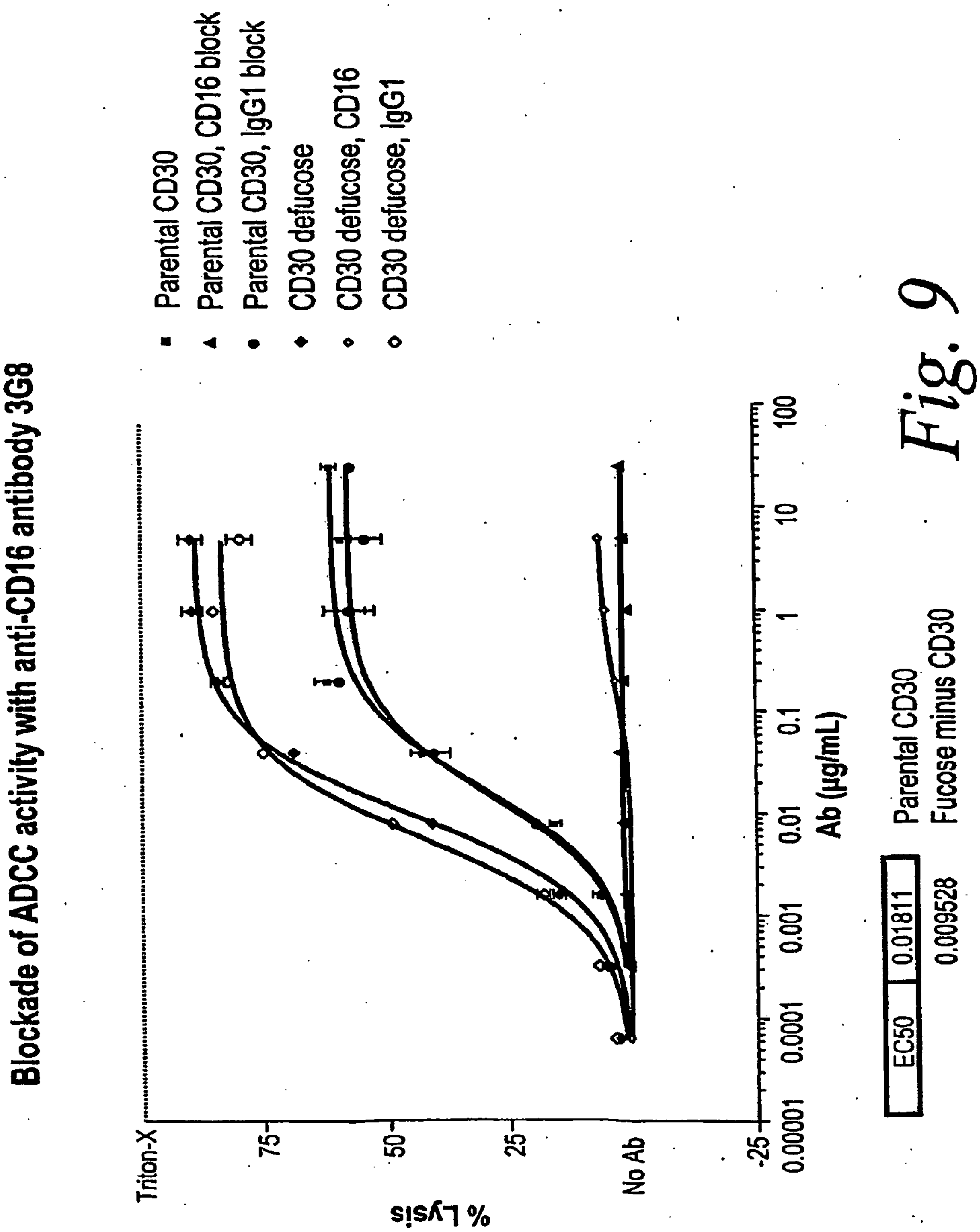
Blockade of ADCC activity with anti-CD16 antibody 3G8
% Lysis
Triton-X
75
50
25
No Ab
-25
0.00001
0.0001
0.001
0.01
0.1
1
10
100
Ab (µg/mL)
Parental CD30
Parental CD30, CD16 block
Parental CD30, IgG1 block
CD30 defucose
CD30 defucose, CD16
CD30 defucose, IgG1
EC50
0.01811
0.009528
Parental CD30
Fucose minus CD30

*Fig. 9*

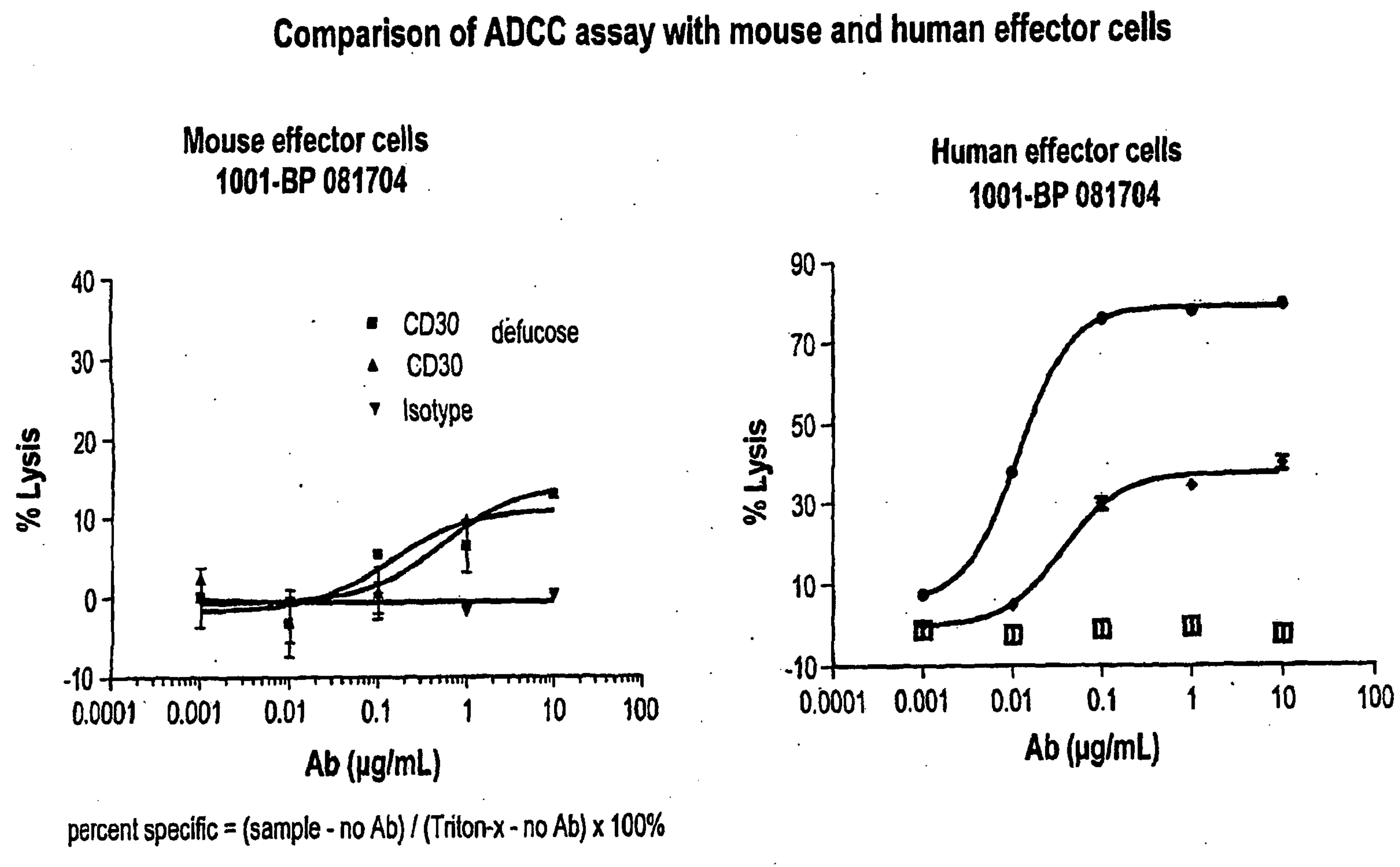
Comparison of ADCC assay with mouse and human effector cells
Mouse effector cells
1001-BP 081704
% Lysis
40
30
20
10
0
-10
CD30 defucose
CD30
Isotype
0.0001
0.001
0.01
0.1
1
10
100
Ab (µg/mL)
percent specific = (sample - no Ab) / (Triton-x - no Ab) x 100%
Human effector cells
1001-BP 081704
% Lysis
90
70
50
30
10
-10
0.0001
0.001
0.01
0.1
1
10
100
Ab (µg/mL)

Fig. 10

## ADCC assay using cynomolgus blood

### Experiment #1

ADCC CD30 with Karpas 299 and Cyno Cells
1:50 T/E Ratio
50U/ml IL2 Overnight Treatment

### Experiment #2

ADCC CD30 with Karpas 299 and Cyno Cells
1:50 T/E Ratio
50U/ml IL2 Overnight Treatment

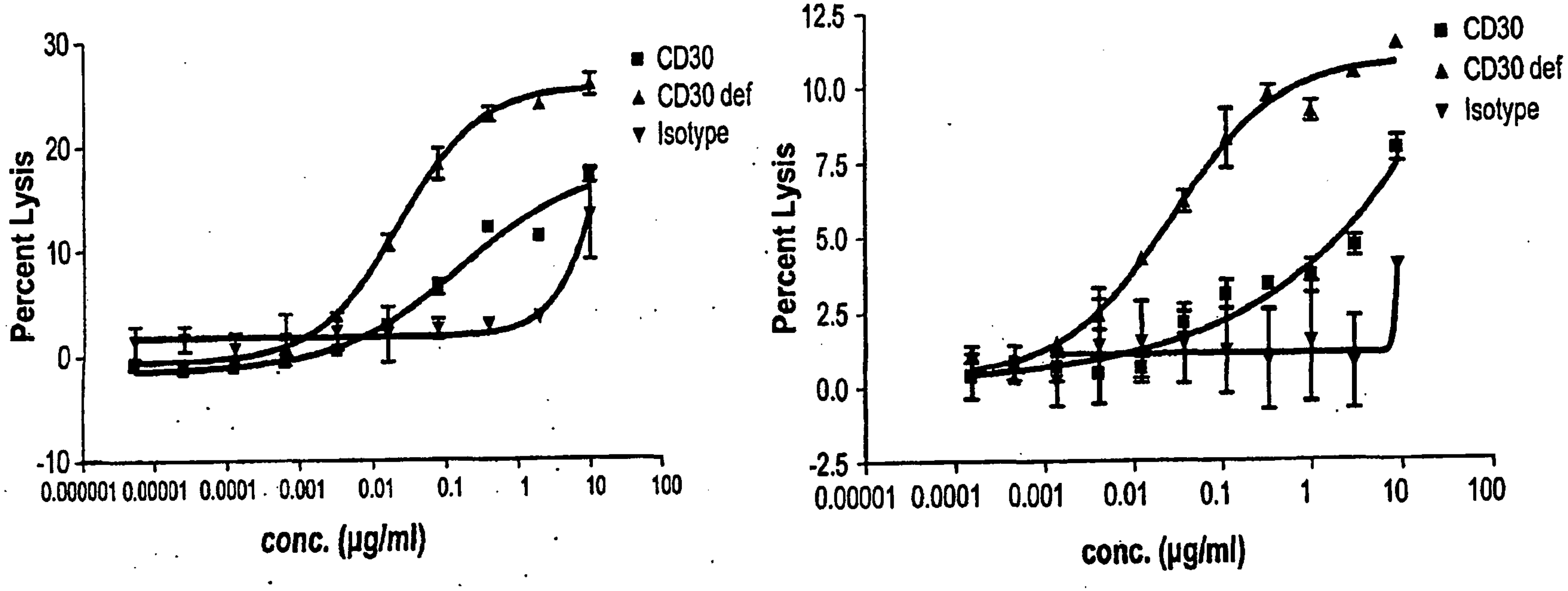


Fig. 11

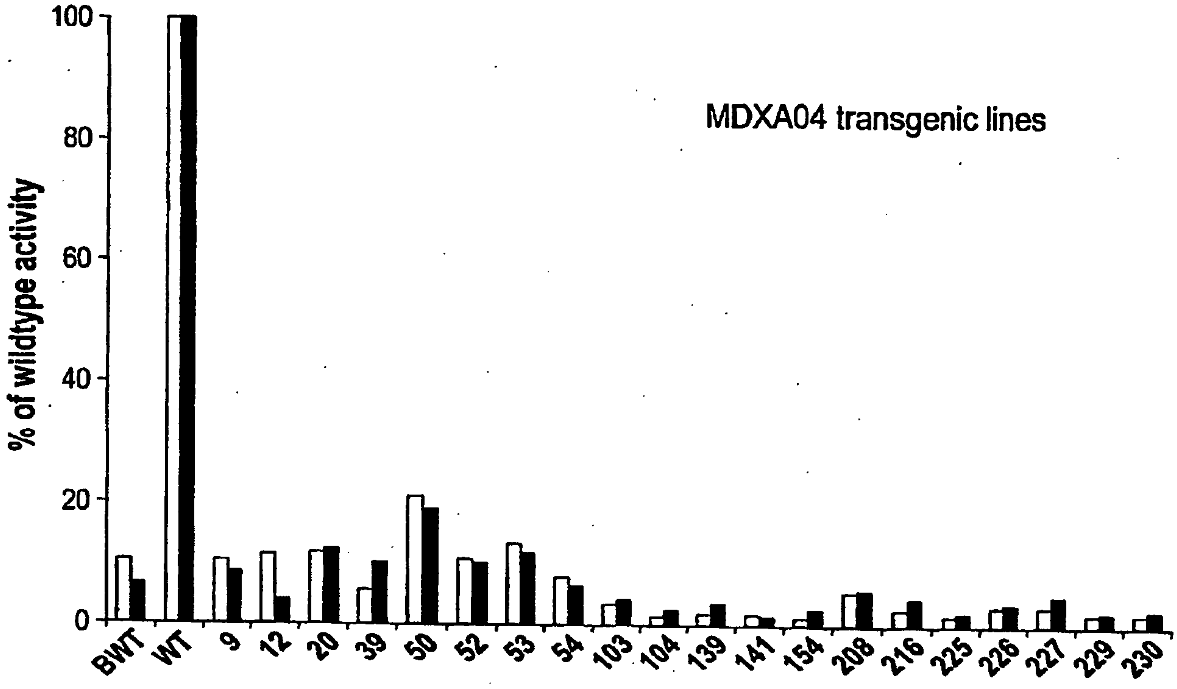
100
80
60
40
20
0
% of wildtype activity
MDXA04 transgenic lines
BWT
WT
9
12
20
39
50
52
53
54
103
104
139
141
154
208
216
225
226
227
229
230

*Fig. 12*

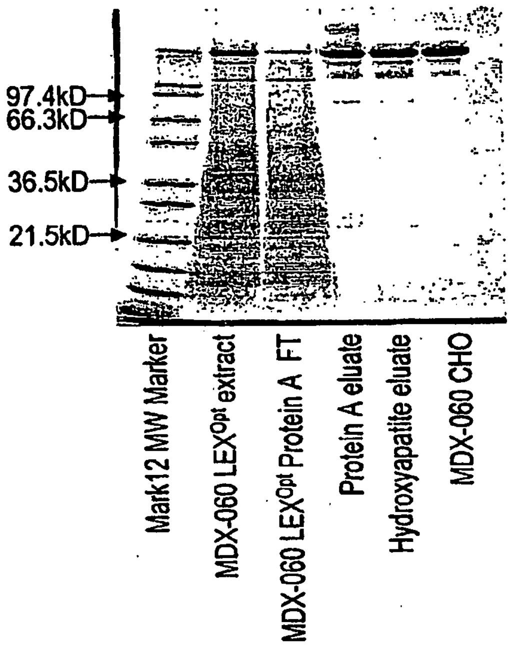


*Fig. 13A*

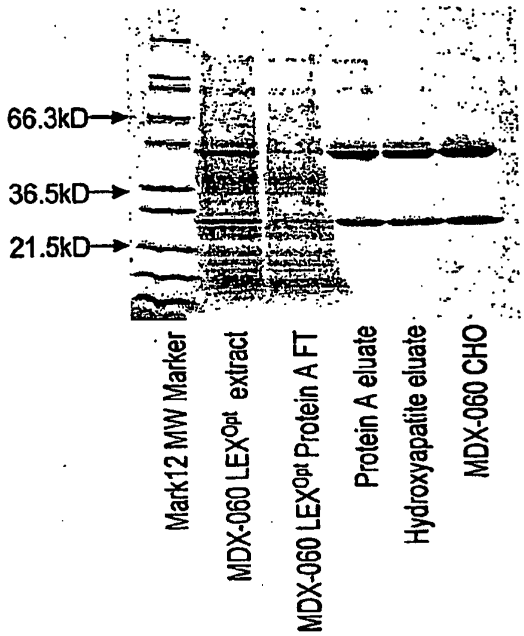


*Fig. 13B*

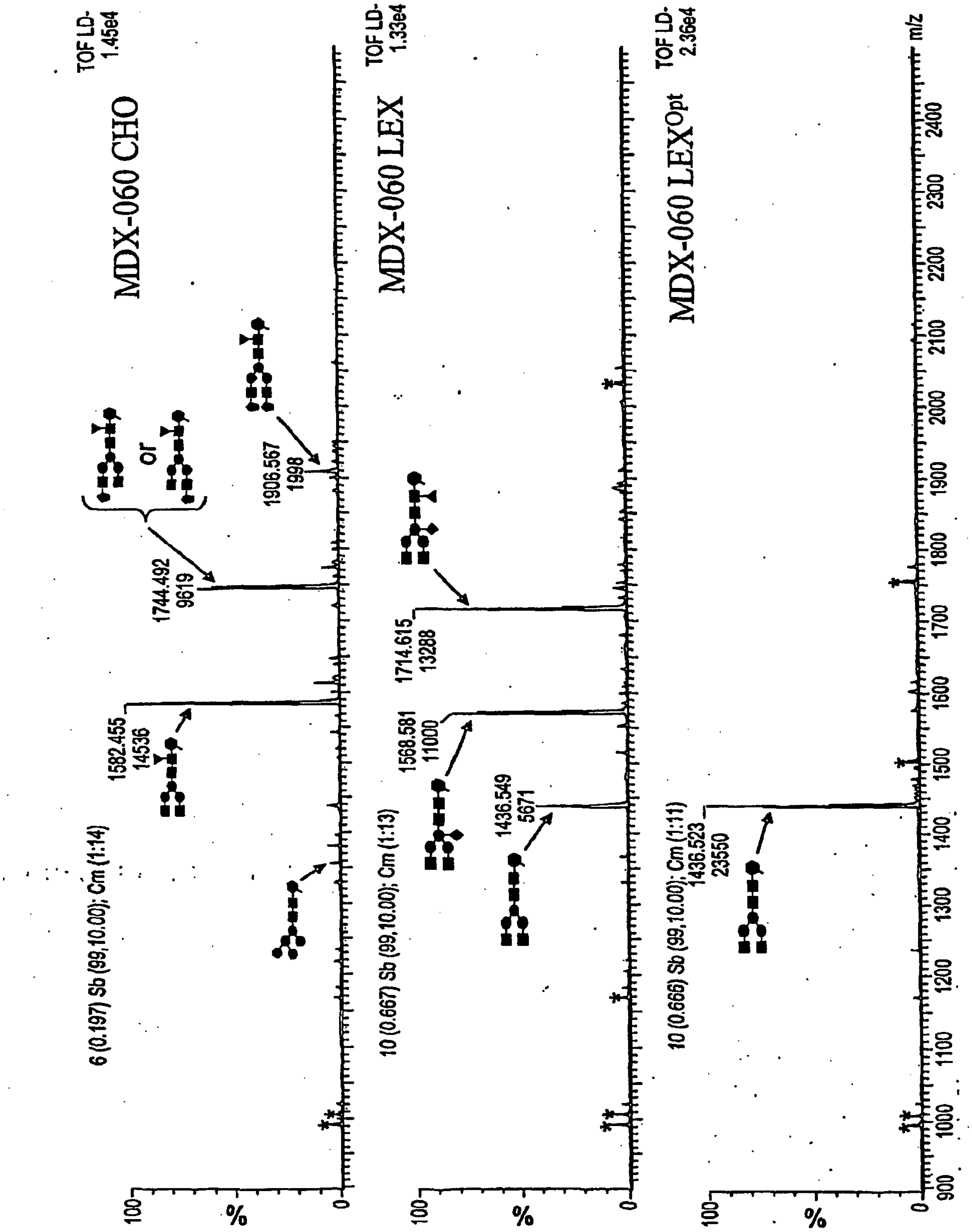
MDX-060 CHO
TOF LD-
1.45e4
6 (0.197) Sb (99,10.00); Cm (1:14)
1582.455
14536
1744.492
9619
or
1906.567
1998
MDX-060 LEX
TOF LD-
1.33e4
10 (0.667) Sb (99,10.00); Cm (1:13)
1436.549
5671
1568.581
11000
1714.615
13288
MDX-060 LEX^Opt
TOF LD-
2.36e4
10 (0.666) Sb (99,10.00); Cm (1:11)
1436.523
23550
%
100
0
900
1000
1100
1200
1300
1400
1500
1600
1700
1800
1900
2000
2100
2200
2300
2400
m/z

*Fig. 14*

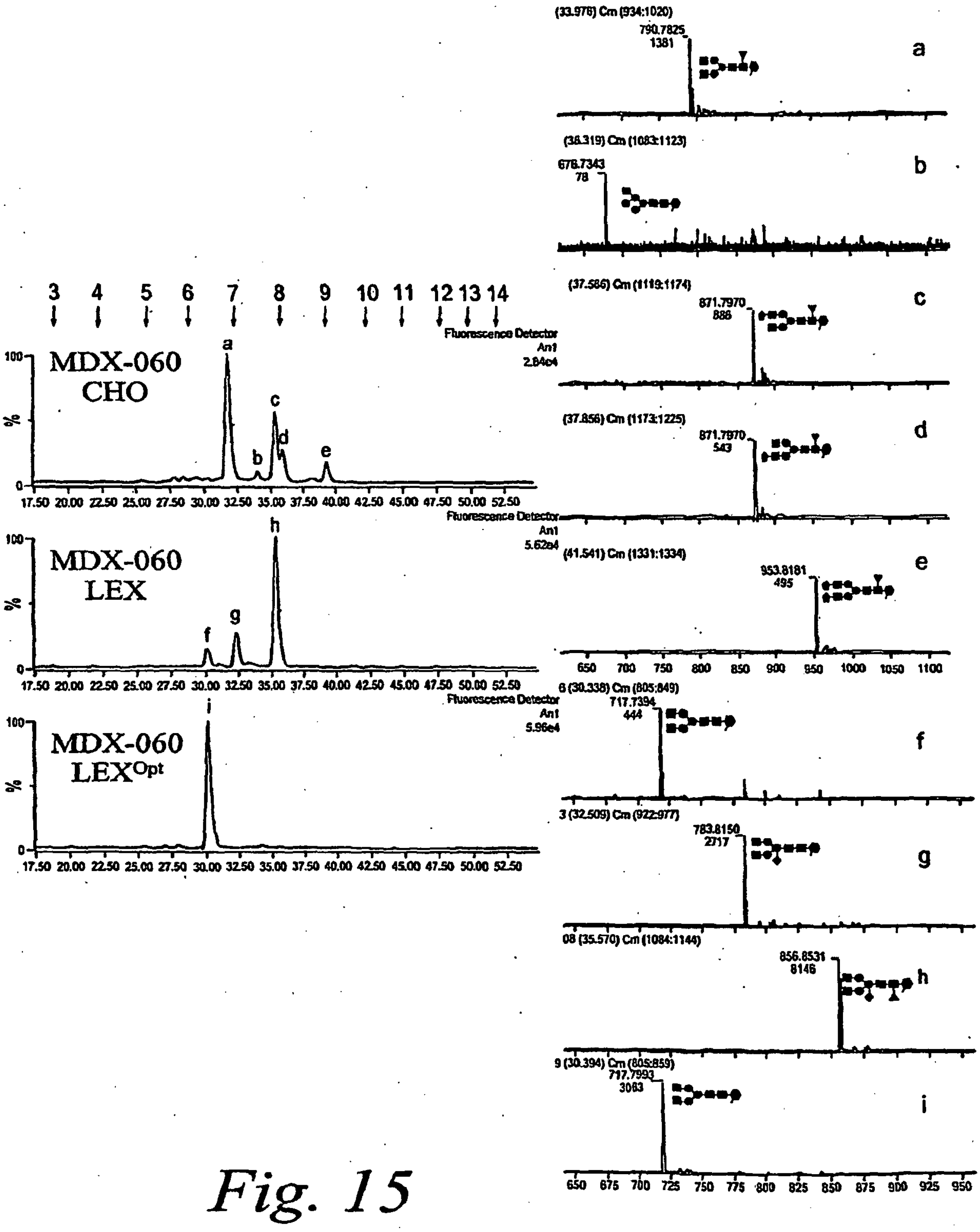
MDX-060
CHO
MDX-060
LEX
MDX-060
LEXOpt
Fluorescence Detector

*Fig. 15*

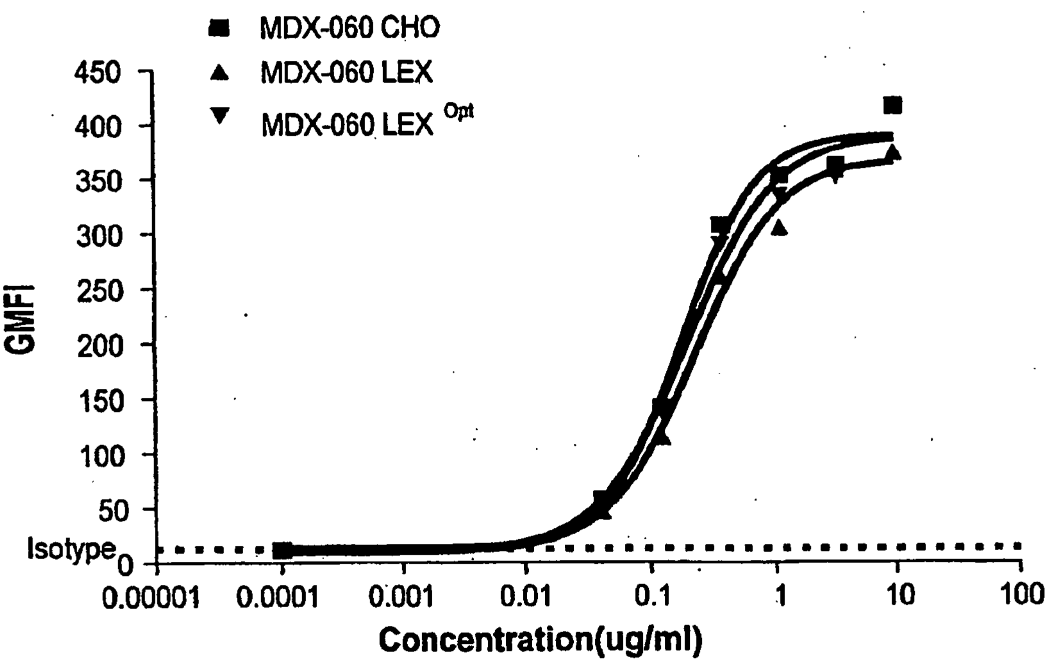
MDX-060 CHO
MDX-060 LEX
MDX-060 LEX Opt
450
400
350
300
250
200
150
100
50
Isotype 0
GMFI
0.00001
0.0001
0.001
0.01
0.1
1
10
100
Concentration(ug/ml)

*Fig. 16*

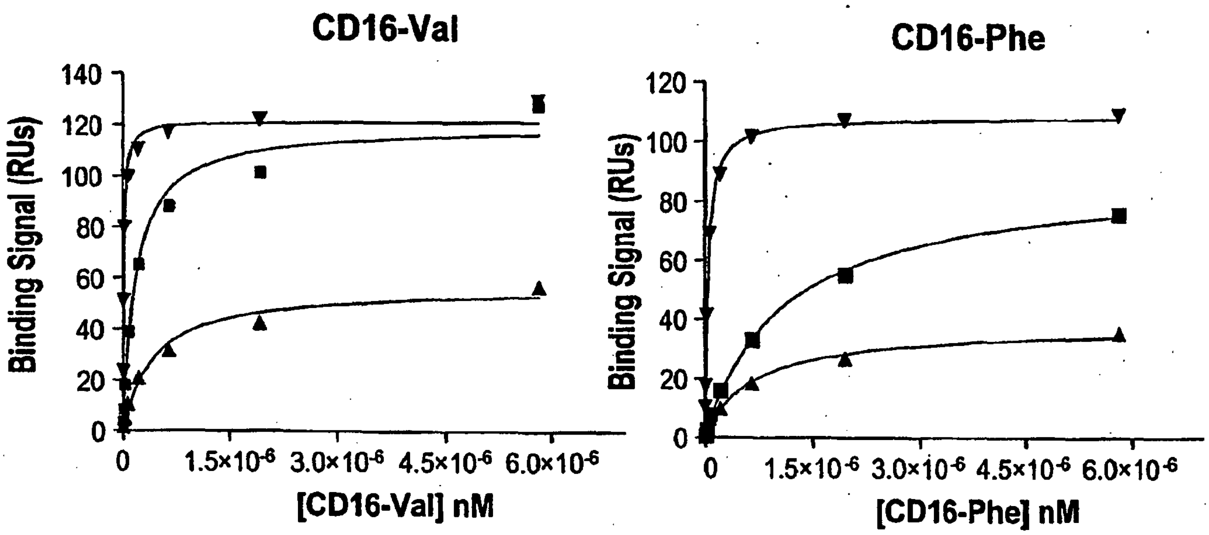


| | Binding Constants (nM) | | |
|---|---|---|---|
| Human FcR | MDX-060 CHO | MDX-060 LEX | MDX-060 LEX$^{Opt}$ |
| CD16-Val | 177 | 427 | 12 |
| CD16-Phe | 1091 | 641 | 41 |

*Fig. 17*

Phe158/Phe158
Val158/Phe158

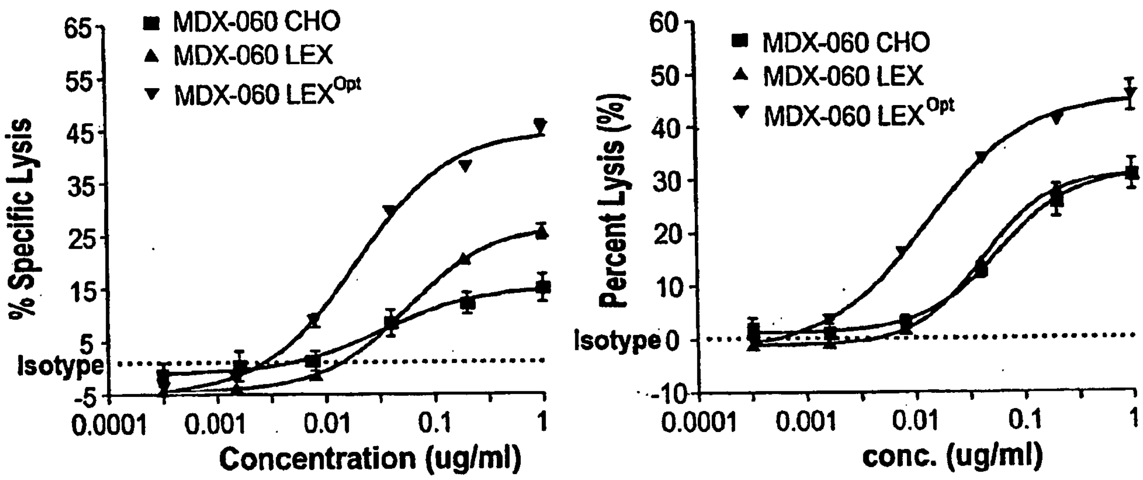


*Fig. 18*

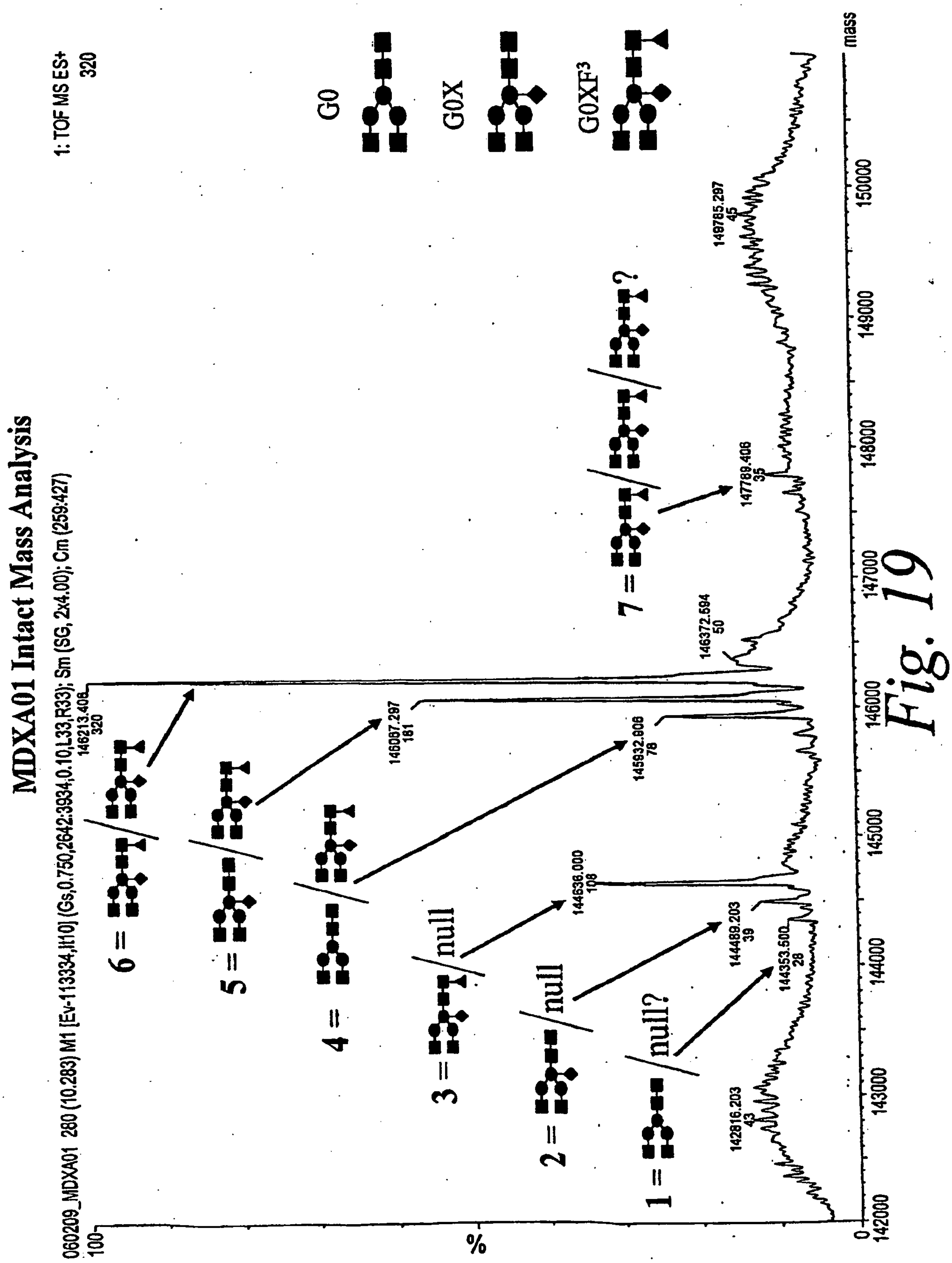
MDXA01 Intact Mass Analysis
060209_MDXA01 280 (10.283) M1 [Ev-113334,It10] (Gs,0.750,2642:3934,0.10,L33,R33); Sm (SG, 2x4.00); Cm (259:427)
1: TOF MS ES+
320
G0
G0X
G0XF³
6 =
5 =
4 =
3 =
/null
2 =
/null
1 =
/null?
7 =
?
146213.406
320
146067.297
181
145932.906
78
144636.000
108
144489.203
39
144353.500
28
142816.203
43
146372.594
50
147789.406
35
149785.297
45
100
%
0
142000
143000
144000
145000
146000
147000
148000
149000
150000
mass

Fig. 19

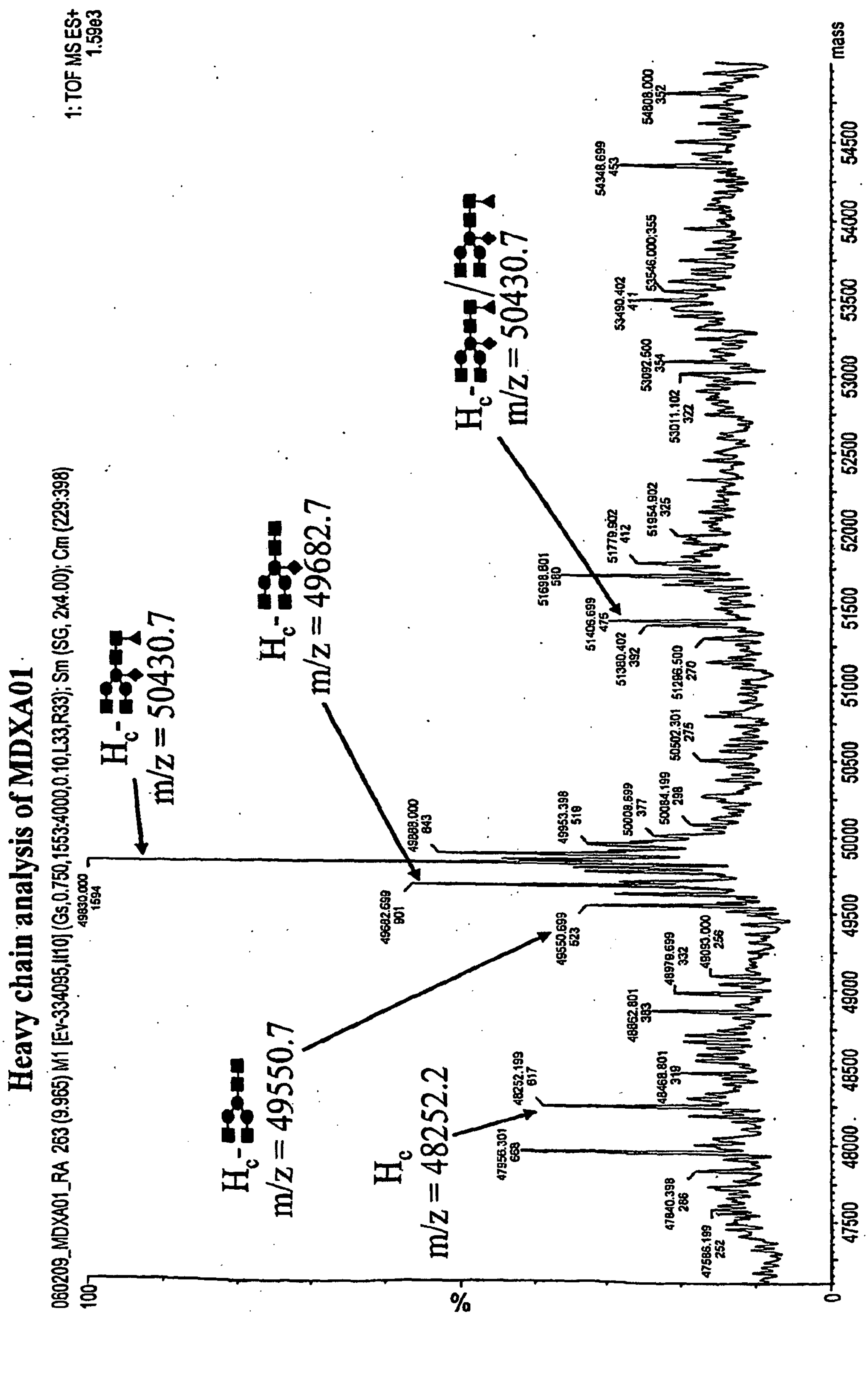
Heavy chain analysis of MDXA01
060209_MDXA01_RA 263 (9.965) M1 [Ev-334095,It10] (Gs,0.750,1553:4000,0.10,L33,R33); Sm (SG, 2x4.00); Cm (229:398)
1: TOF MS ES+
1.59e3
100
%
0
mass
$H_c$
m/z = 48252.2
$H_c$-
m/z = 49550.7
$H_c$-
m/z = 50430.7
$H_c$-
m/z = 49682.7
$H_c$- /
m/z = 50430.7
49830.000
1594
49682.699
901
49888.000
843
47556.301
668
48252.199
617
49550.699
523
49953.398
519
47586.199
252
47840.398
286
48468.801
319
48862.801
383
48976.699
332
49093.000
256
50008.699
377
50084.199
298
50502.301
275
51266.500
270
51380.402
392
51406.699
475
51698.801
580
51779.902
412
51954.902
325
53011.102
322
53092.500
354
53490.402
411
53546.000,355
54348.699
453
54808.000
352
47500
48000
48500
49000
49500
50000
50500
51000
51500
52000
52500
53000
53500
54000
54500

*Fig. 20*

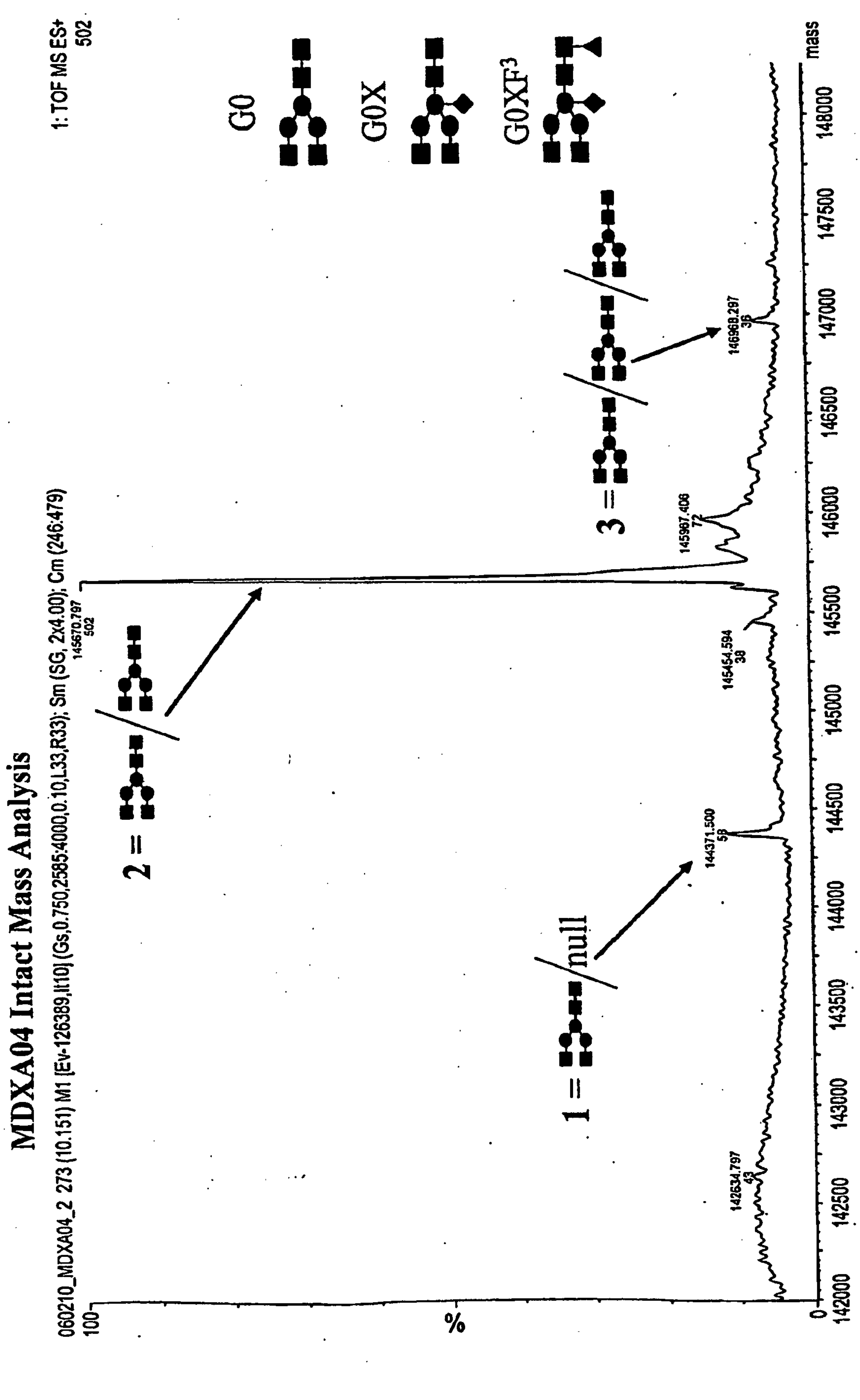
MDXA04 Intact Mass Analysis
060210_MDXA04_2 273 (10.151) M1 [Ev-126389,It10] (Gs,0.750,2585:4000,0.10,L33,R33); Sm (SG, 2x4.00); Cm (246:479)
1: TOF MS ES+
502
G0
G0X
G0XF3
2 =
1 =
null
3 =
145670.797
502
145454.594
38
145967.406
72
146968.297
36
144371.500
58
142634.797
43
100
%
0
142000
142500
143000
143500
144000
144500
145000
145500
146000
146500
147000
147500
148000
mass

*Fig. 21*

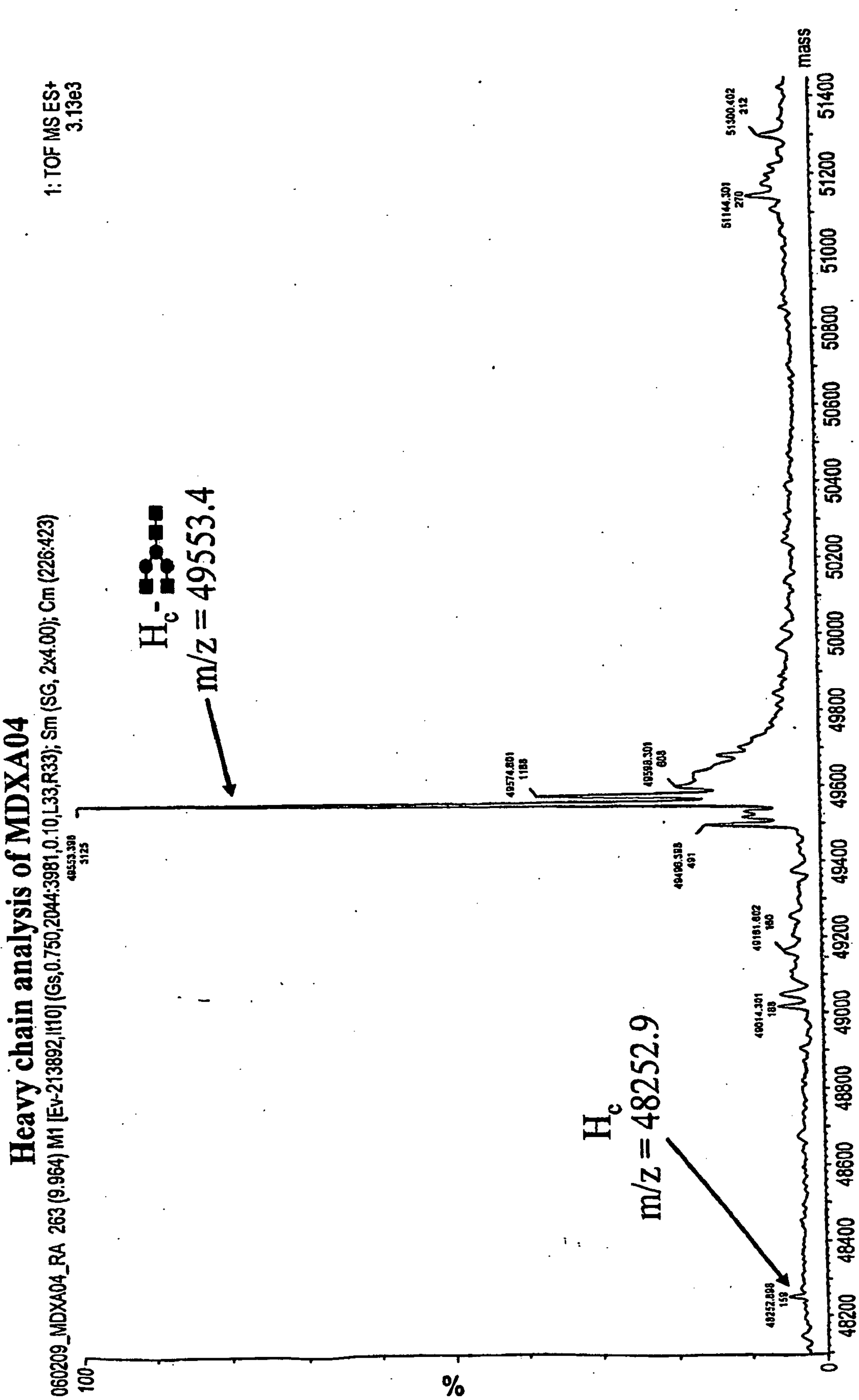
Heavy chain analysis of MDXA04
060209_MDXA04_RA 263 (9.964) M1 [Ev-213892,It10] (Gs,0.750,2044:3981,0.10,L33,R33); Sm (SG, 2x4.00); Cm (226:423)
1: TOF MS ES+
3.13e3
H$_c$-
m/z = 49553.4
H$_c$
m/z = 48252.9
100
%
0
48200
48400
48600
48800
49000
49200
49400
49600
49800
50000
50200
50400
50600
50800
51000
51200
51400
mass

*Fig. 22*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03059282 A
- US 2004261148 A
- WO 0243478 A
- US 20040006215 A
- US 20040110704 A, Yamane
- EP 1176195 A
- WO 03035835 A, Presta
- WO 9954342 A, Umana
- US 5714350 A
- US 6350861 A, Co
- US 5563055 A
- US 5981840 A
- US 4945050 A
- US 5879918 A
- US 5886244 A
- US 5932782 A
- WO 024661 A
- US 5225539 A, Winter
- US 5530101 A
- US 5585089 A
- US 5693762 A
- US 6180370 A, Queen
- US 20030153043 A, Carr
- US 5677425 A, Bodmer
- US 6165745 A, Ward
- US 6277375 B, Ward
- US 5869046 A
- US 6121022 A, Presta
- US 5624821 A
- US 5648260 A, Winter
- US 6194551 B, Idusogie
- WO 9429351 A, Bodmer
- WO 0042072 A, Presta
- EP 0154316 A, Nishimura
- EP 0401384 A, Ishikawa
- WO 02092780 A, Short
- WO 03074679 A, Lazar
- US 4816567 A, Cabilly
- US 5545806 A
- US 5569825 A
- US 5625126 A
- US 5633425 A
- US 5789650 A
- US 5877397 A
- US 5661016 A
- US 5814318 A
- US 5874299 A
- US 5770429 A
- US 5545807 A, Surani
- WO 9203918 A
- WO 9312227 A
- WO 9425585 A
- WO 9713852 A
- WO 9824884 A
- WO 9945962 A
- WO 0114424 A, Korman
- US 5939598 A
- US 6075181 A
- US 6114598 A
- US 6150584 A
- US 6162963 A, Kucherlapati
- US 5223409 A
- US 5403484 A
- US 5571698 A, Ladner
- US 5427908 A
- US 5580717 A, Dower
- US 5969108 A
- US 6172197 A, McCafferty
- US 5885793 A
- US 6521404 A
- US 6544731 A
- US 6555313 A
- US 6582915 A
- US 6593081 A, Griffiths
- US 5476996 A
- US 5698767 A, Wilson
- US 4399216 A
- US 4634665 A
- US 5179017 A
- WO 8704462 A
- WO 8901036 A
- EP 338841 A
- US 5399163 A
- US 5383851 A
- US 5312335 A
- US 5064413 A
- US 4941880 A
- US 4790824 A
- US 4596556 A
- US 4487603 A
- US 4486194 A
- US 4447233 A
- US 4447224 A
- US 4439196 A
- US 4475196 A
- US 4522811 A
- US 5374548 A

- US 5399331 A
- US 5416016 A, Low
- US 5811097 A
- US 6281354 B
- US 6548530 B
- US 20030050331 A
- US 20030064984 A
- US 20030073852 A
- US 20040087497 A
- WO 03022806 A
- WO 05112919 A
- US 2005153394 A
- US 0701451 W
- US 60759298 B
- US 60790373 B
- US 60791178 B
- US 60812702 B
- US 60836998 B
- US 60837202 B

**Non-patent literature cited in the description**


- **BRUIN, P.C. et al.** *Leukemia,* 1995, vol. 9, 1620-1627
- **SCHWAB, U. et al.** *Nature,* 1982, vol. 299, 65
- **GRUSS, H.J. et al.** *Blood,* 1994, vol. 83, 2045-2056
- **SCHWAB et al.** *Nature,* 1982, vol. 299, 65
- **FROESE et al.** *J. Immunol.,* 1987, vol. 139, 2081
- **CARDE et al.** *Eur. J. Cancer,* 1990, vol. 26, 474
- **STEIN et al.** *Blood,* 1985, vol. 66, 848
- **MIETTINEN.** *Arch. Pathol. Lab. Med.,* 1992, vol. 116, 1197
- **PIRIS et al.** *Histopathology,* 1990, vol. 17, 211
- **BURNS et al.** *Am. J. Clin. Pathol.,* 1990, vol. 93, 327
- **ECKERT et al.** *Am. J. Dermatopathol.,* 1989, vol. 11, 345
- **ANDREESEN et al.** *Blood,* 1984, vol. 63, 1299
- **SCHWARTING et al.** *Blood,* vol. 74, 1678
- **PALLESEN et al.** *Am J. Pathol.,* vol. 133, 446
- **MECHTERSHEIMER et al.** *Cancer,* vol. 66, 1732
- **ANDREESEN et al.** *Am. J. Pathol.,* vol. 134, 187
- **CHAIARLE, R. et al.** *Clin. Immunol.,* vol. 90 (2), 157-164
- **POHL C. et al.** *Int J Cancer,* 1993, vol. 54, 418
- **FALINI B. et al.** *Brit J Haematol.,* 1992, vol. 82, 38-45
- **KOON, H.B. et al.** *Curr Opin in Oncol.,* 2000, vol. 12, 588-593
- **SCHELL, R. et al.** Annals of Oncology. 2002, vol. 13, 57-66
- **BORCHMANN et al.** *Blood,* 2003, vol. 102 (10), 3737-3742
- **SHINKAWA et al.** *Journal of Biological Chemistry,* 2003, vol. 278 (5), 3466-3473
- **YAMANE-OHNUKI et al.** *Biotechnology and Bioengineering,* 2004, vol. 87 (5), 614-622
- **MA et al.** *Nat. Rev. Genet.,* vol. 4, 794-805
- **RASKIN et al.** *Trends Biotechnol.,* vol. 20, 522-531
- **GASDASKA et al.** *Bioprocessing,* 2003, vol. 2, 49-56
- FUNDAMENTAL IMMUNOLOGY. 1993, vol. 3
- **WARD et al.** *Nature,* 1989, vol. 341, 544-546
- **BIRD et al.** *Science,* 1988, vol. 242, 423-426
- **HUSTON et al.** *Proc. Natl. Acad Sci. USA,* 1988, vol. 85, 5879-5883
- **KABAT, E. A. et al.** Sequences of Proteins of Immunological Interest. NIH Publication No. 91-3242, 1991
- **KLIMKA et al.** *British J. of Cancer,* 2000, vol. 83 (2), 252-260
- **BEIBOER et al.** *J. Mol. Biol.,* 2000, vol. 296, 833-849
- **RADER et al.** *Proc. Natl. Acad Sci. U.S.A,* 1998, vol. 95, 8910-8915
- **BARBAS et al.** *J. Am. Chem. Soc.,* 1994, vol. 116, 2161-2162
- **BARBAS et al.** *Proc. Natl. Acad Sci. U.S.A.,* 1995, vol. 92, 2529-2533
- **DITZEL et al.** *J. Immunol.,* 1996, vol. 157, 739-749
- **YAMANE-OHNUKI et al.** *Biotechnol Bioeng,* 2004, vol. 87, 614-22
- **SHIELDS, R.L. et al.** *J. Biol. Chem.,* 2002, vol. 277, 26733-26740
- **UMANA et al.** *Nat. Biotech.,* 1999, vol. 17, 176-180
- **TARENTINO, A.L. et al.** *Biochem.,* 1975, vol. 14, 5516-23
- **LEROUGE et al.** *Plant Mol. Biol.,* 1998, vol. 38, 31-48
- **REE et al.** *J Biol. Chem.,* 2000, vol. 15, 11451-11458
- **BARDOR et al.** *Glycobiol.,* 2003, vol. 13, 427-434
- **GARCIA-CASADO et al.** *Glycobiol.,* 1996, vol. 6, 471-477
- **BAKKER et al.** *Proc Nat Acad Sci, USA,* vol. 98, 2899-2904
- **CHUANG ; MEYEROWITZ.** *Proc. Natl. Acad Sci. USA,* vol. 97, 4985-4990

- **STOUTJESDIJK et al.** *Plant Physiol.,* 2002, vol. 129, 1723-1731
- **WATERHOUSE ; HELLIWELL.** *Nat. Rev. Genet.,* 2003, vol. 4, 29-38
- **CROSSWAY et al.** *Biotechniques,* 1986, vol. 4, 320-334
- **RIGGS et al.** *Proc. Natl. Acad Sci. USA,* 1986, vol. 83, 5602-5606
- **PASZKOWSKI et al.** *EMBO J.,* 1984, vol. 3, 2717-2722
- Direct DNA Transfer into Intact Plant Cells via Microprojectile Bombardment. **TOMES et al.** Plant Cell, Tissue, and Organ Culture: Fundamental Methods. Springer-Verlag, 1995
- **MCCABE et al.** *Biotechnology,* 1988, vol. 6, 923-926
- **MCCORMICK et al.** *Plant Cell Reports,* 1986, vol. 5, 81-84
- **TEDDER et al.** *Springer Semin Immunol,* 2006, vol. 28, 351-64
- **MARSHALL et al.** *Annu Rev Biochem,* 1972, vol. 41, 673-702
- **GALA FA ; MORRISON SL.** *J Immunol,* 2004, vol. 172, 5489-94
- **WALLICK et al.** *J Exp Med,* 1988, vol. 168, 1099-109
- **SPIRO RG.** *Glycobiology,* 2002, vol. 12, 43R-56R
- **PAREKH et al.** *Nature,* 1985, vol. 316, 452-7
- **MIMURA et al.** *Mol Immunol,* 2000, vol. 37, 697-706
- **JANINI et al.** *Electrophoresis,* 2002, vol. 23, 1605-11
- **MA et al.** *Chromatographia,* 2001, vol. 53, 75-89
- **HUNT et al.** *J Chromatogr A,* 1998, vol. 800, 355-67
- **KRISHNAMURTHY R ; MANNING MC.** *Curr Pharm Biotechnol,* 2002, vol. 3, 361-71
- **CHEN et al.** *Pharm Res,* 2003, vol. 20, 1952-60
- **GHIRLANDO et al.** *Immunol Lett,* 1999, vol. 68, 47-52
- **MURRAY et al.** *J. Chromatogr Sci,* 2002, vol. 40, 343-9
- **ALEXANDER AJ ; HUGHES DE.** *Anal Chem,* 1995, vol. 67, 3626-32
- **E. MEYERS ; W. MILLER.** *Comput. Appl. Biosci.,* 1988, vol. 4, 11-17
- **NEEDLEMAN ; WUNSCH.** *J. Mol. Biol.,* 1970, vol. 48, 444-453
- **ALTSCHUL et al.** *J. Mol. Biol.,* 1990, vol. 215, 403-10
- **ALTSCHUL et al.** *Nucleic Acids Res.,* 1997, vol. 25 (17), 3389-3402
- **RIECHMANN, L. et al.** *Nature,* 1998, vol. 332, 323-327
- **JONES, P. et al.** *Nature,* 1986, vol. 321, 522-525
- **QUEEN, C. et al.** *Proc. Natl. Acad. See. U.S.A.,* 1989, vol. 86, 10029-10033
- **TOMLINSON, I. M. et al.** The Repertoire of Human Germline VH Sequences Reveals about Fifty Groups of VH Segments with Different Hypervariable Loops. *J. Mol. Biol.,* 1992, vol. 227, 776-798
- **COX, J. P. L. et al.** A Directory of Human Germ-line VH Segments Reveals a Strong Bias in their Usage. *Eur. J. Immunol.,* 1994, vol. 24, 827-836
- **ALTSCHUL et al.** *Nucleic Acids Research,* 1997, vol. 25, 3389-3402
- **SHIELDS, R.L. et al.** *J. Biol. Chem.,* 2001, vol. 276, 6591-6604
- Current Protocols in Molecular Biology. Greene Publishing and Wiley Interscience, 1987
- **KABAT, E. A.** Sequences of Proteins of Immunological Interest. NIH Publication No. 91-3242, 1991
- **HUSTON et al.** *Proc. Natl. Acad. Sci. USA,* 1988, vol. 85, 5879-5883
- **MCCAFFERTY et al.** *Nature,* 1990, vol. 348, 552-554
- **KOHLER ; MILSTEIN.** *Nature,* 1975, vol. 256, 495
- **LONBERG et al.** *Nature,* 1994, vol. 368 (6474), 856-859
- **LONBERG, N.** Handbook of Experimental Pharmacology. 1994, vol. 113, 49-101
- **LONBERG, N. ; HUSZAR, D.** *Intern. Rev. Immunol.,* 1995, vol. 13, 65-93
- **HARDING, F. ; LONBERG, N.** *Ann. N.Y. Acad. Sci.,* 1995, vol. 764, 536-546
- **TAYLOR, L. et al.** *Nucleic Acids Research,* 1992, vol. 20, 6287-6295
- **CHEN, J. et al.** *International Immunology,* 1993, vol. 5, 647-656
- **TUAILLON et al.** *Proc. Natl. Acad. Sci. USA,* 1993, vol. 90, 3720-3724
- **CHOI et al.** *Nature Genetics,* 1993, vol. 4, 117-123
- **CHEN, J. et al.** *EMBO J,* 1993, vol. 12, 821-830
- **TUAILLON et al.** *J. Immunol.,* 1994, vol. 152, 2912-2920
- **TAYLOR, L. et al.** *International Immunology,* 1994, vol. 6, 579-591
- **FISHWILD, D. et al.** *Nature Biotechnology,* 1996, vol. 14, 845-851
- **TOMIZUKA et al.** *Proc. Natl. Acad. Sci. USA,* 2000, vol. 97, 722-727
- **KUROIWA et al.** *Nature Biotechnology,* 2002, vol. 20, 889-894
- **LONBERG, N. et al.** *Nature,* 1994, vol. 368 (6474), 856-859
- **MORRISON, S.** *Science,* 1985, vol. 229, 1202

- Gene Expression Technology. **GOEDDEL.** Methods in Enzymology. Academic Press, 1990, vol. 185
- **TAKEBE, Y. et al.** *Mol. Cell. Biol.,* 1988, vol. 8, 466-472
- **BOSS, M. A. ; WOOD, C. R.** *Immunology Today,* 1985, vol. 6, 12-13
- **URLAUB ; CHASM.** *Proc. Natl. Acad. Sci. USA,* 1980, vol. 77, 4216-4220
- **R. J. KAUFMAN ; P. A. SHARP.** *Mol. Biol.,* 1982, vol. 159, 601-621
- Sustained and Controlled Release Drug Delivery Systems. Marcel Dekker, Inc, 1978
- **V.V. RANADE.** *J. Clin. Pharmacol.,* 1989, vol. 29, 685
- **UMEZAWA et al.** *Biochem. Biophys. Res. Commun.,* 1988, vol. 153, 1038
- **P.G. BLOEMAN et al.** *FEBS Lett.,* 1995, vol. 357, 140
- **M. OWAIS et al.** *Antimicrob. Agents Chemother.,* 1995, vol. 39, 180
- **BRISCOE et al.** *Am. J Physiol.,* 1994, vol. 1233, 134
- **SCHREIER et al.** *J. Biol. Chem.,* 1994, vol. 269, 9090
- **K. KEINANEN ; M.L. LAUKKANEN.** *FEBS Lett.,* 1994, vol. 346, 123
- **J.J. KILLION ; I.J. FIDLER.** *Immunomethods,* 1994, vol. 4, 273
- **RIDGE, J. et al.** *Nature,* 1998, vol. 393, 474-478
- **WEINBERG, A. et al.** *Immunol,* 2000, vol. 164, 2160-2169
- **MELERO, I. et al.** *Nature Medicine,* 1997, vol. 3, 682-685
- **HUTLOFF, A. et al.** *Nature,* 1999, vol. 397, 262-266
- **RUGGERI et al.** *Histol Histolpathol.,* 2006, vol. 21, 249-56
- **CHIARLE et al.** *Pathologica,* 2003, vol. 95, 229-30
- **CHAKRABARTY et al.** *Clin Exp Immunol,* 2003, vol. 133, 318-225
- **WATANABE et al.** *Thyroid,* 2003, vol. 13, 259-63
- **PELLEGRINI et al.** *Neuroimmunomodulation,* 2005, vol. 12, 220-34
- **NENOI, M. et al.** *Gene,* 1996, vol. 175, 179
- **TRIGUERO et al.** *Plant Biotechnol. J.,* 2005, vol. 3, 449-457
- **TAKAHASHI et al.** *Anal. Biochem.,* 1998, vol. 255, 183-187
- **DILLON et al.** *J. Chromatogr. A.,* 2004, vol. 1053, 299-305

SEQUENCE LISTING

<110> MEDAREX, INC.

<120> MONOCLONAL ANTIBODIES AGAINST CD30 LACKING IN FUCOSYL AND XYLOSYL RESIDUES

<130> MXI-357PC

<140> PCT/US07/01451
<141> 2007-01-17

<150> 60/759,298
<151> 2006-01-17

<150> 60/790,373
<151> 2006-04-07

<150> 60/791,178
<151> 2006-04-11

<150> 60/812, 702
<151> 2006-06-09

<150> 60/836,998
<151> 2006-08-11

<150> 60/837,202
<151> 2006-08-11

<160> 43

<170> Patent In Ver. 3.3

<210> 1
<211> 112
<212> PRT
<213> Homo sapiens

<400> 1

```
Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Ala Tyr
            20                  25                  30

Tyr Trp Ser Trp Ile Arg Gln Pro Pro Gly Lys Gly Leu Glu Trp Ile
        35                  40                  45

Gly Asp Ile Asn His Gly Gly Gly Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Asn Ser Val Thr Ala Ala Asp Thr Ala Val Tyr Tyr Cys Ala
                85                  90                  95

Ser Leu Thr Ala Tyr Trp Gly Gln Gly Ser Leu Val Thr Val Ser Ser
            100                 105                 110
```

<210> 2
<211> 116
<212> PRT
<213> Homo sapiens

<400> 2

Glu Val Gln Leu Val Glu Ser Gly Gly Gly Leu Val Gln Pro Gly Gly
1               5                   10                  15

Ser Leu Arg Leu Ser Cys Val Ala Ser Gly Phe Thr Phe Ser Asn Ser
            20                  25                  30

Trp Met Ser Trp Val Arg Gln Ala Pro Gly Lys Gly Leu Glu Trp Val
        35                  40                  45

Ala Asn Ile Asn Glu Asp Gly Ser Glu Lys Phe Tyr Val Asp Ser Val
    50                  55                  60

Lys Gly Arg Phe Thr Phe Ser Arg Asp Asn Ala Glu Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Val His Trp Tyr Phe His Leu Trp Gly Arg Gly Thr Leu Val
            100                 105                 110

Thr Val Ser Ser
        115

<210> 3
<211> 116
<212> PRT
<213> Homo sapiens

<400> 3

Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asn His Ser Gly Ser Thr Lys Tyr Thr Pro Ser Leu Lys
    50                  55                  60

Ser Arg Val Thr Ile Ser Val Asp Thr Ser Lys His Gln Phe Ser Leu
65                  70                  75                  80

Lys Leu Ser Ser Val Thr Ala Ala Asp Thr Ala Val Tyr Tyr Cys Ala
                85                  90                  95

Arg Glu Thr Val Tyr Tyr Phe Asp Leu Trp Gly Arg Gly Thr Leu Val
            100                 105                 110

Thr Val Ser Ser
        115

<210> 4
<211> 107
<212> PRT
<213> Homo sapiens

<400> 4

Asp Ile Gln Met Thr Gln Ser Pro Thr Ser Leu Ser Ala Ser Val Gly
1               5                   10                  15

Asp Arg Val Thr Ile Thr Cys Arg Ala Ser Gln Gly Ile Ser Ser Trp
            20                  25                  30

Leu Thr Trp Tyr Gln Gln Lys Pro Glu Lys Ala Pro Lys Ser Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asp Ser Tyr Pro Ile
                85                  90                  95

Thr Phe Gly Gln Gly Thr Arg Leu Glu Ile Lys
            100                 105

<210> 5
<211> 108
<212> PRT
<213> Homo sapiens

<400> 5

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Ser Leu Glu
65                  70                  75                  80

Pro Glu Asp Phe Ala Val Tyr Tyr Cys Gln Gln Tyr Gly Ser Ser Pro
                85                  90                  95

Trp Thr Phe Gly Gln Gly Thr Lys Val Glu Ile Lys
            100                 105

<210> 6
<211> 107
<212> PRT
<213> Homo sapiens

<400> 6

```
Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Asn
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Leu Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Trp
                85                  90                  95

Thr Phe Gly Gln Gly Thr Lys Val Glu Ile Lys
            100                 105
```

<210> 7
<211> 5

<212> PRT
<213> Homo sapiens

<400> 7

```
Ala Tyr Tyr Trp Ser
1               5
```

<210> 8
<211> 5
<212> PRT
<213> Homo sapiens

<400> 8

```
Asn Ser Trp Met Ser
1               5
```

<210> 9
<211> 5
<212> PRT
<213> Homo sapiens

<400> 9

```
Gly Tyr Tyr Trp Ser
1               5
```

<210> 10
<211> 16
<212> PRT
<213> Homo sapiens

<400> 10

```
Asp Ile Asn His Gly Gly Gly Thr Asn Tyr Asn Pro Ser Leu Lys Ser
1               5                   10                  15
```

<210> 11
<211> 17
<212> PRT
<213> Homo sapiens

<400> 11

```
Asn Ile Asn Glu Asp Gly Ser Glu Lys Phe Tyr Val Asp Ser Val Lys
1               5                   10                  15

Gly
```

<210> 12
<211> 16
<212> PRT
<213> Homo sapiens

<400> 12

```
Glu Ile Asn His Ser Gly Ser Thr Lys Tyr Thr Pro Ser Leu Lys Ser
1               5                   10                  15
```

<210> 13
<211> 4
<212> PRT
<213> Homo sapiens

<400> 13

```
Leu Thr Ala Tyr
1
```

<210> 14
<211> 7
<212> PRT
<213> Homo sapiens

<400> 14

```
Val His Trp Tyr Phe His Leu
1               5
```

<210> 15
<211> 8
<212> PRT
<213> Homo sapiens

<400> 15

```
Glu Thr Val Tyr Tyr Phe Asp Leu
1               5
```

<210> 16
<211> 11
<212> PRT
<213> Homo sapiens

<400> 16

Arg Ala Ser Gln Gly Ile Ser Ser Trp Leu Thr
1 5 10

<210> 17
<211> 12
<212> PRT
<213> Homo sapiens

<400> 17

Arg Ala Ser Gln Ser Val Ser Ser Ser Tyr Leu Ala
1 5 10

<210> 18
<211> 11
<212> PRT
<213> Homo sapiens

<400> 18

Arg Ala Ser Gln Ser Val Ser Ser Asn Leu Ala
1 5 10

<210> 19
<211> 7
<212> PRT
<213> Homo sapiens

<400> 19

Ala Ala Ser Ser Leu Gln Ser
1 5

<210> 20
<211> 7
<212> PRT
<213> Homo sapiens

<400> 20

Gly Ala Ser Ser Arg Ala Thr
1 5

<210> 21
<211> 7
<212> PRT
<213> Homo sapiens

<400> 21

Asp Ala Ser Asn Arg Ala Thr
1 5

<210> 22
<211> 9
<212> PRT

<213> Homo sapiens

<400> 22

Gln Gln Tyr Asp Ser Tyr Pro Ile Thr
1               5

<210> 23

<211> 9
<212> PRT
<213> Homo sapiens

<400> 23

Gln Gln Tyr Gly Ser Ser Pro Trp Thr
1               5

<210> 24
<211> 9
<212> PRT
<213> Homo sapiens

<400> 24

Gln Gln Arg Ser Asn Trp Pro Trp Thr
1               5

<210> 25
<211> 97
<212> PRT
<213> Homo sapiens

<400> 25

Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asn His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg

<210> 26
<211> 98
<212> PRT

<213> Homo sapiens

<400> 26

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Asn Ile Lys Gln Asp Gly Ser Glu Lys Tyr Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg
```

<210> 27

<211> 94
<212> PRT
<213> Homo sapiens

<400> 27

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln Gln Lys Pro Glu Lys Ala Pro Lys Ser Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Ser Tyr
                85                  90
```

<210> 28
<211> 95

<212> PRT
<213> Homo sapiens

<400> 28

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Ser Ser Ser
20 25 30

Tyr Leu Ala Trp Tyr Gln Gln Lys Pro Gly Gln Ala Pro Arg Leu Leu
35 40 45

Ile Tyr Gly Ala Ser Ser Arg Ala Thr Gly Ile Pro Asp Arg Phe Ser
50 55 60

Gly Ser Gly Ser Gly Thr Asp Phe Thr Leu Thr Ile Ser Arg Leu Glu
65 70 75 80

Pro Glu Asp Phe Ala Val Tyr Tyr Cys Gln Gln Tyr Gly Ser Ser
85 90 95

<210> 29
<211> 94
<212> PRT
<213> Homo sapiens

<400> 29

Glu Ile Val Leu Thr Gln Ser Pro Ala Thr Leu Ser Leu Ser Pro Gly
1 5 10 15

Glu Arg Ala Thr Leu Ser Cys Arg Ala Ser Gln Ser Val Ser Ser Tyr
20 25 30

Leu Ala Trp Tyr Gln Gln Lys Pro Gly Gln Ala Pro Arg Leu Leu Ile
35 40 45

Tyr Asp Ala Ser Asn Arg Ala Thr Gly Ile Pro Ala Arg Phe Ser Gly
50 55 60

Ser Gly Ser Gly Thr Asp Phe Thr Leu Thr Ile Ser Ser Leu Glu Pro
65 70 75 80

Glu Asp Phe Ala Val Tyr Tyr Cys Gln Gln Arg Ser Asn Trp
85 90

<210> 30
<211> 282
<212> DNA
<213> Homo sapiens

<220>
<221> CDS
<222> (1) .. (282) .

<400> 30

```
cag gtg cag cta cag cag tgg ggc gca gga ctg ttg aag cct tcg gag    48
Gln Val Gln Leu Gln Gln Trp Gly Ala Gly Leu Leu Lys Pro Ser Glu
 1               5                  10                  15

acc ctg tcc ctc acc tgc gct gtc tat ggt ggg tcc ttc agt gct tac    96
Thr Leu Ser Leu Thr Cys Ala Val Tyr Gly Gly Ser Phe Ser Ala Tyr
            20                  25                  30

tac tgg agc tgg atc cgc cag ccc cca ggg aag ggg ctg gag tgg att   144
Tyr Trp Ser Trp Ile Arg Gln Pro Pro Gly Lys Gly Leu Glu Trp Ile
        35                  40                  45

ggg gac atc aat cat ggt gga ggc acc aac tac aac ccg tcc ctc aag   192
Gly Asp Ile Asn His Gly Gly Gly Thr Asn Tyr Asn Pro Ser Leu Lys
    50                  55                  60

agt cga gtc acc ata tca gta gac acg tcc aag aac cag ttc tcc ctg   240
Ser Arg Val Thr Ile Ser Val Asp Thr Ser Lys Asn Gln Phe Ser Leu
65                  70                  75                  80

aag ctg aac tct gta acc gcc gcg gac acg acc gtc tcc tca           282
Lys Leu Asn Ser Val Thr Ala Ala Asp Thr Thr Val Ser Ser
                85                  90
```

<210> 31
<211> 348
<212> DNA
<213> Homo sapiens

<220>
<221> CDS .
<222> (1)..(348)

<400> 31

```
gag gtg cag ttg gtg gag tct ggg gga ggc ttg gtc cag cct ggg ggg    48
Glu Val Gln Leu Val Glu Ser Gly Gly Gly Leu Val Gln Pro Gly Gly
 1               5                  10                  15
```

```
tcc ctg aga ctc tcc tgt gta gcc tct gga ttc acc ttt agt aac tct      96
Ser Leu Arg Leu Ser Cys Val Ala Ser Gly Phe Thr Phe Ser Asn Ser
            20                  25                  30

tgg atg agc tgg gtc cgc cag gct cca ggg aaa ggg ctg gag tgg gtg     144
Trp Met Ser Trp Val Arg Gln Ala Pro Gly Lys Gly Leu Glu Trp Val
        35                  40                  45

gcc aac ata aac gaa gat gga agt gag aaa ttc tat gtg gac tct gtg     192
Ala Asn Ile Asn Glu Asp Gly Ser Glu Lys Phe Tyr Val Asp Ser Val
    50                  55                  60

aag ggc cga ttc acc ttc tcc aga gac aac gcc gag aac tca ctg tat     240
Lys Gly Arg Phe Thr Phe Ser Arg Asp Asn Ala Glu Asn Ser Leu Tyr
65                  70                  75                  80

ctg caa atg aac agc ctg aga gcc gag gac acg gct gtg tat tac tgt     288
Leu Gln Met Asn Ser Leu Arg Ala Glu Asp Thr Ala Val Tyr Tyr Cys
                85                  90                  95

gcg agg gtt cat tgg tac ttc cat ctc tgg ggc cgt ggc acc ctg gtc     336
Ala Arg Val His Trp Tyr Phe His Leu Trp Gly Arg Gly Thr Leu Val
            100                 105                 110

act gtc tcc tca                                                     348
Thr Val Ser Ser
        115
```

<210> 32

<211> 348
<212> DNA
<213> Homo sapiens

<220>
<221> CDS
<222> (1) .. (348)

<400> 32

```
cag gtg cag cta cag cag tgg ggc gca gga ctg ttg aag cct tcg gag      48
Gln Val Gln Leu Gln Gln Trp Gly Ala Gly Leu Leu Lys Pro Ser Glu
1               5                   10                  15

acc ctg tcc ctc acc tgc gct gtc tat ggt ggg tcc ttc agt ggt tac      96
Thr Leu Ser Leu Thr Cys Ala Val Tyr Gly Gly Ser Phe Ser Gly Tyr
            20                  25                  30

tac tgg agc tgg atc cgc cag ccc cca ggg aag ggg ctg gag tgg att     144
Tyr Trp Ser Trp Ile Arg Gln Pro Pro Gly Lys Gly Leu Glu Trp Ile
        35                  40                  45

ggg gaa atc aat cat agt gga agc acc aag tac acc ccg tcc ctc aag     192
Gly Glu Ile Asn His Ser Gly Ser Thr Lys Tyr Thr Pro Ser Leu Lys
    50                  55                  60

agc cga gtc acc ata tca gta gac acg tcc aag cac caa ttc tcc ctg     240
Ser Arg Val Thr Ile Ser Val Asp Thr Ser Lys His Gln Phe Ser Leu
65                  70                  75                  80
```

```
aag ctg agc tct gtg acc gcc gcg gac acg gct gtg tat tac tgt gcg     288
Lys Leu Ser Ser Val Thr Ala Ala Asp Thr Ala Val Tyr Tyr Cys Ala
                85                  90                  95

aga gag act gtc tac tac ttc gat ctc tgg ggc cgt ggc acc ctg gtc     336
Arg Glu Thr Val Tyr Tyr Phe Asp Leu Trp Gly Arg Gly Thr Leu Val
            100                 105                 110

act gtc tcc tca                                                     348
Thr Val Ser Ser
        115
```

<210> 33
<211> 321
<212> DNA
<213> Homo sapiens

<220>
<221> CDS
<222> (1)..(321)

<400> 33

```
gac atc cag atg acc cag tct cca acc tca ctg tct gca tct gta gga     48
Asp Ile Gln Met Thr Gln Ser Pro Thr Ser Leu Ser Ala Ser Val Gly
1               5                   10                  15

gac aga gtc acc atc act tgt cgg gcg agt cag ggt att agc agc tgg     96
Asp Arg Val Thr Ile Thr Cys Arg Ala Ser Gln Gly Ile Ser Ser Trp
            20                  25                  30

tta acc tgg tat cag cag aaa cca gag aaa gcc cct aag tcc ctg atc     144
Leu Thr Trp Tyr Gln Gln Lys Pro Glu Lys Ala Pro Lys Ser Leu Ile
        35                  40                  45

tat gct gca tcc agt ttg caa agt ggg gtc cca tca agg ttc agc ggc     192
Tyr Ala Ala Ser Ser Leu Gln Ser Gly Val Pro Ser Arg Phe Ser Gly
    50                  55                  60

agt gga tct ggg aca gat ttc act ctc acc atc agc agc ctg cag cct     240
Ser Gly Ser Gly Thr Asp Phe Thr Leu Thr Ile Ser Ser Leu Gln Pro
65                  70                  75                  80

gaa gat ttt gca act tat tac tgc caa cag tat gat agt tac cct atc     288
Glu Asp Phe Ala Thr Tyr Tyr Cys Gln Gln Tyr Asp Ser Tyr Pro Ile
                85                  90                  95

acc ttc ggc caa ggg aca cga ctg gag att aaa                         321
Thr Phe Gly Gln Gly Thr Arg Leu Glu Ile Lys
            100                 105
```

<210> 34
<211> 324
<212> DNA
<213> Homo sapiens

<220>
<221> CDS
<222> (1)..(324)

<400> 34

```
gaa att gtg ttg acg cag tct cca ggc acc ctg tct ttg tct cca ggg    48
Glu Ile Val Leu Thr Gln Ser Pro Gly Thr Leu Ser Leu Ser Pro Gly
1               5                   10                  15

gaa aga gcc acc ctc tcc tgc agg gcc agt cag agt gtt agc agc agc    96
Glu Arg Ala Thr Leu Ser Cys Arg Ala Ser Gln Ser Val Ser Ser Ser
            20                  25                  30

tac tta gcc tgg tac cag cag aaa cct ggc cag gct ccc agg ctc ctc    144
Tyr Leu Ala Trp Tyr Gln Gln Lys Pro Gly Gln Ala Pro Arg Leu Leu
        35                  40                  45

atc tat ggt gca tcc agc agg gcc act ggc atc cca gac agg ttc agt    192
Ile Tyr Gly Ala Ser Ser Arg Ala Thr Gly Ile Pro Asp Arg Phe Ser
    50                  55                  60

ggc agt ggg tct ggg aca gac ttc act ctc acc atc agc agc ctg gag    240
Gly Ser Gly Ser Gly Thr Asp Phe Thr Leu Thr Ile Ser Ser Leu Glu
65                  70                  75                  80

cct gaa gat ttt gca gtg tat tac tgt cag cag tat ggt agc tca ccg    288
Pro Glu Asp Phe Ala Val Tyr Tyr Cys Gln Gln Tyr Gly Ser Ser Pro
                85                  90                  95

tgg acg ttc ggc caa ggg acc aag gtg gaa atc aaa                    324
Trp Thr Phe Gly Gln Gly Thr Lys Val Glu Ile Lys
            100                 105
```

<210> 35
<211> 321
<212> DNA
<213> Homo sapiens

<220>
<221> CDS
<222> (1)..(321)

<400> 35

```
gaa att gtg ttg aca cag tct cca gcc acc ctg tct ttg tct cca ggg    48
Glu Ile Val Leu Thr Gln Ser Pro Ala Thr Leu Ser Leu Ser Pro Gly
1               5                   10                  15

gaa aga gcc acc ctc tcc tgc agg gcc agt cag agt gta agc agc aac    96
Glu Arg Ala Thr Leu Ser Cys Arg Ala Ser Gln Ser Val Ser Ser Asn
            20                  25                  30

tta gcc tgg tac caa cag aaa cct ggc cag gct ccc agg ctc ctc atc    144
Leu Ala Trp Tyr Gln Gln Lys Pro Gly Gln Ala Pro Arg Leu Leu Ile
        35                  40                  45
```

```
tat gat gca tcc aac agg gcc act ggc atc cca gcc agg ctc agt ggc   192
Tyr Asp Ala Ser Asn Arg Ala Thr Gly Ile Pro Ala Arg Leu Ser Gly
     50                  55                  60

agt ggg tct ggg aca gac ttc act ctc acc atc agc agc cta gag cct   240
Ser Gly Ser Gly Thr Asp Phe Thr Leu Thr Ile Ser Ser Leu Glu Pro
 65                  70                  75                  80

gaa gat ttt gca gtt tat tac tgt caa cag cgt agc aac tgg ccg tgg   288
Glu Asp Phe Ala Val Tyr Tyr Cys Gln Gln Arg Ser Asn Trp Pro Trp
                 85                  90                  95

acg ttc ggc caa ggg acc aag gtg gaa atc aaa                       321
Thr Phe Gly Gln Gly Thr Lys Val Glu Ile Lys
            100                 105
```

<210> 36
<211> 28
<212> DNA
<213> Artificial Sequence

<220>
<223> Description of Artificial Sequence: Synthetic primer

<400> 36
atggtcgact gctgctggtg ctctcaac 28

<210> 37
<211> 28
<212> DNA
<213> Artificial Sequence

<220>
<223> Description of Artificial Sequence: Synthetic primer

<400> 37
atgtctagaa tgcagcagca agtgcacc 28

<210> 38
<211> 33
<212> DNA <213> Artificial Sequence

<220>
<223> Description of Artificial Sequence: Synthetic primer

<400> 38
atgactagtt gcgaagccta cttcggcaac agc 33

<210> 39
<211> 30
<212> DNA
<213> Artificial Sequence

<220>
<223> Description of Artificial Sequence: Synthetic primer

<400> 39
atgggatccg aatctcaaga acaactgtcg 30

<210> 40

<211> 33
<212> DNA
<213> Artificial Sequence

<220>
<223> Description of Artificial Sequence: Synthetic primer

<400> 40
atgggtacct gcgaagccta cttcggcaac agc 33

<210> 41
<211> 29
<212> DNA
<213> Artificial Sequence

<220>
<223> Description of Artificial Sequence: Synthetic primer

<400> 41
atgggatcca ctggctggga gaagttctt 29

<210> 42
<211> 28
<212> DNA
<213> Artificial Sequence

<220>
<223> Description of Artificial Sequence: Synthetic primer

<400> 42
atggagctct gctgctggtg ctctcaac 28

<210> 43
<211> 28
<212> DNA
<213> Artificial Sequence

<220>
<223> Description of Artificial Sequence: Synthetic primer

<400> 43
atgggtacca tgcagcagca agtgcacc 28